(19) Europäisches Patentamt
European Patent Office
Office européen des brevets

(11) Publication number: **0 391 652 B1**

(12) **EUROPEAN PATENT SPECIFICATION**

(45) Date of publication of patent specification: **15.03.95**

(51) Int. Cl.[6]: **C07D 493/08**, C07D 417/04, C07D 405/04, C07D 413/04, A61K 31/557

(21) Application number: **90303537.6**

(22) Date of filing: **02.04.90**

(54) **Interphenylène 7-oxabicycloheptyl substituted heterocyclic amide prostaglandin analogs useful in the treatment of thrombotic and vasopastic disease.**

(30) Priority: **03.04.89 US 334070**

(43) Date of publication of application:
**10.10.90 Bulletin 90/41**

(45) Publication of the grant of the patent:
**15.03.95 Bulletin 95/11**

(84) Designated Contracting States:
**AT BE CH DE DK ES FR GB GR IT LI LU NL SE**

(56) References cited:
**EP-A- 0 220 848**
**US-A- 4 663 337**

(73) Proprietor: **E.R. SQUIBB & SONS, INC.**
**Lawrenceville-Princeton Road**
**Princeton**
**New Jersey 08543-4000 (US)**

(72) Inventor: **Misra, Raj Narain**
**12 Eaton Place**
**Hopewell,**
**New Jersey (US)**

(74) Representative: **Thomas, Roger Tamlyn et al**
**D. Young & Co.**
**21 New Fetter Lane**
**London EC4A 1DA (GB)**

**EP 0 391 652 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to interphenylene 7-oxabicycloheptyl substituted heterocyclic amide prostaglandin analogs which are cardiovascular agents useful, for example, in the treatment of thrombotic and/or vasospastic disease, and have good in vivo stability and good duration of action. These compounds have the structural formula

I

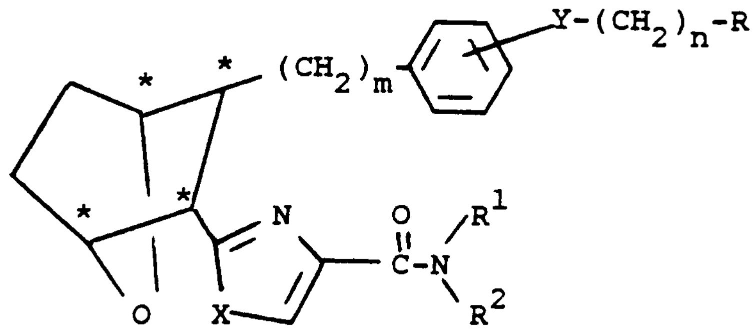

and including all stereoisomers thereof, wherein

m is 1, 2 or 3; n is 0, 1, 2, 3 or 4, Y is O, vinyl or a single bond, with the proviso that when n is 0, Y is a single bond;

R is $CO_2H$, $CO_2$ alkyl, $CO_2$alkali metal, $CH_2OH$, $CONHSO_2R^3$, $CONHR^{3a}$ or 5-tetrazolyl, with the proviso that when R is 5-tetrazolyl, n cannot be 0; X is O, S or NH;

$R^1$ is hydrogen, alkyl, alkenyl, lower alkynyl, aralkyl, aryl, cycloalkyl, cycloalkylalkyl, saturated heterocycle, saturated heterocyclicalkyl, aromatic heterocycle, aromatic heterocyclicalkyl or amido which is optionally substituted with an alkyl, aryl, cycloalkyl, or cycloalkylalkyl;

$R^2$ is hydrogen, alkyl, aryl, or aralkyl; or $R^1$ and $R^2$ together with the nitrogen to which they are linked may form a 5- to 8-membered ring; and

$R^3$ is alkyl, aryl or aralkyl.

$R^{3a}$ is hydrogen, alkyl, aryl or aralkyl; encompassing the following types of compounds:

IA

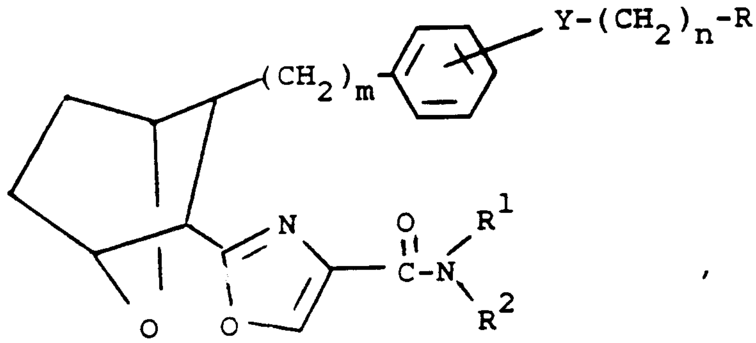

,

IB

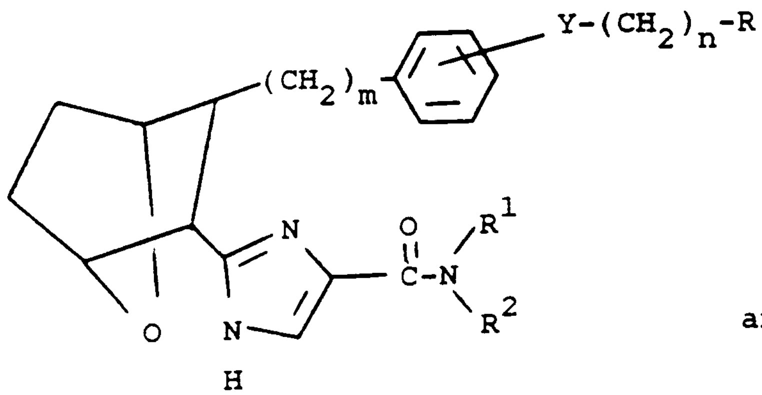

and

IC

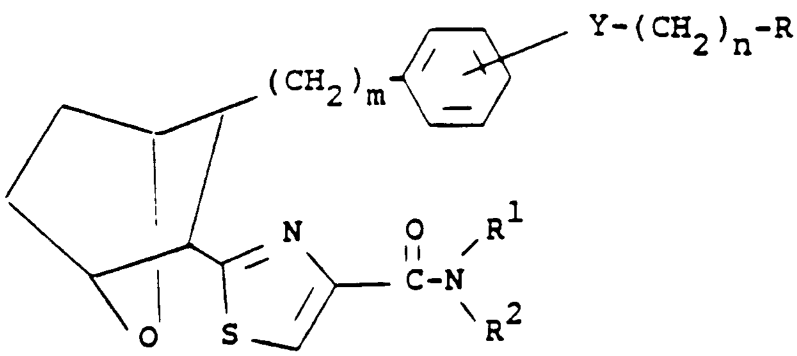

Other compounds of the invention are set out in claims 15-18.

The term "lower alkyl" or "alkyl" as employed herein includes both straight and branched chain radicals of up to 12 carbons, preferably 1 to 8 carbons, such as methyl, ethyl, propyl, isopropyl, butyl, t-butyl, isobutyl, pentyl, hexyl, isohexyl, heptyl, 4,4-dimethylpentyl, octyl, 2,2,4-trimethylpentyl, nonyl, decyl, undecyl, dodecyl, the various branched chain isomers thereof, and the like as well as such groups including 1, 2 or 3 halo substituents, an aryl substituent, an alkyl-aryl substituent, a haloaryl substituent, a cycloalkyl substituent or an alkylcycloalkyl substituent.

The term "cycloalkyl" includes saturated cyclic hydrocarbon groups containing 3 to 12 carbons, preferably 3 to 8 carbons, which include cyclopropyl, cyclobutyl, cyclopentyl, cyclohexyl, cycloheptyl, cyclooctyl, cyclodecyl and cyclododecyl, any of which groups may be substituted with substituents such as halogen, lower alkyl and/or lower alkoxy.

The term "aryl" or "Ar" as employed herein refers to monocyclic or bicyclic aromatic groups containing from 6 to 10 carbons in the ring portion, such as phenyl, naphthyl. Aryl (or Ar), phenyl or naphthyl may include substituted aryl, substituted phenyl or substituted naphthyl, which may include 1 or 2 substituents

on either the phenyl or naphthyl such as lower alkyl, trifluoromethyl, halogen (Cl, Br or F), lower alkoxy, alkylthio, alkylsulfinyl, and/or alkylsulfonyl.

The term "aralkyl", "aryl-alkyl" or "aryl-lower alkyl" as used herein refers to lower alkyl groups as discussed above having an aryl substituent, such as benzyl.

The term "lower alkoxy", "alkoxy" or "aralkoxy" includes any of the above lower alkyl, alkyl or aralkyl groups linked to an oxygen atom.

The term "halogen" or "halo" as used herein refers to chlorine, bromine, fluorine or iodine with chlorine being preferred.

The term saturated heterocycle as used herein refers to a 5-, 6- or 7-membered saturated ring which includes 1 or 2 hetero atoms such as nitrogen, oxygen and/or sulfur, such as

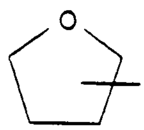 , 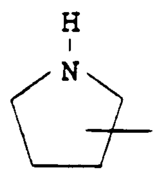 , 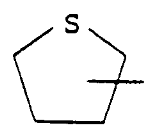 ,

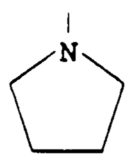 , 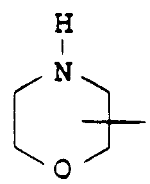 , 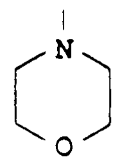

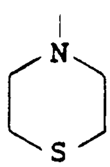 , 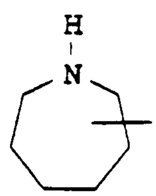 , 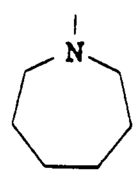

and the like.

The term aromatic heterocycle or heteroaromatic as used herein refers to a 5- or 6-membered aromatic ring which includes 1 or 2 hetero atoms such as nitrogen, oxygen or sulfur, such as

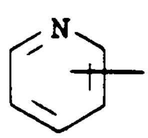 , 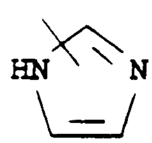 , 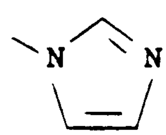 ,

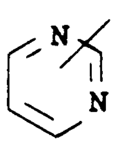 , 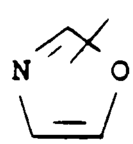 , 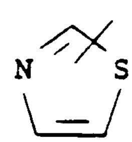

and the like

Preferred are those compounds of formula I wherein m is 1, n is 2, Y is a single bond, X is O, R is $CO_2H$, $R^1$ is substituted alkyl and $R^2$ is H or alkyl, and Y-$(CH_2)_n$-R is in the ortho or meta position.

The various compounds of the invention may be prepared as outlined in the claims.

The compounds of formula I of the invention are preferably prepared as follows.

Compounds of the invention where Y is a single bond, n is 1, 2, 3 or 4 and X is O are prepared starting with bromophenylalkyl alcohol A

A

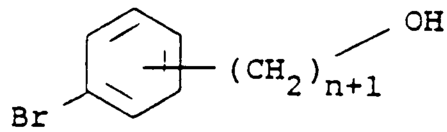

wherein n is 1, 2, 3 or 4
which is treated with a protecting compound such as t-butylchlorodiphenylsilane, in the presence of an amine base such as triethylamine and an inert solvent, employing conventional procedures, to form the protected bromophenylalkyl compound B

B

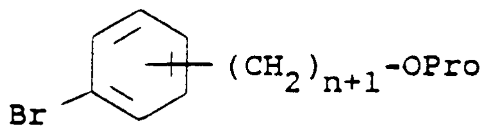

wherein Pro represents a protecting group.

Examples of protecting compounds suitable for use herein in reacting with bromophenalkyl alcohol A include but are not limited to

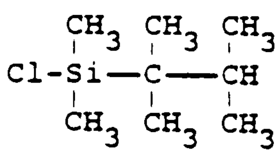

(chlorodimethyl-thexylsilane ,

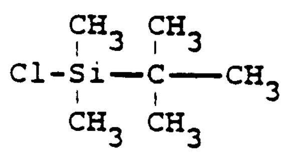

(chloro-dimethyl t-butylsilane) or

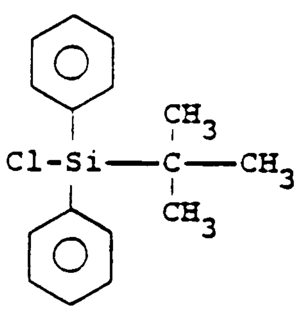

(t-butylchlorodiphenylsilane)

The protected compound B is then transmetallated by treatment with t-$C_4H_9Li$ or n-$C_4H_9Li$ in the presence of diethyl ether or tetrahydrofuran at reduced temperature of from -100° to 0°C (or is subjected to a Grignard reaction by treatment with magnesium in the presence of an inert organic solvent such as tetrahydrofuran (THF) or diethyl ether) and then is condensed with (exo)octahydro-5,8-epoxy-1H-benzopyran-3-ol or (exo)octahydro-4,7-epoxyisobenzofuran-1-ol (prepared as described in U.S. Patent No. 4,143,054) of the structure C

C

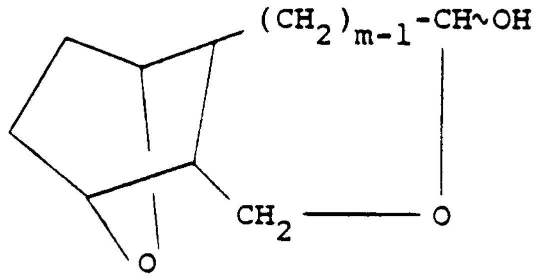

employing a molar ratio of C:B of within the range of from 1:2 to 1:4, in the presence of an inert organic solvent such as THF at a reduced temperature of from -78 to 0 ° C, to form the condensed 7-oxabicycloheptane compound II

II

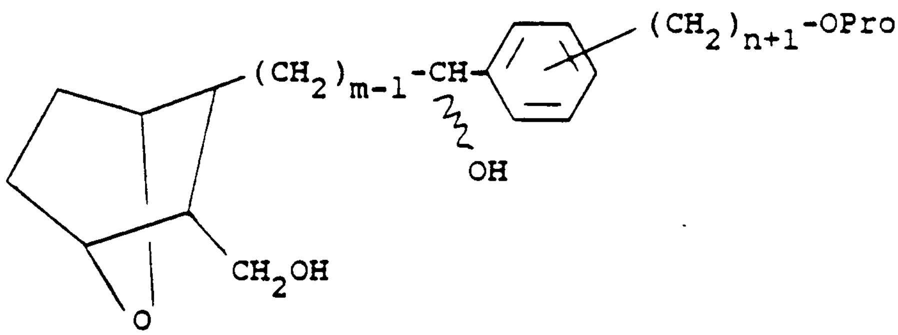

The condensed compound II is then subjected to hydrogenolysis by treatment with hydrogen in the presence of a catalyst such as palladium hydroxide on charcoal in acetic acid or an inert organic solvent such as ethyl acetate, to form the alcohol III

III

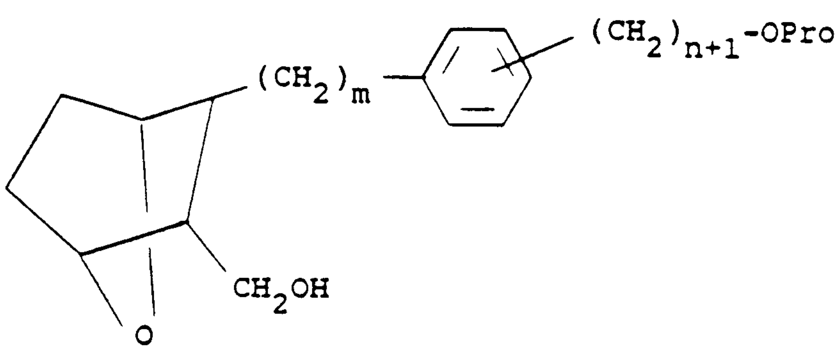

In an alternative procedure, when the protecting group (Pro) in III is thexyldimethylsilyl or t-butyldimethylsilyl, alcohol III may be subjected to acetylation by treatment with acetyl chloride in the presence of pyridine and methylene chloride to acetylate the free alcohol and the so-formed acetate is treated with aqueous hydrofluoric acid in the presence of acetonitrile to cleave off the silyl protecting group to form IIIA

IIIA

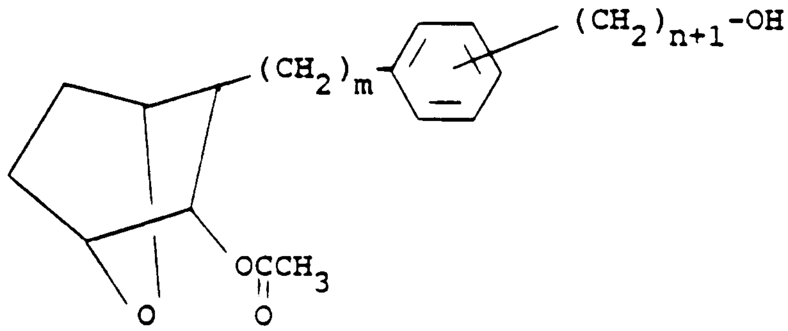

IIIA is then treated with a protecting compound such as t-butyldiphenylsilyl chloride in the presence of 4-dimethylaminopyridine and triethylamine and methylene chloride to add the protecting group and the acetate is removed by treatment with aqueous hydroxide in tetrahydrofuran or methyllithium in the presence of an inert solvent such as diethyl ether to form IIIB

IIIB

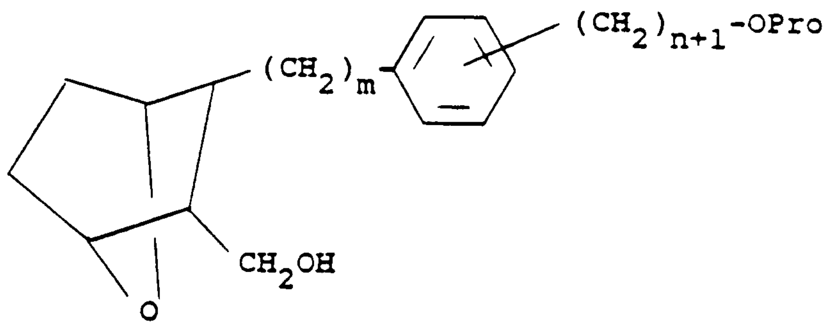

(where Pro is t-butyldiphenylsilyl)

The protected alcohol III or IIIB is then subjected to a Jones oxidation wherein a solution of protected alcohol III or IIIB in acetone cooled to from -10 to 25°C is treated with Jones reagent (that is, $CrO_3$ dissolved or suspended in sulfuric acid in the presence of water, prepared as described in Fieser & Fieser, "Reagents for Organic Synthesis," Vol. 1, p. 142 (1967)) to form acid IV

IV

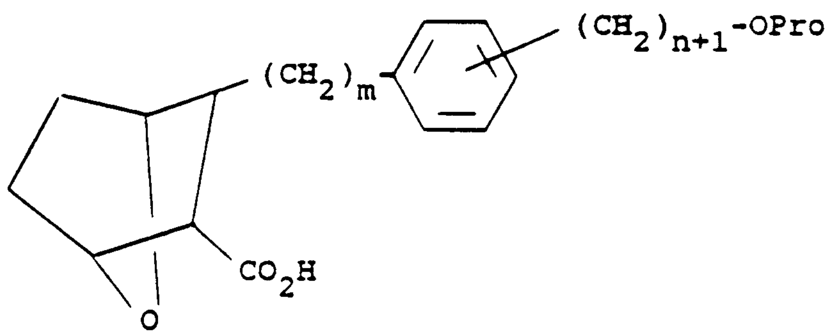

Acid IV, in an inert organic solvent, such as tetrahydrofuran, is then made to undergo a carbodiimide coupling reaction with amine hydrochloride D

D

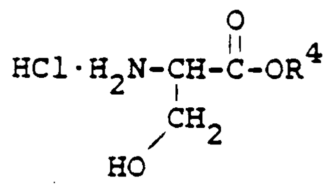

where $R^4$ is lower alkyl such as methyl or ethyl, or arylalkyl, such as benzyl, in the presence of dicyclohexylcarbodiimide (DCC) or (3-dimethylaminopropyl)-3-ethylcarbodiimide (WSC) and 1-hydroxybenzotriazole and triethylamine under an inert atmosphere such as argon employing a molar ratio of D:IV of within the range of from 1.2:1 to 1:1, to form hydroxyamide V

V

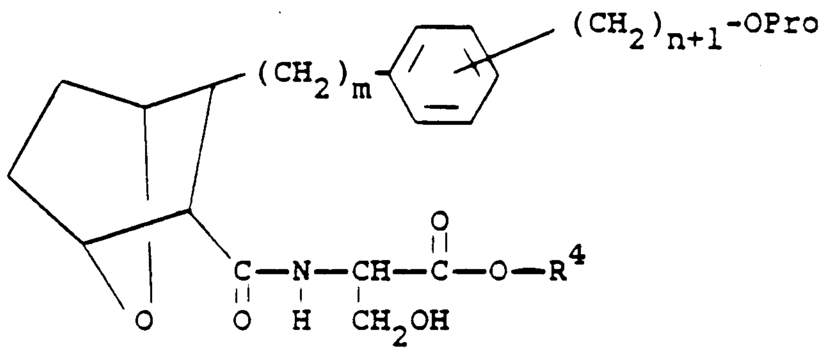

Hydroxyamide V is then subjected to cyclodehydration wherein a solution of V in an inert organic solvent such as tetrahydrofuran, acetonitrile or chloroform , under an inert atmosphere such as argon, is treated with triphenylphosphine (employing a molar ratio of V:triphenylphosphine of from 1:1 to 5:1) and carbon tetrachloride in the presence of an amine base such as triethylamine or diisopropylethylamine, to form oxazoline VI

VI

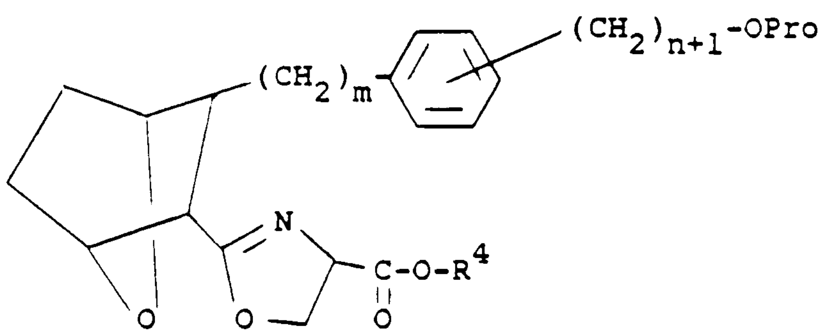

Oxazoline VI is oxidized by treatment with manganese dioxide or preferably nickel peroxide to form the oxazole VII

VII 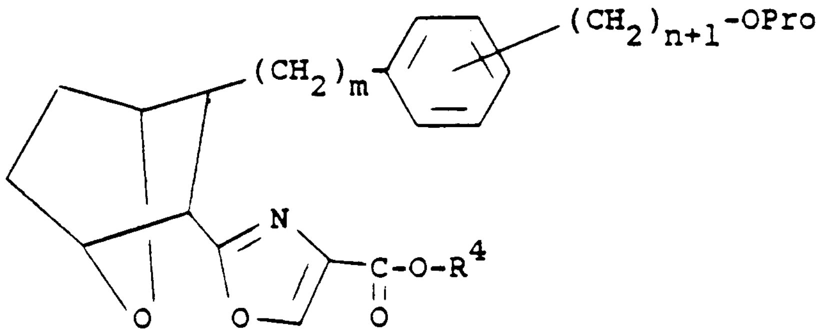

Oxazole VII is converted to the corresponding acid by treating VII with a base, such as lithium hydroxide, sodium hydroxide or potassium hydroxide to form the corresponding alkali metal salt, followed by neutralization with an acid, such as dilute hydrochloric acid or oxalic acid to form acid compound VIII

VIII 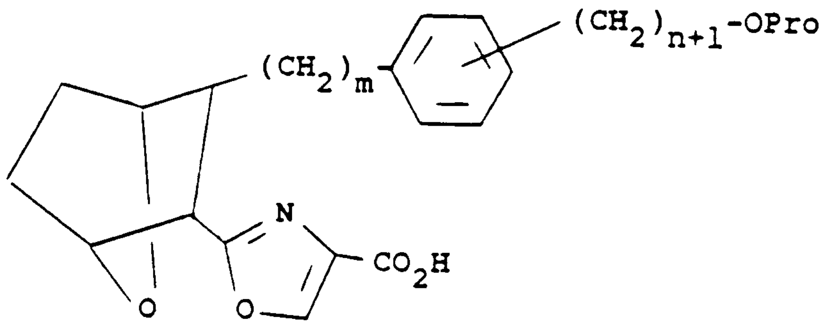

Acid VIII is converted to the corresponding acid chloride by treating VIII with oxalyl chloride optionally in the presence of catalytic amounts of dimethylformamide, and a non-polar solvent such as benzene, toluene or methylene chloride. The so-formed acid chloride is dissolved in an inert solvent such as methylene chloride or toluene cooled to a temperature within the range of from -10 °C to +10 °C, and amine base such as triethylamine or pyridine and amine E are added

E 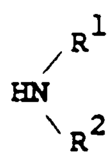

or a salt thereof, employing a molar ratio of E:VIII of within the range of from 1.1:1 to 1.5:1, form the oxazole IX

IX

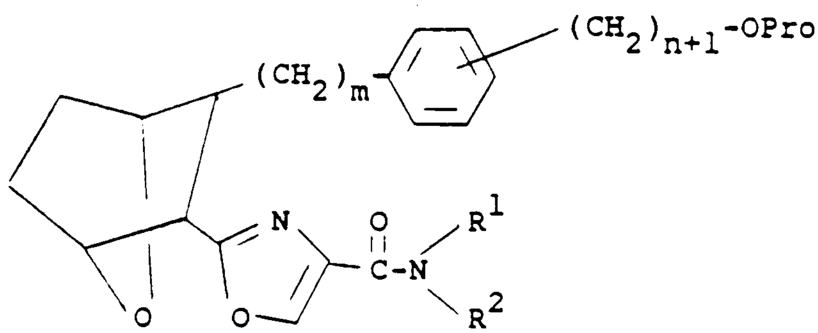

Silyl ether IX is then deprotected by treatment with aqueous hydrofluoric acid in the presence of acetonitrile and methylene chloride and is then subjected to a Jones oxidation employing procedures described hereinbefore to form the oxazole ID of the invention

ID

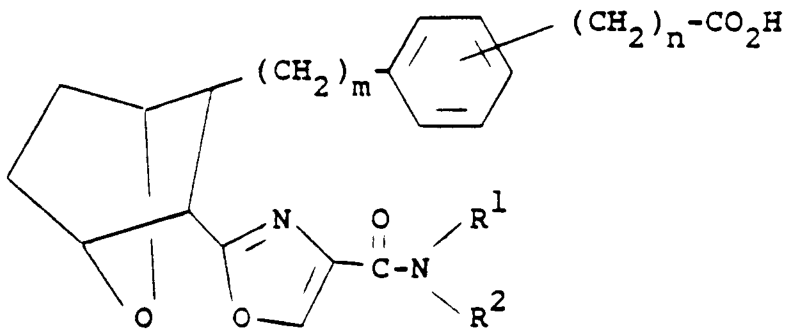

In an alternative procedure, compounds of formula I wherein Y is a single bond and X is O may be prepared starting with alcohol III by protecting the alcohol function thereof by treatment, for example, with a solution of acetic anhydride, pyridine and 4-dimethylaminopyridine to form the protected alcohol X

X

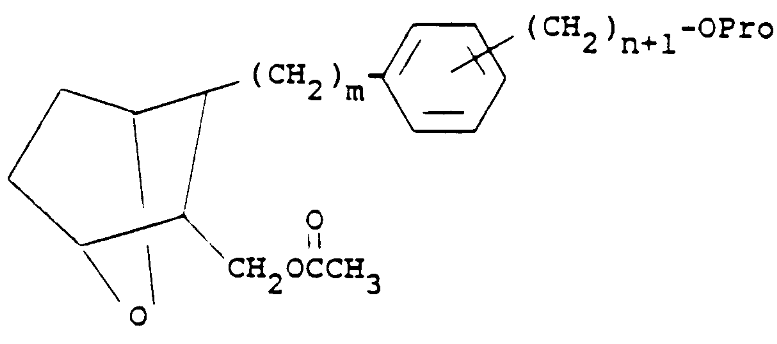

Alternatively, compound II can be protected by treatment with, for example, a solution of acetic anhydride and pyridine to form compound XI

XI

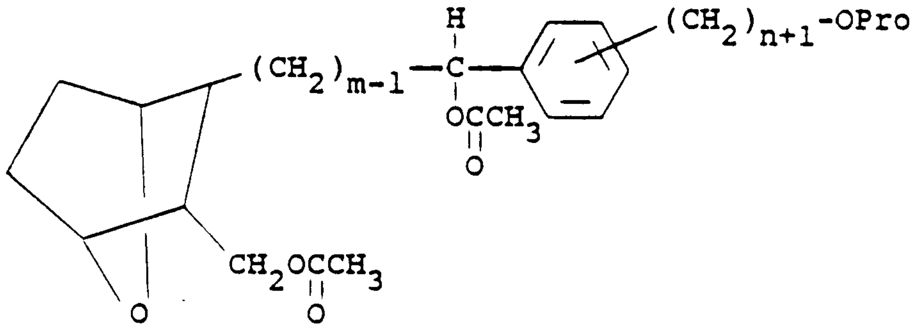

which is then subjected to hydrogenolysis as described above to provide compound X.

The protected alcohol X is subjected to a Jones oxidation employing procedures described hereinbefore to form crude acid which is deprotected by reaction with aqueous hydroxide in the presence of inert organic solvent such as THF and then esterified, for example, by treatment with diazoalkane, such as diazomethane, or acidic alcohol such as methanol HCl, to form the alcohol ester XII

XII

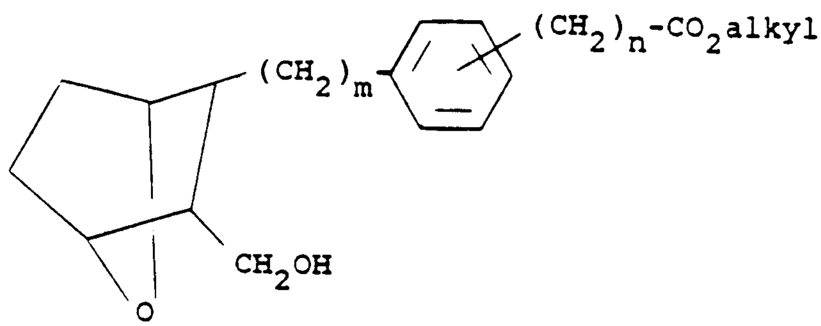

Next, the alcohol ester XII is subjected to a Jones oxidation to form the acid XIII

XIII

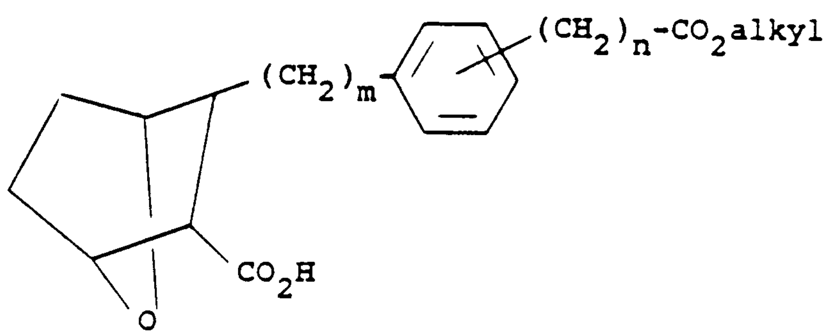

The acid XIII is then made to undergo a carbodiimide coupling reaction with amine hydrochloride D, where $R^4$ is benzyl, as described hereinbefore (with respect to coupling of acid IV) to form the amide XIV

XIV

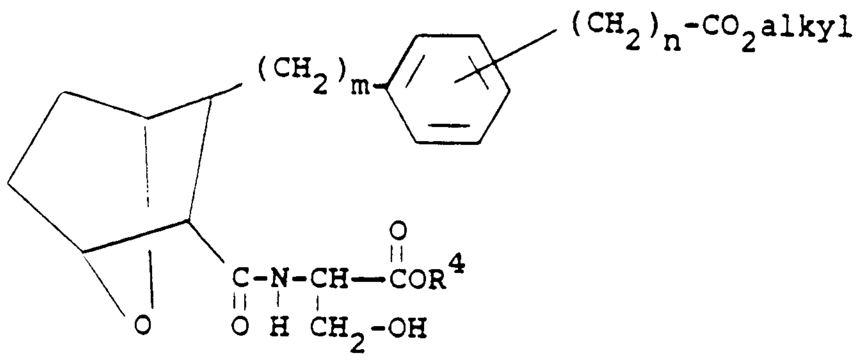

Amide XIV is then subjected to cyclodehydration (using a procedure simiar to the cyclodehydration of amide V) to form oxazoline XV

XV

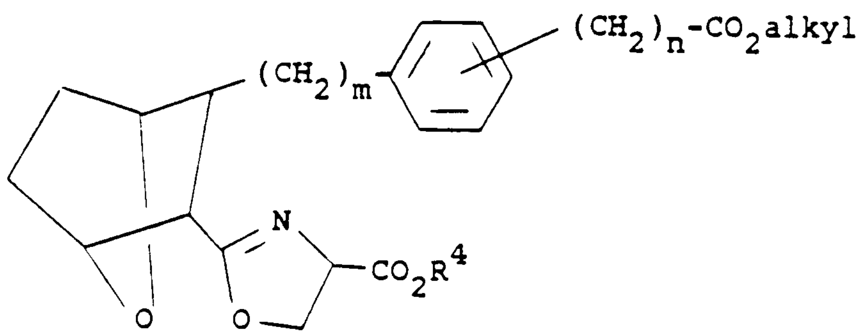

which is made to undergo oxidation using manganese dioxide or preferably nickel peroxide to form the oxazole XVI

XVI

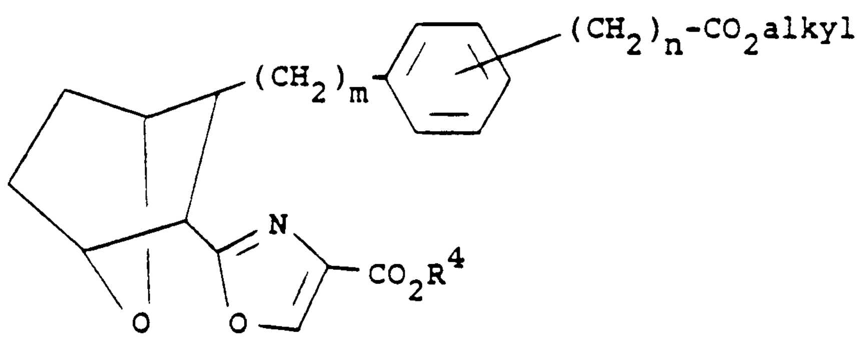

Oxazole XVI is then deprotected to remove $R^4$, for example, by treatment with palladium hydroxide on charcoal and hydrogen in the presence of an inert solvent such as ethyl acetate, to form the corresponding acid XVII

XVII

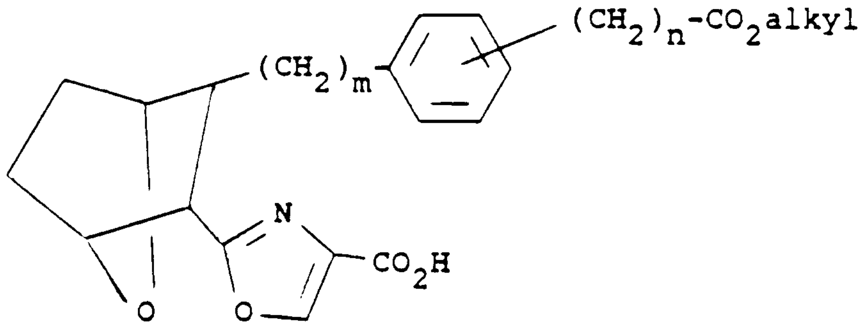

Acid XVII is then converted to the corresponding acid chloride employing a procedure similar to that described with respect to acid VIII and the resulting acid chloride is treated with amine E employing a procedure and molar ratio of E:XVII similar to that described hereinbefore with respect to acid VIII to form the ester of the invention IE

IE

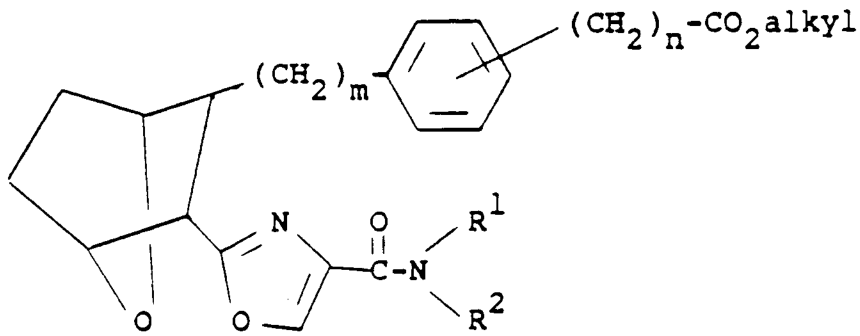

Ester IE may then be hydrolyzed by treatment with alkali metal base and then aqueous acid to form the corresponding acid ID.

Compounds of the invention wherein Y is O and X is O may be prepared as follows:

Bromophenol $\underline{A}^1$

$\underline{A}^1$

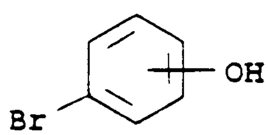

is treated with a protecting compound such as chloro-t-butyldimethylsilane, benzyl bromide or bromomethyl methyl ether, preferably benzyl bromide or bromomethyl methyl ether for ortho-bromophenol, employing conventional procedures to form the protected bromophenyl compound $\underline{B}^1$

$\underline{B}^1$

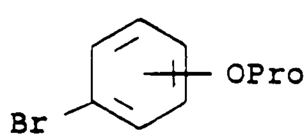

wherein Pro represents a protecting group.

Examples of protecting compounds suitable for use herein in reacting with bromophenol A[1] include those set out hereinbefore with respect to protection of alcohol A.

Protected compound B[1] is then transmetallated (using a procedure similar to that set out above with respect to transmetallation of B sing n-butyllithium in THF) to form the condensed 7-oxabicycloheptane compound XXII

XXII

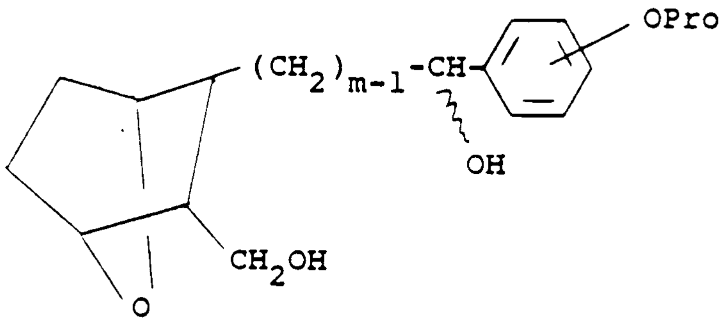

The condensed compound XXII is then subjected to hydrogenolysis by treatment with hydrogen in the presence of a catalyst such as palladium on charcoal in acetic acid, to form the alcohol XXIII in the case where Pro is a silyl or methoxymethyl ether protecting group or to form XXIV directly when Pro is benzyl.

XXIII

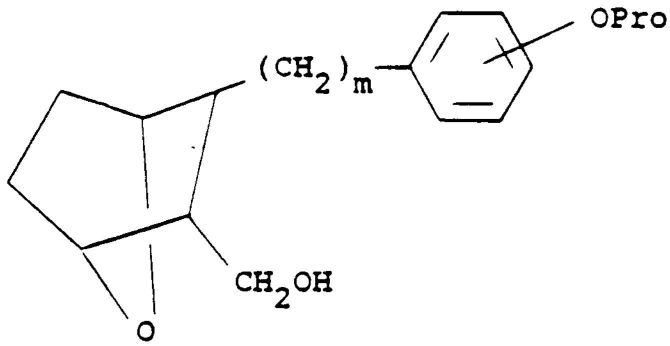

When Pro is a silyl protecting group,compound XXIII is deprotected by treatment with, for example, a solution of acetonitrile and aqueous hydrofluoric acid to form the deprotected alcohol XXIV

XXIV

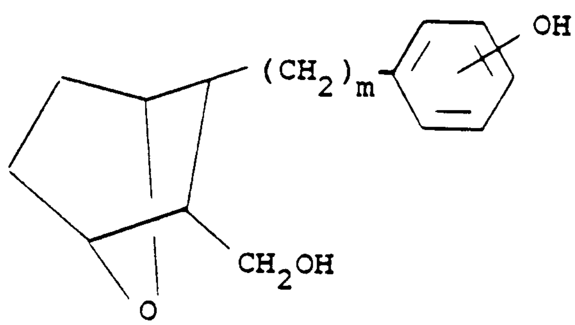

The alcohol XXIV is then alkylated by treating a solution of alcohol XXIV in tetrahydrofuran with a molar equivalent of sodium hydride or one to four equivalents of a carbonate base such as potassium carbonate. The resulting phenoxide solution is alkylated by treating with a haloalkanoic acid ester F

F Hal-$(CH_2)_n$-$CO_2$alkyl

employing a molar ratio of F:XXIV of from 1:1 to 3:1, in the presence of an inert organic solvent such as THF or dimethylformamide or dimethoxyethane, to form ester XXV

XXV

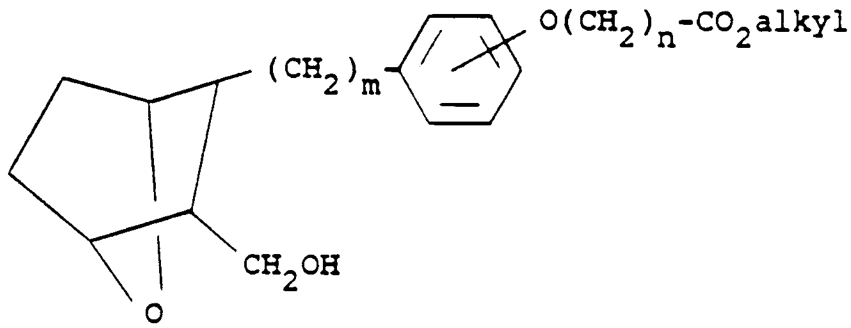

Alternatively, when the protecting group in XXIII is methoxymethyl, the free hydroxyl is protected as a benzyl ether. The methoxymethyl protecting group is removed by treatment with aqueous acid. The resulting phenol is alkylated with ethyl bromoacetate as described above for the alkylation of XXIV. The benzyl protecting group is then removed by hydrogenolysis with palladium hydroxide and hydrogen to give XXV.

Alternatively, alcohol ester starting materials of formula XXV may be prepared by following the procedure as described in U.S. Patent No. 4,536,513.

Next, the alcohol ester XXV is subjected to a Jones oxidation as described hereinbefore with respect to the oxidation of alcohol III, to form acid XXVI

XXVI

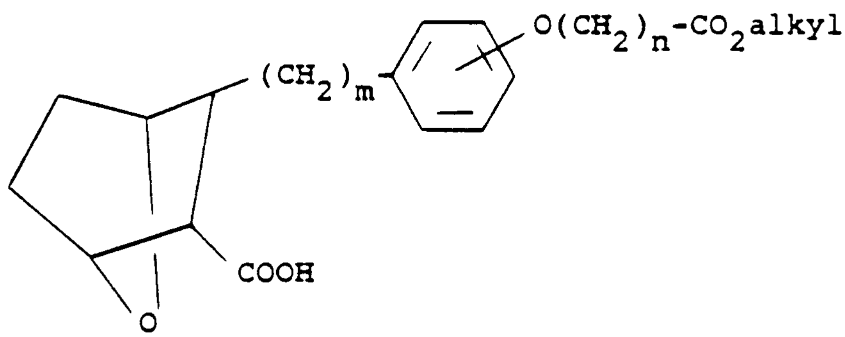

The acid XXVI is then used to prepare compounds of the invention of formula IF and IG using the procedures set out hereinbefore with respect to conversion of acid XIII to ester IF and acid IG

IF

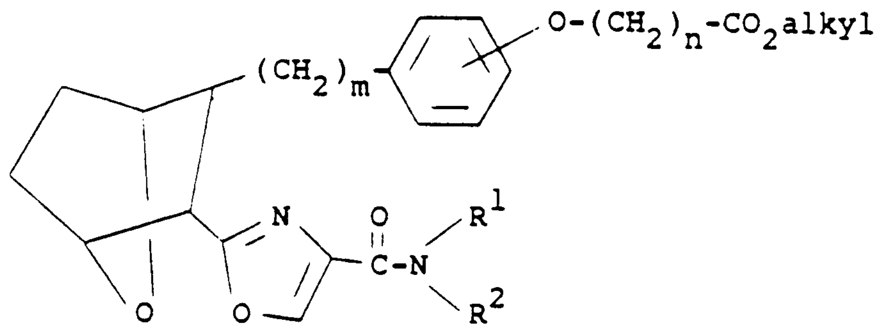

IG

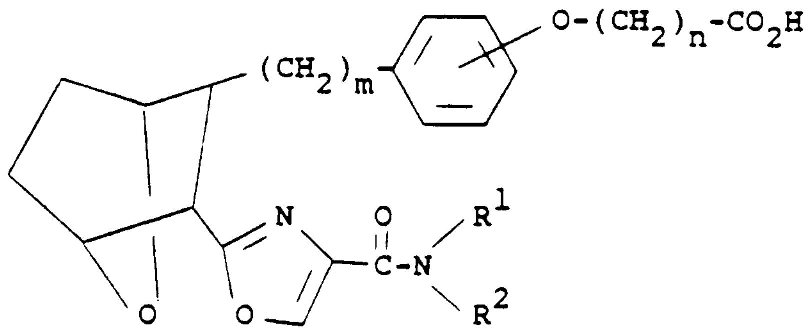

Compounds of the invention wherein Y is a single bond or O and X is S may be prepared starting with acid XIII or XXVI as follows:

Acid XIII or XXVI is reacted with oxalyl chloride, optionally in the presence of catalytic amounts of dimethylformamide, in methylene chloride, to form the corresponding acid chloride which is amidated by reacting with ammonia to form the amide XXVII

XXVII

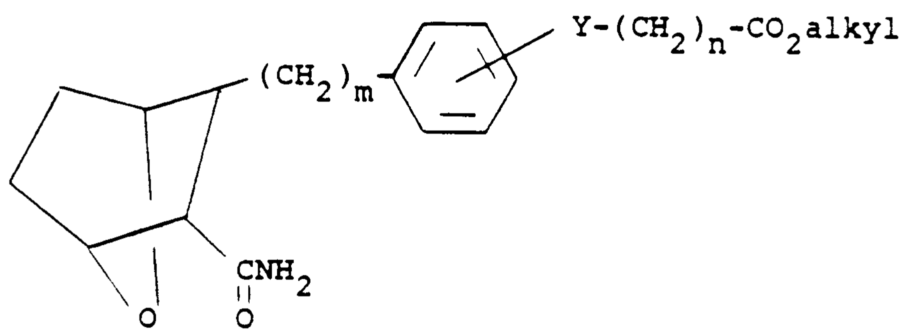

Alternatively, acid XIII or XXVI is reacted with an alkylchloroformate in the presence of an amine such as triethylamine to form the mixed anhydride which is amidated by reacting with methanol-ammonia solution to form amide XXVII.

Amide XXVII is then treated with phosphorus pentasulfide ($P_2S_5$) or Lawesson's Reagent (2,4-bis(4-methoxyphenyl)-1,3-dithia-2,4-diphosphetane-2,4-disulfide) to form the corresponding thioamide XXVIII

XXVIII

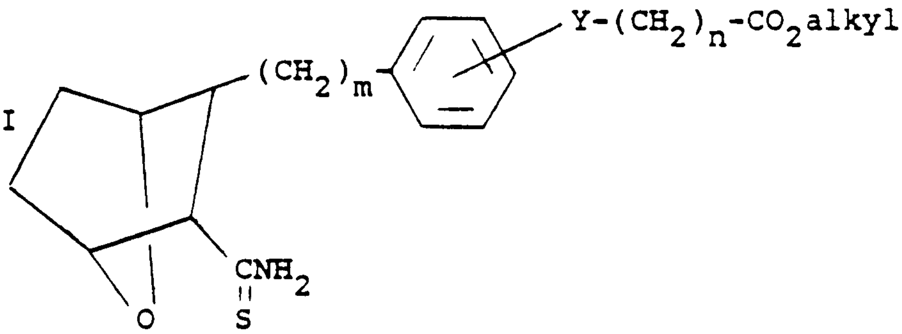

which is treated with bromopyruvic acid

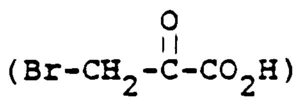

in a polar solvent such as dimethylformamide in the presence of a base such as $K_2CO_3$ employing a molar ratio of XXVIII: bromopyruvic acid of within the range of from 1:1 to 1:1.5 to form thiazoline XXIX

XXIX

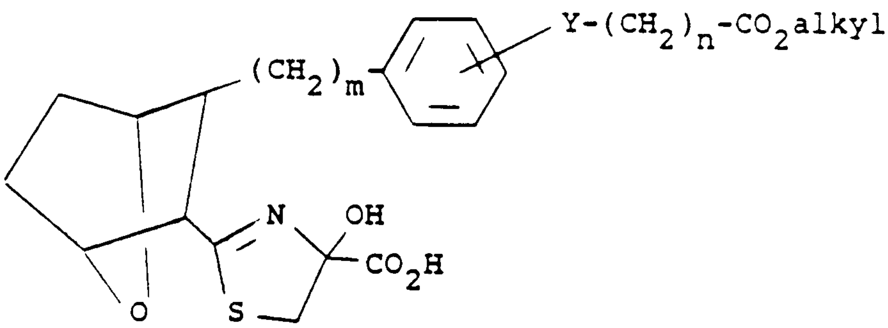

Thiazoline XXIX is then dehydrated by treatment with a sulfonyl chloride such as methanesulfonyl chloride in the presence of a base such as triethylamine to form thiazole acid XXX

XXX

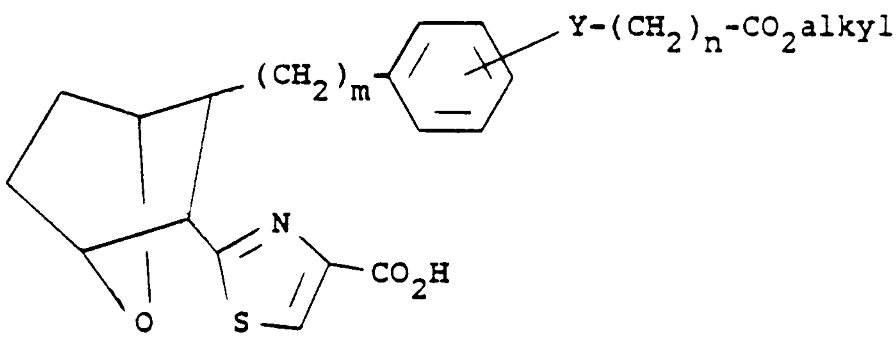

which is then made to undergo a carbodiimide coupling reaction with amine

E 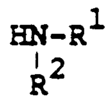

in the presence of DCC or WSC under an inert atmosphere such as argon employing a molar ratio of E:XXX of within the range of from 1:1 to 2:1, to form amide IH

IH 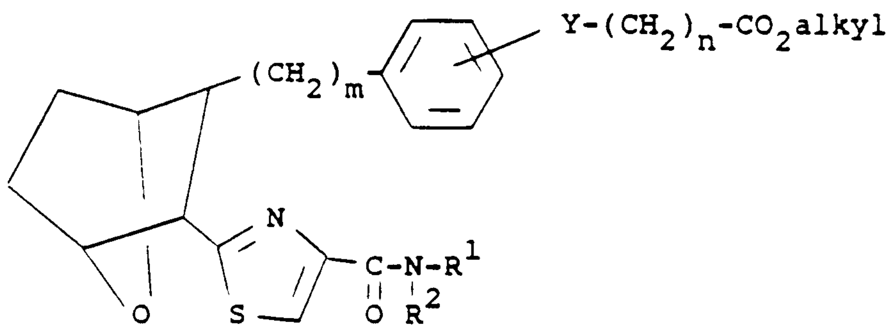

Alternatively, acid XXX can be activated by conversion to the corresponding acid chloride by treating acid XXX with oxalyl chloride in a non-polar solvent such as benzene. The acid chloride is then coupled with amine E using an amine base such as triethylamine or pyridine to form IH.

Compounds of the invention where Y is a single bond or O and X is NH are prepared starting with amide XXVII which is dehydrated by heating at from 80° to 120°C in the presence of pyridine, oxalyl chloride and dimethylformamide to form the nitrile XXXI

XXXI 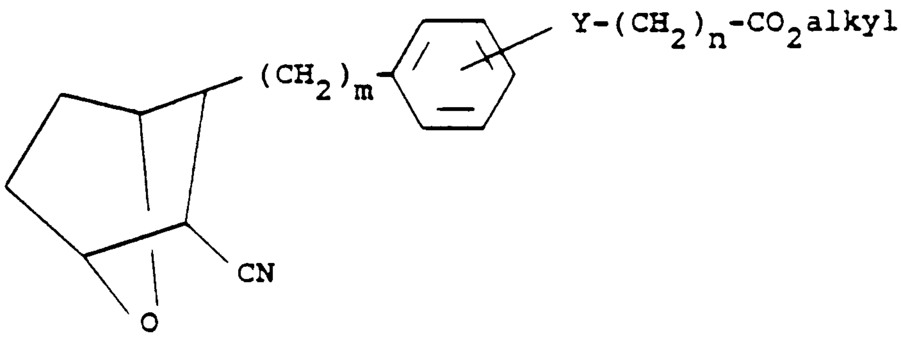

Nitrile XXXI undergoes an addition reaction by treating XXXI with hydrochloric acid and methanol to form the corresponding imino ether salt XXXII

XXXII

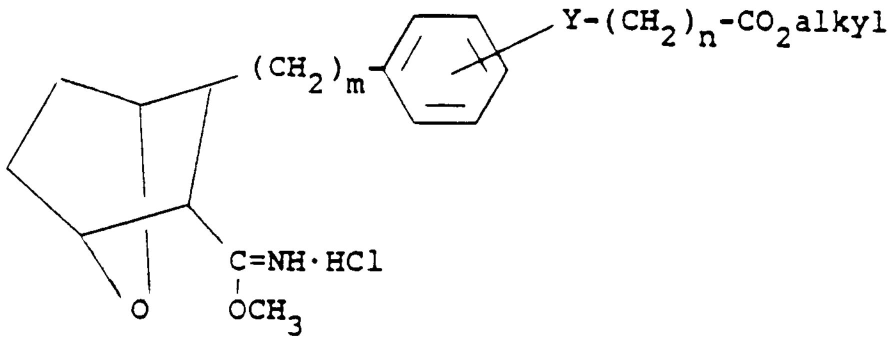

The imino ether XXXII is condensed with amine ester G in the presence of a tertiary amine base, for example triethylamine, diisopropylethylamine and the like,

G

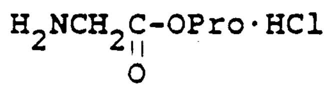

where Pro is a protecting group such as $-CH_2CH_2Si(CH_3)_3$ or $-CH_2OCH_2CH_2Si(CH_3)_3$ to form ester XXXIII

XXXIII

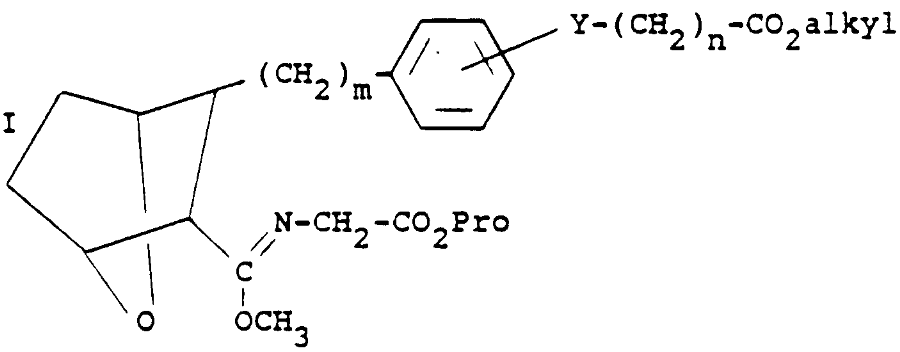

which is treated with a basic salt such as potassium methoxide, potassium t-butoxide or sodium ethoxide, in the presence of ethyl formate to form the ester salt XXXIV

XXXIV

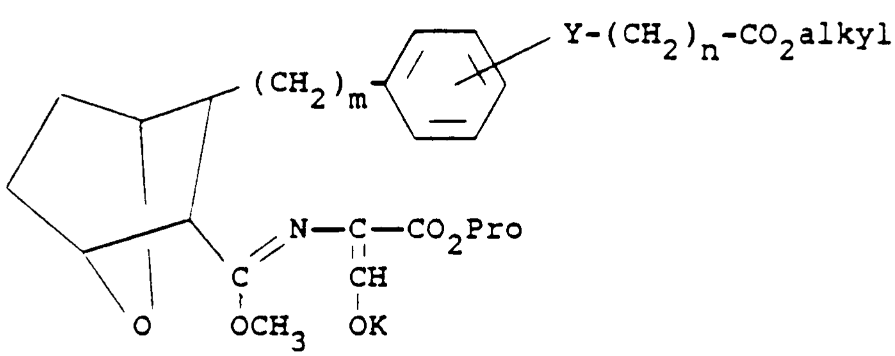

The ester XXXIV is dissolved in ammonia to form the imidazole XXXV

XXXV

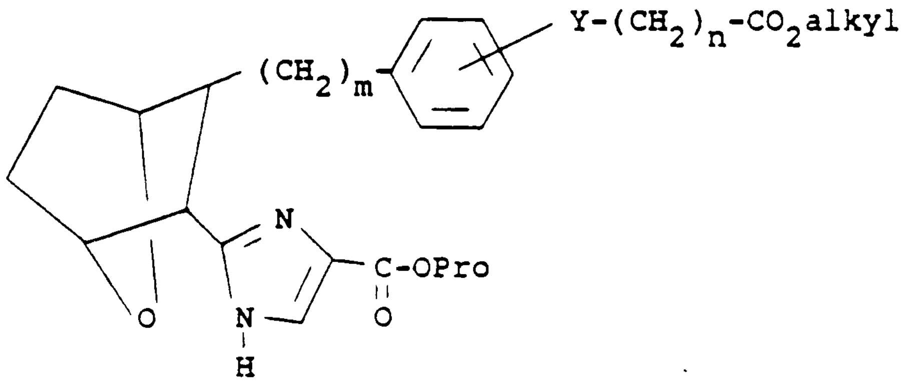

The imidazole XXXV is deprotected by treatment with tetra-n-butylammonium fluoride to form the acid XXXVI

XXXVI

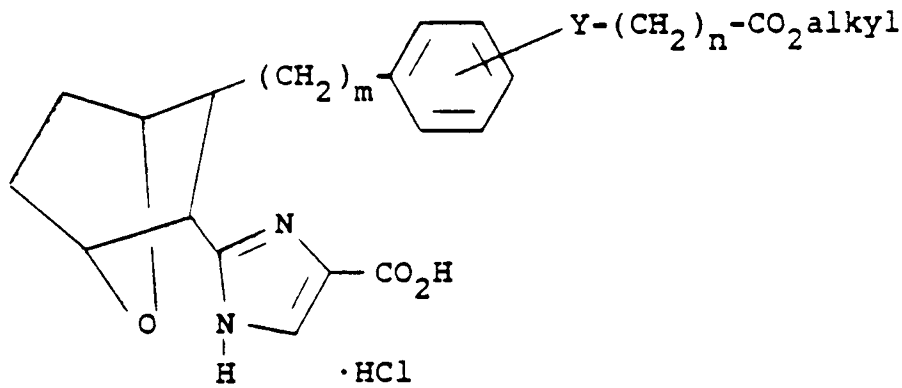

Acid XXXVI is then made to undergo a coupling reaction by conversion of XXXVI to the acid chloride (using the procedure used in converting XVII to IE) and then condensation with amine E in the presence of an amine base such as pyridine or triethylamine under an inert atmosphere such as argon employing a molar ratio of E:XXXVI of within the range of from 0.8:1 to 1.2:1 to form amide IJ

IJ

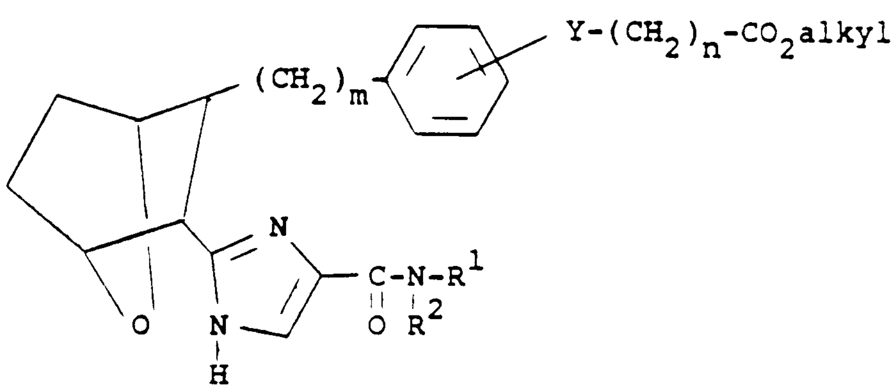

Compounds of the invention wherein n is 0 and Y is a single bond, that is benzoic acids or derivatives thereof of the structure IK

IK

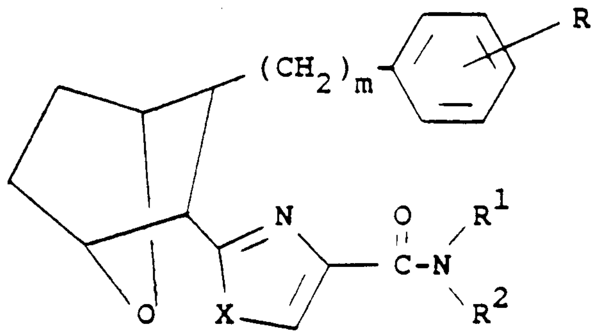

may be prepared starting with bromobenzyl alcohol $\underline{A}^2$

$\underline{A}^2$

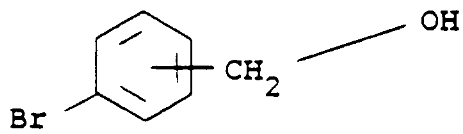

which is treated with a protecting compound such as t-butylchlorodiphenylsilane, in the presence of 4-dimethylaminopyridine and an amine base such as triethylamine and an inert solvent, such as methylene chloride, employing conventional procedures, to form the protected bromobenzyl compound $\underline{B}^2$

$\underline{B}^2$

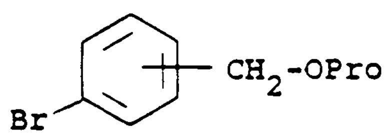

wherein Pro represents a protecting group.

Examples of protecting compounds suitable for use herein are as set out hereinbefore in reacting with bromophenalkyl alcohol $\underline{A}$.

The protected compound $\underline{B}^2$ is then transmetallated by treatment with t-$C_4H_9Li$ or n-$C_4H_9Li$ in the presence of diethyl ether or tetrahydrofuran at reduced temperature of from -100° to 0°C (or is subjected to a Grignard reaction by treatment with magnesium in the presence of an inert organic solvent such as tetrahydrofuran (THF) or diethyl ether) and then is condensed with (exo)octahydro-5,8-epoxy-1H-benzopyran-3-ol or (exo)octahydro-4,7-epoxyisobenzofuran-1-ol (prepared as described in U.S. Patent No. 4,143,054) of the structure $\underline{C}$

$\underline{C}$

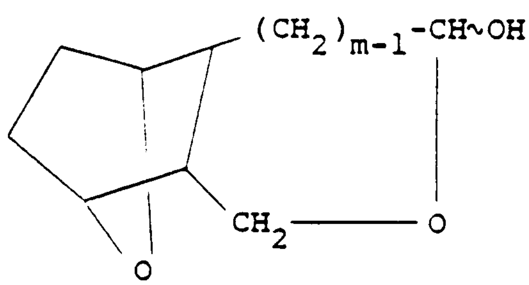

employing a molar ratio of $\underline{C}$:$\underline{B}^2$ of within the range of from 1:2 to 1:4, in the presence of an inert organic solvent such as THF at a reduced temperature of from -78 to 0°C, to form the condensed 7-oxabicycloheptane compound IIA

IIA

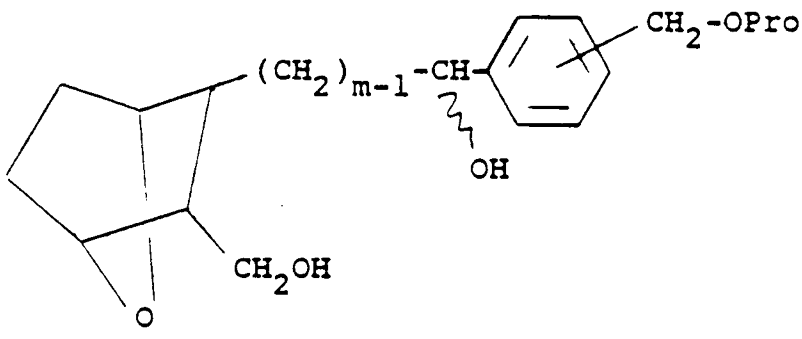

Compound IIA is then protected by treatment with, for example, a solution of acetic anhydride and pyridine in the presence of 4-dimethylaminopyridine to form compound XIA

XIA

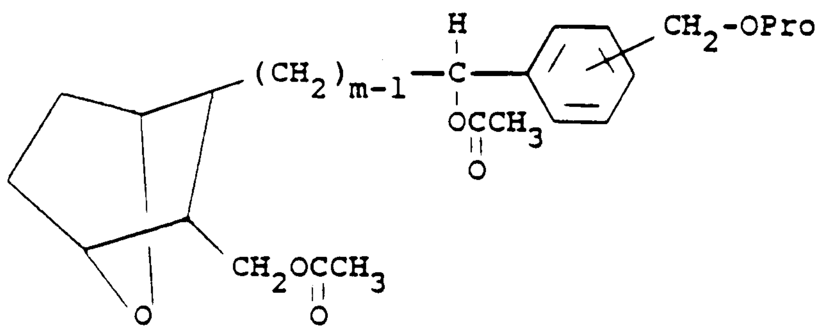

The protected alcohol XIA is subjected to a Jones oxidation employing procedures described hereinbefore to form crude acid which is deprotected by reaction with aqueous hydroxide in the presence of inert organic solvent such as THF and then esterified, for example, by treatment with diazoalkane, such as diazomethane, or acidic alcohol, to form the alcohol ester XIIA

XIIA

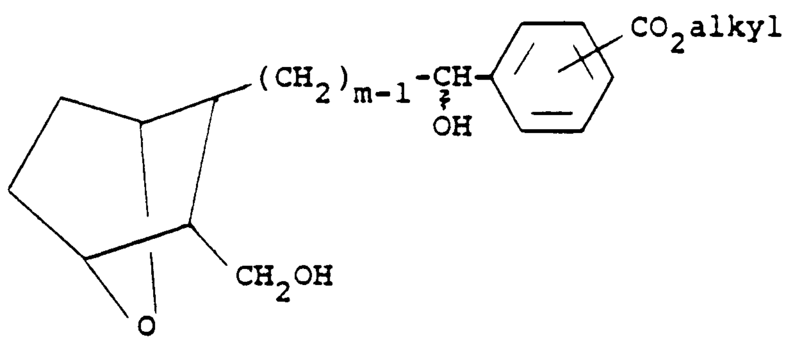

The alcohol ester is then subjected to hydrogenolysis as described above to provide alcohol ester compound XIIB

XIIB

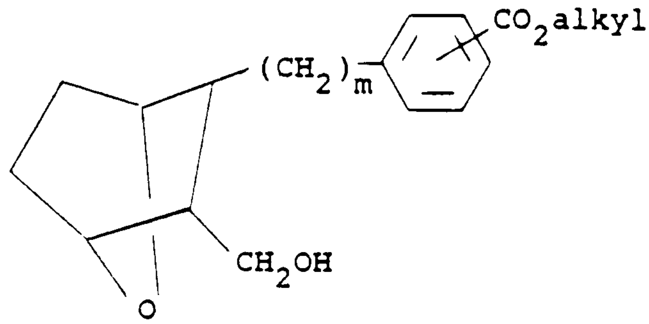

Next, the alcohol ester XIIB is subjected to a Jones oxidation to form the acid XIIIA

XIIIA

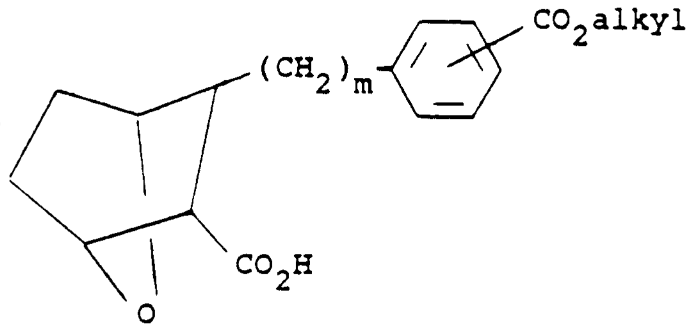

The acid XIIIA is then used in place of acid XIII to form the corresponding benzoic acids of structures IK, including

IL

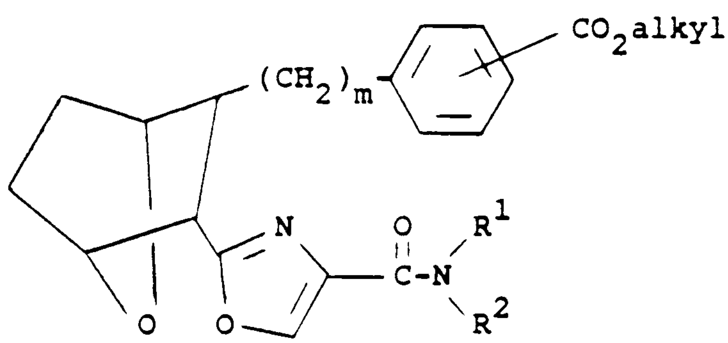

IM

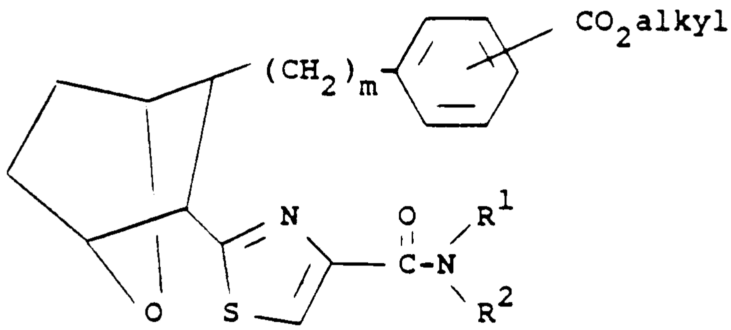

IN

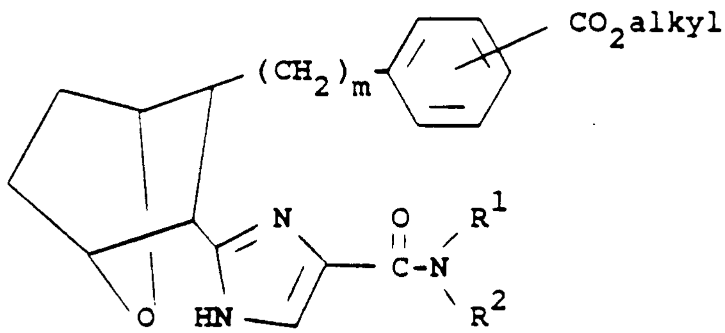

The esters IE, IF, IH, IJ, IL, IM and IN may be converted to the corresponding acids, that is IO

IO

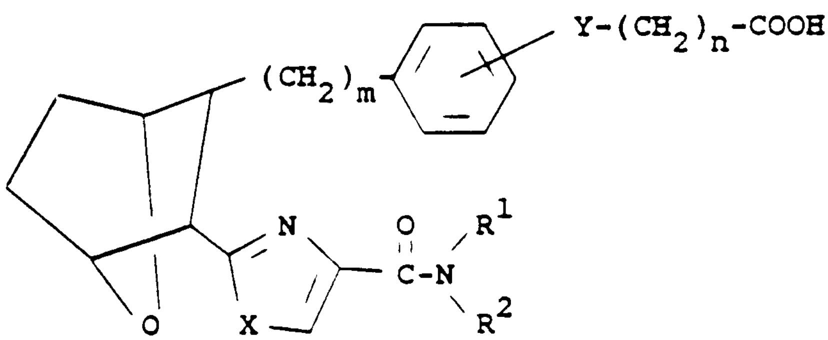

by treating the esters with a base, such as lithium hydroxide, sodium hydroxide or potassium hydroxide to form the corresponding alkali metal salt, followed by neutralization with an acid, such as dilute hydrochloric acid or oxalic acid to form the acid compounds of the invention.

Compounds of the invention wherein R is $CONHSO_2R^3$, that is IP

IP

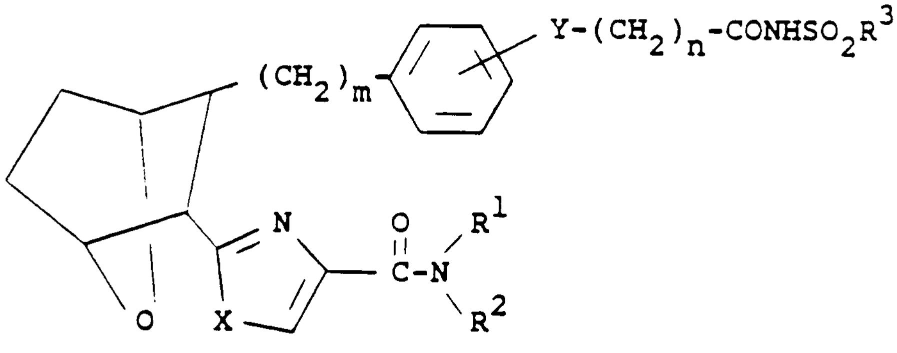

are prepared by treating acid IO with a sulfonamide of the structure H

H 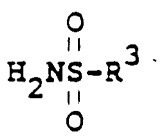

in the presence of a coupling agent such as carbonyldiimidazole or WSC in the presence of an amine such as dimethylaminopyridine under an inert atmosphere such as argon employing a molar ratio of H:IK of within the range of from 0.8:1 to 1.2:1, to form sulfonamide IP.

Compounds of the invention wherein R is 5-tetrazolyl, and Y is a single bond, that is IQ where Y is a single bond

IQ 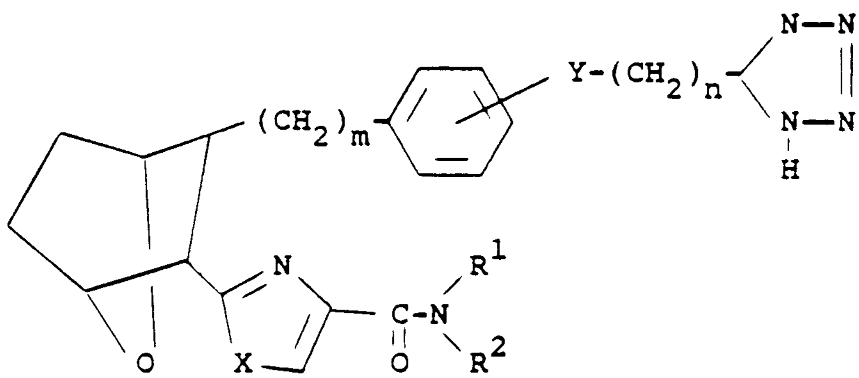

are prepared by subjecting esters IE, IH, IJ, IL, IM or IN to reduction with a hydride reagent such as lithium borohydride or sodium borohydride to afford alcohol XXXVII

XXXVII 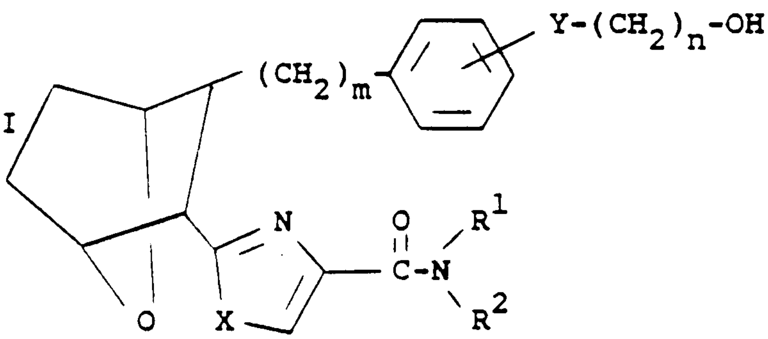

which is converted to the bromide on treatment with triphenylphosphonium dibromide in an inert solvent such as toluene. The bromide is then converted to nitrile XXXVIII on treatment with an alkali metal cyanide in a polar solvent such as methanol/water.

XXXVIII 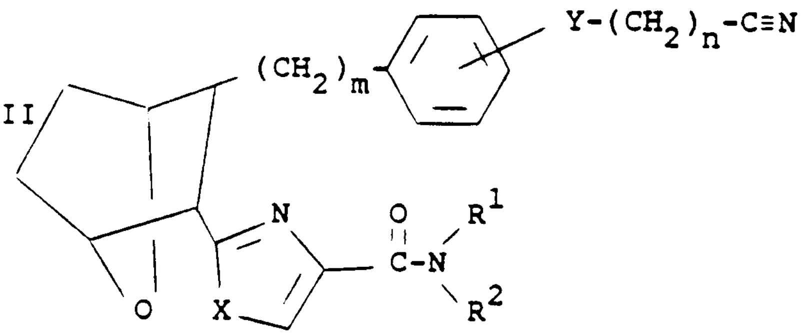

Compounds of the invention wherein R is 5-tetrazolyl and Y = O, that is IQ where Y is O, are prepared by conversion of alcohol XXIII to ether XXXIX using the procedures set out hereinbefore for the conversion of XII to esters IE, IH and IJ

XXXIX 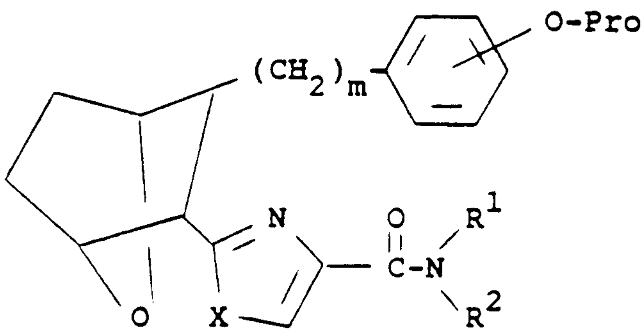

which are converted to nitrile XXXVIII by deprotection, for example with aqueous HF, followed by alkylation with halonitrile J in the

J $X-(CH_2)_n-CN$

presence of a base such as sodium hydride or potassium carbonate.

The nitrile is then subjected to a cycloaddition reaction by treating XXXVIII with sodium azide in the presence of ammonium chloride, dimethylformamide and lithium chloride at temperatures from 100 °C to 130 °C to form IQ.

The starting bromophenylalkyl alcohol A may be prepared by subjecting aldehyde M

M 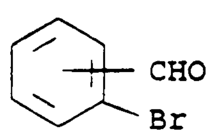

to a Wittig reaction with $(C_6H_5)_3PCHCO_2CH_3$ to form the ester N

N 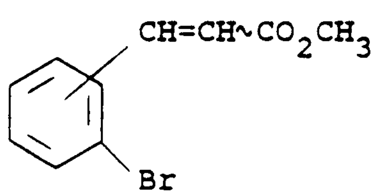

which is made to undergo a double bond reduction by treatment with hydrogen in the presence of rhodium on alumina catalyst in the presence of methanol to form ester O

O 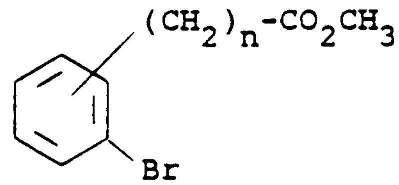

Ester O is then reduced by treatment with diisobutylaluminum hydride in the presence of toluene solvent to form alcohol A.

The compounds of this invention have four centers of asymmetry as indicated by the asterisks in formula I. However, it will be apparent that each of the formulae set out above which do not include asterisks still represent all of the possible stereoisomers thereof. All of the various stereoisomeric forms are within the scope of the invention.

The various stereoisomeric forms of the compounds of the invention, namely, cis-exo, cis-endo and all trans forms and stereoisomeric pairs may be prepared by employing starting materials and following the procedures as outlined in U.S. Patent No. 4,143,054. Examples of such stereoisomers are set out below.

Ia 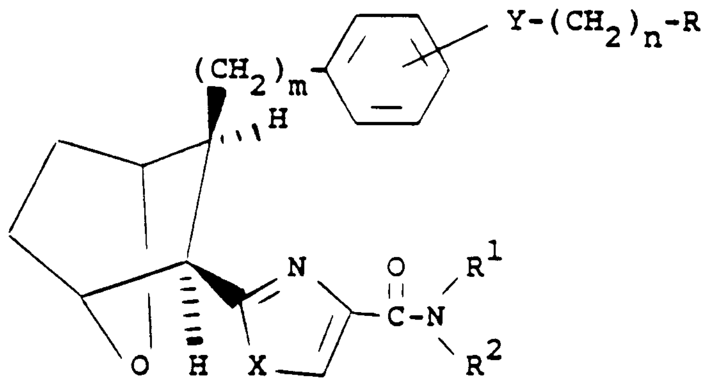

(cis-endo)

Ib 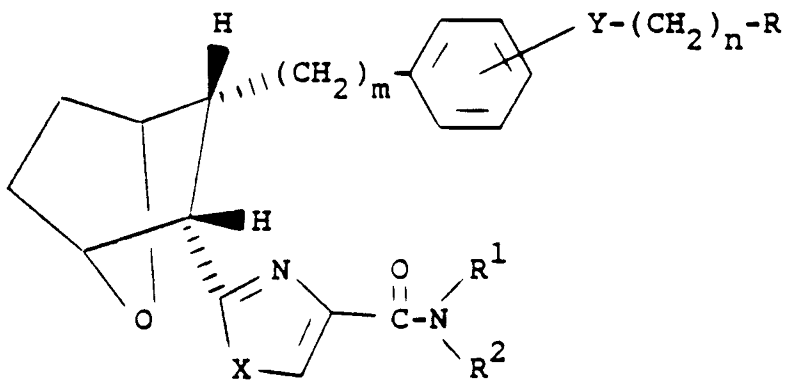

(cis-exo)

Ic

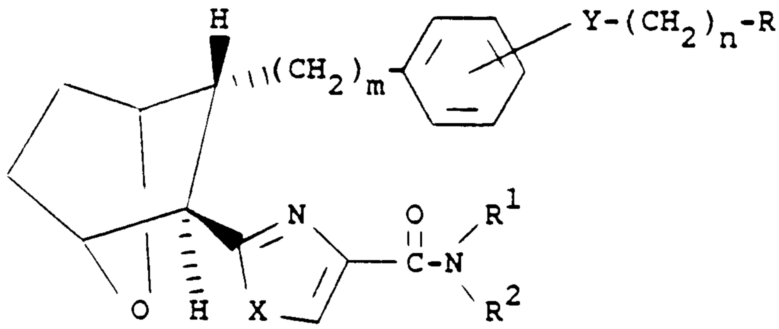

(trans)

Id

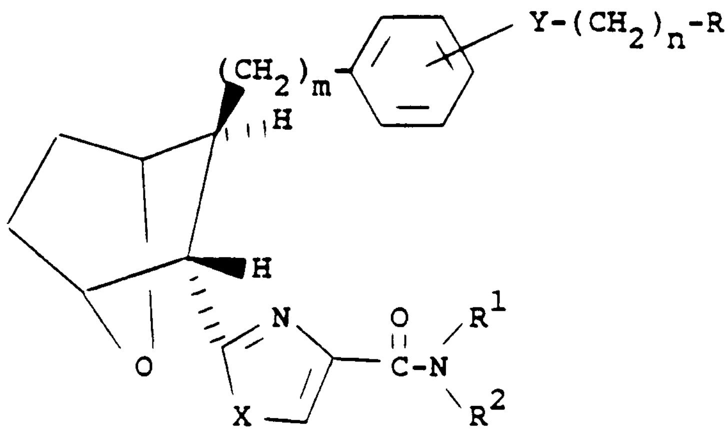

(trans)

The nucleus in each of the compounds of the invention is depicted as

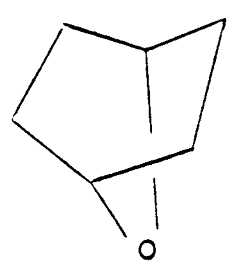

for matter of convenience; it will also be appreciated that the nucleus in the compounds of the invention may be depicted as

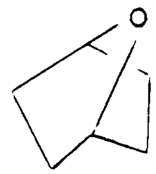

The compounds of this invention are thromboxane receptor antagonists and as such are useful as inhibitors of thromboxane receptor mediated actions. The term "thromboxane receptor antagonist" includes compounds which are so-called thromboxane $A_2$ receptor antagonists, thromboxane $A_2$ antagonists, thromboxane $A_2$/prostaglandin endoperoxide antagonists, TP-receptor antagonists, or thromboxane antagonists.

The compounds of this invention are useful as inhibitors of platelet function, i.e., for the prevention and treatment of thrombotic vascular occlusive disorders, whether complete or partial, for example, arterial thrombosis, including that of the coronary, cerebral, ophthalmic, hepatic, mesenteric, renal, peripheral arteries or vascular or organ grafts, unstable angina, transient ischemic attacks, or intermittent claudication. They may be useful to prevent thrombosis following vascular injury produced in the course of diagnostic or therapeutic procedures such as endarterectomy or angiography. The compounds may be useful in the treatment or prevention of disorders characterized by platelet consumption and/or activation, including, platelet activation, dysfunction, and/or loss during extracorporeal circulation, the use of radiographic contrast agents, thrombotic thrombocytopenia purpura, disseminated intravascular coagulation, purpura fulminans, hemolytic transfusion reaction, or hemolytic uremic syndrome. The compounds may be used in the treatment of venous thrombosis or embolism, including pulmonary embolism, deep venous thrombosis, hepatic vein thrombosis, and renal vein thrombosis.

The compounds of this invention are useful as inhibitors of arterial or venous vasoconstriction. Accordingly, they may be useful to prevent vasoconstriction associated with unstable angina, chronic stable angina, and variant, or Prinzmetal's angina, Raynaud's syndrome, migraine headache, vasospasm of the coronary, cerebral, ophthalmic, hepatic, mesenteric, renal, peripheral arteries or vascular grafts, vascular injury such as that associated with surgery or trauma. Hypertension of pregnancy, the hepato-renal syndrome, and pulmonary hypertension are additional examples of vasoconstrictive disorders treatable by the compounds of this invention.

The compounds of this invention are useful as inhibitors of bronchoconstriction, i.e., airway hyperresponsiveness, allergic bronchospasm, asthma, and bronchoconstrictive responses to environmental, infectious, noxious or mechanical stimuli.

The compounds of this invention are useful as inhibitors of ischemic and reperfusion injury to various tissues, including, myocardium, skin, brain, bowel, or kidney, alone or in combination with other agents intended to restore blood flow. For example, these compounds may be useful for improving postischemic myocardial function and decreasing myocardial infarct size. Ischemia caused by reduced blood flow during diagnostic or therapeutic procedures may benefit by treatment with these compounds, for example, they reduce the myocardial stunning observed after bypass surgery. In addition, they may be useful for reducing the tissue injury caused by a stroke.

The compounds of this invention may be useful in the prevention or treatment of other conditions including burns, diabetic retinopathy, and tardive dyskinesia. The compounds may be useful in potentiating diuretic-induced diuresis.

In addition, the thromboxane receptor antagonists of the invention may be used with a thrombolytic agent such as t-PA, streptokinase, urokinase, prourokinase or anisoylated plasminogen-streptokinase activator complex (APSAC) within 6 hours of a myocardial infarction. In such case, the thrombolytic agent may be used in amounts conventionally employed, for example, as disclosed in the Physicians' Desk Reference for reducing post-ischemic myocardial injury.

The compounds of the invention can be administered orally or parenterally to various mammalian species known to be subject to such maladies, e.g., humans, cats, dogs and the like in an effective amount within the dosage range of 0.1 to 100 mg/kg, preferably 0.2 to 50 mg/kg and more preferably 0.5 to 25 mg/kg (or from 1 to 2500 mg, preferably from 5 to 2000 mg) on a regimen in single or 2 to 4 divided daily doses.

The active substance can be utilized in a composition such as tablet, capsule, solution or suspension containing 5 to 500 mg per unit of dosage of a compound or mixture of compounds of formula I or in topical

form for wound healing (0.01 to 5% by weight compound of formula I, 1 to 5 treatments per day). They may be compounded in conventional matter with a physiologically acceptable vehicle or carrier, excipient, binder, preservative, stabilizer, flavor, etc., or with a topical carrier such as Plastibase (mineral oil gelled with polyethylene) as called for by accepted pharmaceutical practice. Also as indicated in the discussion above, certain members additionally serve as intermediates for other members of the group.

The compounds of the invention may also be administered topically to treat peripheral vascular diseases and as such may be formulated as a cream or ointment.

The following Examples represent preferred embodiments of the present invention. Unless otherwise indicated, all temperatures are expressed in degrees Centigrade.

## Example 1

[1S-(1α,2α,3α,4α)]-2-[[3-[4-[[(4-Cyclohexylbutyl)amino]carbonyl]-2-oxazolyl]-7-oxabicyclo[2.2.1]hept-2-yl]-methyl]benzenepropanoic acid

A. 3-(2-Bromophenyl)-2-propenoic acid, methyl ester

To a stirred suspension of 46.8 g (140 mmol, Aldrich) of methyl(triphenylphosphoranylidene)-acetate in 250 mL of dry tetrahydrofuran (THF) (distilled from potassium/benzophenone) at room temperature was added dropwise 25.0 g (135 mmol, Aldrich) of 2-bromobenzaldehyde, over 30 minutes. The reaction was mildly exothermic and became homogeneous. The resulting solution was stirred for 18 hours then concentrated in vacuo to give an oily solid. This material was slurried with 250 mL hexane and then filtered to remove solid triphenylphosphine oxide. The filtrate was concentrated in vacuo and the resulting oil was passed through a pad of silica gel (Merck silica, 9.5 x 2.0 cm, 1:4 ethyl acetate/petroleum ether elution). The eluant was concentrated in vacuo to give an oil. The crude oil was purified by bulb-to-bulb distillation (125-135°, ~0.5 mm) to afford 32.0 g (133 mmol, 98%) of title acrylate as a pale yellow liquid.

B. 2-Bromobenzenepropanoic acid, methyl ester

A mixture of 14.0 g (58.1 mmol) of Part A acrylate and 750 mg of 5% rhodium on alumina catalyst (MCB) in 150 mL of methanol (Burdick and Jackson) was stirred under an atmosphere of hydrogen (balloon) for 3 hours (until the starting material was consumed by TLC). The reaction mixture was passed through a 4 μM polycarbonate membrane and the filtrate was concentrated in vacuo to give an orange oil. The oil was dissolved in 100 mL of diethyl ether then washed with 50 mL of saturated sodium bicarbonate solution, 50 mL of brine, dried (magnesium sulfate) and concentrated in vacuo to afford 13.7 g (56.4 mmol, 97%) of title compound as a pale yellow liquid.

C. 2-Bromobenzenepropanol

To a solution of 13.6 g (56.0 mmol) of Part B compound in 75 mL of toluene (Burdick and Jackson, sieve-dried) cooled to -78° was added 118 mL (1.0 M in toluene, 118 mmol, Aldrich) of diisobutylaluminum hydride solution. The reaction was stirred at -78° for 2 hours then warmed to 0° for 2 hours. The resulting solution was quenched by very slow addition of 10 mL of 6 N HCl then more rapid addition of 100 mL of 6 N HCl. The reaction was stirred for an additional 10 minutes, then added to 50 mL of diethyl ether and the organic layer was separated. The organic layer was washed with two-100 mL portions of 1 N HCl, 100 mL of brine, dried (magnesium sulfate) and concentrated in vacuo to afford 12.0 g (55.8 mmol, 100%) of title compound as a colorless oil.

D. 1-Bromo-2-[3-[[(1,1-dimethylethyl)diphenylsilyl]oxy]propyl]benzene

To a solution of 12.0 g (55.8 mmol) of Part C compound, 15.3 g (55.8 mmol, Aldrich) of t-butylchlorodiphenylsilane, 8.6 mL (62 mmol, distilled from calcium hydride) of triethylamine in 100 mL of dry methylene chloride (distilled from phosphorous pentoxide) was added 200 mg (1.6 mmol, Aldrich) of 4-dimethylaminopyridine. The reaction mixture was stirred at room temperature for 4 hours then 100 mL of hexane was added and the resulting slurry filtered to remove triethylamine hydrochloride. The filtrate was concentrated in vacuo and the resulting oil purified by flash chromatography (Merck silica, 12 x 9 cm, 1:19 diethyl ether/hexane) to afford 23.2 g (51.2 mmol, 92%) of title compound as a colorless oil.

E. [1S-1α,2α,3α,4α)]-[2-[3-[[(1,1-Dimethylethyl)diphenylsilyl]oxy]propyl]phenyl]-7-oxabicyclo[2.2.1]heptane-2,3-dimethanol

To a solution of 23.0 g (50.8 mmol) of Part D compound in 75 mL of dry diethyl ether (distilled from potassium/benzophenone) cooled to -100° was added dropwise 54 mL (1.7M in pentane, 92 mmol, Aldrich) of t-butyllithium solution over ~20 minutes. The reaction mixture was stirred at -100° for 15 minutes, then warmed to 0° for 30 minutes. The resulting solution was cooled to -78°, 40 mL of dry THF (distilled from ketyl) was introduced then a solution of 3.40 g (21.8 mmol), [3aR-(3aα,4β,7β,7aα)]-octahydro-4,7-epoxyisobenzofuran-1-ol in 20 mL of dry THF was added over 5 minutes. The reaction mixture was warmed to 0°, stirred for 1 hour, quenched with 10 mL of water then partitioned between 200 mL of water and 100 mL of ethyl acetate. The organic layer was separated and the aqueous layer extracted with an additional 100 mL of ethyl acetate. The organic extracts were combined, washed with 200 mL of brine, dried (magnesium sulfate) and concentrated in vacuo to give an oil. The crude oil was purified by flash chromatography (Merck silica, 11 x 12 cm, 1:4 ethyl acetate/petroleum ether then 4:1 ethyl acetate/petroleum ether) to afford 9.81 g (18.5 mmol, 85%) of title compound as an oil.

F. [1S-(1α,2α,3α,4α)]-2-[[2-[3-[[(1,1-Dimethylethyl)diphenylsilyl]oxy]propyl]phenyl]methyl]-7-oxabicyclo-[2.2.1]heptane-3-methanol

A mixture of 8.50 g (16.0 mmol) of Part E compound and 15 g of moist 20% palladium hydroxide on carbon (Aldrich) in 100 mL of reagent ethyl acetate was shaken on a Parr apparatus under hydrogen (50 psi) for 42 hours. The resulting mixture was filtered successively on a Buchner funnel then through a 4 µM polycarbonate membrane filter. The filtrate was concentrated in vacuo and the resulting oil was purified by flash chromatography (Merck silica, 10 x 15 cm, 1:2 ethyl acetate/petroleum ether) to afford 2.07 g (4.03 mmol, 25%) of title compound as a colorless oil.

G. [1S-(1α,2α,3α,4α)]-2-[[2-[3-[[(1,1-Dimethylethyl)diphenylsilyl]oxy]propyl]phenyl]methyl]-7-oxabicyclo-[2.2.1]heptane-3-carboxylic acid

To a solution of 2.07 g (4.03 mmol) of Part F compound in 20 mL of reagent acetone cooled to 0° was added dropwise 3.5 mL (2.6M in water, for preparation see Fieser and Fieser, "Reagents for Organic Synthesis," Vol. 1, p. 142) of Jones reagent. The reaction was stirred for 1.5 hours then quenched by addition of 2 mL of isopropanol and stirred for 15 minutes. The resulting green slurry was filtered through a pad of Celite and the filtrate concentrated in vacuo. The residue was partitioned between 30 mL of water and 30 mL of diethyl ether. The organic layer was separated, washed with 30 mL of brine, dried (magnesium sulfate) and concentrated in vacuo to give 2.10 g (3.98 mmol, 99%) of crude title compound as a colorless oil.

H. [1S-(1α,2α,3α,4α)]-2-[[[2-[[2-[3-[[(1,1-Dimethylethyl)diphenylsilyl]oxy]propyl]phenyl]methyl]-7-oxabicyclo-[2.2.1]hept-3-yl]carbonyl]amino]-3-hydroxypropanoic acid, methyl ester

To a solution of 2.00 g (3.79 mmol) of crude Part G compound in 30 mL of dry THF (distilled from ketyl) cooled to 0° was added successively 512 mg (3.79 mmol, Aldrich) of 1-hydroxybenzotriazole hydrate, 590 mg (3.79 mmol, Aldrich) of L-serine methyl ester hydrochloride, 1.1 mL (8.6 mmol, distilled from calcium hydride) of triethylamine then after 5 minutes, 781 mg (3.79 mmol, Aldrich) of dicyclohexylcarbodiimide. The reaction was stirred at 0° for 3 hours then warmed to room temperature for 16 hours followed by the addition of 15 mL of ethyl acetate. The resulting slurry was filtered and the filtrate concentrated in vacuo to give an oil. The crude material was purified by flash chromatography (Merck silica, 15 x 5 cm, ethyl acetate) to afford 1.98 g (3.15 mmol, 83%) of title compound as a white foam.

I. [1S-(1α,2α,3α,4α)]-2-[2-[[2-[3-[[(1,1-Dimethylethyl)diphenylsilyl]oxy]propyl]phenyl]methyl]-7-oxabicyclo-[2.2.1]hept-3-yl]-4,5-dihydro-4-oxazolecarboxylic acid, methyl ester

A mixture of 1.81 g (2.96 mmol) of Part H compound and 2.33 g (8.88 mmol, Aldrich) of triphenylphosphine in 15 mL of acetonitrile (Burdick and Jackson, sieve-dried) was stirred until homogeneous then added was 1.5 mL (8.6 mmol, Aldrich) of diisopropylethylamine followed by 0.86 mL (8.9 mmol) of carbon tetrachloride. The reaction was mildly exothermic and cooled with an ambient water bath. The resulting solution was stirred at room temperature for 16 hours then added was 50 mL of ethyl acetate followed by

50 mL of saturated aqueous sodium bicarbonate solution. The organic layer was separated, washed with 20 mL of brine, dried (sodium sulfate) and concentrated in vacuo to give a dark oil. The crude material was purified by flash chromatography (Merck silica, 20 x 5 cm, 1:2 ethyl acetate/petroleum ether then 2:1 ethyl acetate/petroleum ether) to afford 1.24 g (2.03 mmol, 69%) of title compound as a yellow oil.

J. Nickel peroxide oxidant

Nickel peroxide oxidant was prepared as described by K. Nakagawa et al, J. Org. Chem., 27 1597 (1962). To a solution of 130 g (0.49 mols, Aldrich) of nickel(II) sulfate hexahydrate in 240 mL of water was added dropwise over ~45 minutes a solution of 360 mL of aqueous sodium hypochlorite (min. 5%, Aldrich) containing 42 g of sodium hydroxide. The reaction was mildly exothermic. The temperature was maintained between 20-25° with a cool water bath. The oxidant formed as a fine black precipitate upon addition. The reaction mixture was stirred for an additional 2 hours then divided into two centrifuge bottles. The black solid was separated by centrifugation (2500 rpm/10 minutes) and the supernatant was decanted. The solid in each bottle was slurried with 400 mL of ice-cold water and centrifuged. This washing procedure was repeated four additional times (pH = 10) then the resulting black solid was collected on a Buchner funnel with the aid of a latex dam. The solid cake (144 g) was placed in a vacuum oven (~ 1 mm/25°) for two days. The resulting material (56.5 g) was crushed then dried on a vacuum line (~ 0.1 mm) for 24 hours to afford 48.3 g (0.53 mols, 108%) of active oxidant as a black powder.

K. [1S-(1α,2α,3α,4α)]-2-[2-[[2-[3-[[(1,1-Dimethylethyl)diphenyl]silyl]oxy]propyl]phenyl]methyl]-7-oxabicyclo-[2.2.1]hept-3-yl]-4-oxazolecarboxylic acid, methyl ester

To a solution of 1.20 g (1.96 mmol) of Part I oxazoline in 30 mL of methylene chloride (distilled from phosphorous pentoxide) at room temperature was added 2.4 g of Part J nickel peroxide oxidant. The reaction mixture was stirred for 45 minutes (~1/2 complete by TLC) then an additional 2.4 g of oxidant was added and stirring continued for 45 minutes. The reaction was diluted with 100 mL of ethyl acetate and 50 mL of 3M aqueous sodium bisulfite solution then stirred rapidly for 30 minutes. The resulting green emulsion was added to 100 mL of 1M aqueous sodium citrate solution, the organic layer was separated and the aqueous layer was extracted with an additional 100 mL of ethyl acetate. The organic extracts were combined, dried (magnesium sulfate) and concentrated in vacuo to give an oil. The crude material was purified by flash chromatography (Merck silica, 20 x 3 cm, 1:2 ethyl acetate/petroleum ether) to afford 582 mg (0.96 mmol, 49%) of title oxazole as a colorless oil.

L. Cyclohexylbutylamine, monohydrochloride

To a stirred solution of 4-phenylbutylamine (10.6 g, 71.1 mmol, Aldrich) in 100 ml of glacial acetic acid under argon was added 87% PtO (1.06 g, 10% weight based on 4-phenylbutylamine). The reaction mixture was hydrogenated at 54 psi at room temperature for 4 hours. The catalyst was removed by filtration through a 2" pad of Celite, and the filtrate was concentrated in vacuo. The residue was diluted with 200 ml of diethyl ether, 100 ml of $CH_3OH$ and 8 ml of concentrated HCl. This mixture was concentrated in vacuo and triturated in diethyl ether to give 13.1 g (97%) of desired amine•HCl.

M. [1S-(1α,2α,3α,4α)]-N-(4-Cyclohexylbutyl)-2-[2-[[2-[3-[[(1,1-dimethylethyl)diphenylsilyl]oxy]propyl]phenyl]-methyl]-7-oxabicyclo[2.2.1]hept-3-yl]-4-oxazolecarboxamide

A solution of 290 mg (0.48 mmol) of Part K oxazole and 40 mg (0.95 mmol, Aldrich) of lithium hydroxide monohydrate in 4 mL of 3:1 THF/water was stirred rapidly at room temperature for 1.5 hours. The reaction mixture was acidified with 2 mL of 1M aqueous HCl then partitioned between 20 mL of diethyl ether and 20 mL of water. The organic layer was separated, dried (magnesium sulfate) and concentrated in vacuo to give the crude acid as an oil.

To a solution of the crude acid in 5 mL of sieve-dried benzene (Burdick and Jackson) was added a small drop of dimethylformamide (DMF) then 60 µL (0.69 mmol, Aldrich) of oxalyl chloride. The reaction was stirred until gas evolution ceased (~30 minutes) then concentrated in vacuo to give the crude acid chloride as a yellow oil.

To a solution of the crude acid chloride in 5 mL of dry methylene chloride (distilled from phosphorous pentoxide) cooled to 0° was added 165 µL (1.2 mmol, distilled from calcium hydride) of triethylamine then 110 mg (0.57 mmol) of Part L cyclohexylbutylamine hydrochloride. The reaction mixture was warmed to

room temperature, stirred for 1 hour then partitioned between 20 mL of ethyl acetate and 20 mL of 1M aqueous HCl solution. The organic layer was separated, dried (magnesium sulfate) and concentrated in vacuo to give an oil. The crude oil was purified by flash chromatography (Merck silica, 15 x 3 cm, 2:3 ethyl acetate/petroleum ether) to afford 277 mg (0.38 mmol, 79%) of title compound as a white foam.

N. [1S-(1α,2α,3α,4α)]-2-[[3-[4-[[(4-Cyclohexylbutyl)amino]carbonyl]-2-oxazolyl]-7-oxabicyclo[2.2.1]hept-2-yl]-methyl]benzenepropanoic acid

To a solution of 270 mg (0.37 mmol) of Part M compound in 6 mL of 2:1 acetonitrile/methylene chloride was added 0.60 mL of 48% aqueous hydrofluoric acid at room temperature. The reaction mixture was stirred rapidly for 2 hours then diluted with 20 mL of diethyl ether and quenched by slow addition of 10 mL of saturated aqueous sodium bicarbonate solution. The resulting mixture was added to 20 mL of water then the organic layer was separated, dried (magnesium sulfate) and concentrated in vacuo to give the crude alcohol as an oil.

To a solution of the crude alcohol in 6 mL of 5:1 acetone/methylene chloride was added 0.35 mL (2.6M in $Cr^{+6}$ in water) of Jones reagent. The reaction mixture was stirred for 20 minutes, then quenched by addition of 0.5 mL of isopropanol. After 10 minutes, 20 mL of diethyl ether and 20 mL of 3M aqueous sodium bisulfite solution were added and the mixture stirred for an additional 15 minutes. The organic layer was separated, dried (magnesium sulfate) and concentrated in vacuo to give an oil. The crude material was purified by flash chromatography (Merck silica, 15 x 1 cm, ethyl acetate then 1:9 methanol/methylene chloride) to afford 75 mg (0.15 mmol, 41%) of title product as a white foam.

IR (KBr): 3413, 2920, 1721, 1644, 1601, 1522, 1447 $cm^{-1}$

Partial 270 MHz $^1H$ NMR ($CDCl_3$):

2.20 (dd, 1H)
2.34 (t, 1H)
2.55 (t, J = 8, 2H)
2.91 (t, J = 8, 2H)
3.40 (m, 3H)
4.40 (d, J = 4, 1H)
4.98 (d, J = 4, 1H)
7.14 (m, 5H)
8.14 (s, 1H)

67.5 MHz $^{13}C$ NMR ($CDCl_3$): 24.2, 26.4, 26.7, 27.4, 28.9, 29.9, 32.4, 33.3, 34.7, 37.1, 37.5, 39.2, 47.0, 50.0, 78.7, 79.7, 126.6, 126.7, 129.0, 129.7, 136.0, 137.8, 138.5, 141.0, 160.8, 163.9, 176.0.

MS(CI): 509 $(M+H)^+$

TLC: $R_f$ (silica gel, 1:19 methanol/methylene chloride) = 0.33, ammonium molybdate/ceric sulfate, UV

| Analysis Calc'd for $C_{30}H_{40}N_2O_5$ + 0.42 $H_2O$: | | | |
|---|---|---|---|
| | C, 69.80; | H, 7.97; | N, 5.43 |
| Found: | C, 69.83; | H, 7.90; | N, 5.40 |

Example 2

[1S-(1α,2α,3α,4α)]-2-[[3-[4-[[[4-(4-Chlorophenyl)butyl]amino]carbonyl]-2-oxazolyl]-7-oxabicyclo[2.2.1]hept-2-yl]methyl]benzenepropanoic acid

A. 1-Bromo-2-[3-[[Dimethyl(1,1,2-trimethylpropyl)silyl]oxy]propyl]benzene

To a solution of 29.0 g (135 mmol) of crude Example 1 Part C alcohol and 24.1 g (135 mmol, Petrarch) of thexyldimethylchlorosilane in 200 mL of dry methylene chloride (distilled from phosphorous pentoxide) was added at room temperature 20 mL (143 mmol, distilled from calcium hydride) of triethylamine then 200 mg (1.64 mmol, Aldrich) of 4-dimethylaminopyridine. The reaction mixture was stirred at room temperature for 18 hours. The resulting slurry was diluted with 100 mL of hexane, cooled to 0° with stirring for 15 minutes then filtered to remove solid triethylamine hydrochloride. The filtrate was concentrated in vacuo to give an oil. The crude oil was purified by flash chromatography (Merck silica, 15 x 10 cm, 1:9 ethyl acetate/petroleum ether) to afford 45.5 g (127 mmol, 94%) of title compound as a colorless liquid.

B. [1S-(1α,2α,3α,4α)]-[2-[3-[[Dimethyl(1,1,2-trimethylpropyl)silyl]oxy]propyl]phenyl]-7-oxabicyclo[2.2.1]-heptane-2,3-dimethanol

To a solution of 5.00 g (14.0 mmol) of Part A compound in 30 mL of dry diethyl ether (distilled from ketyl) cooled to -100° was added dropwise 15 mL (1.7M in pentane, 25 mmol, Aldrich) of t-butyllithium solution over 15 minutes. The reaction mixture was stirred at -100° for 15 minutes then at 0° for 15 minutes. The resulting pale yellow anion solution was recooled to -78° then 30 mL of dry THF (distilled from ketyl) was introduced followed by the rapid addition of a solution of 875 mg (5.61 mmol) of [3aR-(3aα,4β,7β,7aα)]-octahydro-4,7-epoxyisobenzofuran-1-ol in 10 mL of THF. The reaction mixture was warmed to 0°, stirred for 1 hour, quenched with 5 mL of water then partitioned between 100 mL of water and 25 mL of ethyl acetate. The organic layer was separated and the aqueous layer was extracted with an additional 25 mL of ethyl acetate. The organic extracts were combined, dried (magnesium sulfate) and concentrated in vacuo to give an oil. The crude oil was purified by flash chromatography (Merck silica, 12 x 5.0 cm, 1:4 ethyl acetate/petroleum ether then 4:1 ethyl acetate/petroleum ether) to afford 2.35 g (5.41 mmol, 97%) of title diasteromeric alcohols as a colorless oil.

C. [1S-(1α,2α,3α,4α)]-2-[[2-[3-[[Dimethyl(1,1,2-trimethylpropyl)silyl]oxy]propyl]phenyl]methyl]-7-oxabicyclo-[2.2.1]heptane-3-methanol

A mixture of 1.90 g (4.38 mmol) of Part B diastereomeric alcohols and 1.9 g of 20% palladium hydroxide on carbon catalyst (moist, <50% water, Aldrich) in 60 mL of glacial acetic acid was stirred rapidly under an atmosphere of hydrogen (balloon) for 5 hours. The reaction mixture was filtered through a 4μM polycarbonate membrane and the filtrate was concentrated in vacuo (room temperature bath). The residue was partitioned between 50 mL of water and 50 mL of ethyl acetate. The organic layer was separated, washed with 50 mL of 1M aqueous sodium hydroxide solution, dried (magnesium sulfate) and concentrated in vacuo to give an oil. The crude material was purified by flash chromatography (Merck silica, 12 x 5.0 cm, 1:2 ethyl acetate/petroleum ether) to afford 1.03 g (2.39 mmol, 55%) of title compound as a colorless oil. In addition, 573 mg (1.37 mmol, 30%) of Part C starting material (as a single diastereomer) was recovered.

D. [1S-(1α,2α,3α,4α)]-2-[[3-(Hydroxymethyl)-7-oxabicyclo[2.2.1]hept-2-yl]methyl]benzenepropanoic acid, methyl ester

A solution of 1.00 g (2.39 mmol) of Part D compound and 50 mg (0.41 mmol, Aldrich) of 4-dimethylaminopyridine in 6 mL of 1:1 dry pyridine/acetic anhydride was stirred at room temperature for 2 hours. The reaction mixture was concentrated in vacuo and the residue partitioned between 25 mL of ethyl acetate and 20 mL 1M aqueous HCl solution. The organic layer was separated, washed with 20 mL of 1M aqueous NaOH then 20 mL of brine, dried (magnesium sulfate) and concentrated in vacuo to afford the crude acetate as an oil.

To a solution of the crude acetate in 15 mL of reagent acetone cooled to 0° was added rapidly 3.3 mL (2.6M in $Cr^{+6}$, for preparation see Fieser & Fieser, "Reagents for Organic Synthesis," Vol. 1, p. 142) of Jones reagent. The reaction mixture was stirred for 2 hours, quenched by addition of 1 mL of isopropanol and stirred for an additional 30 minutes. The resulting green slurry was filtered through a pad of Celite. The filtrate was concentrated in vacuo and the residue partitioned between 25 mL of diethyl ether and 25 mL of water. The organic layer was separated and concentrated in vacuo to give the crude acetate-acid as an oil.

A solution of the crude acetate-acid in 15 mL of 2:1 1M aqueous NaOH/THF was stirred at room temperature for 30 minutes. The reaction mixture was cooled in an ice-bath, quenched by addition of 15 mL of 1M aqueous HCl solution then extracted with two-25 mL portions of diethyl ether. The ether extracts were combined, washed with 25 mL of brine and concentrated in vacuo to give the crude alcohol-acid as an oil.

A solution of the crude alcohol-acid in 10 mL of acidic methanol (prepared by addition of 0.5 mL of acetyl chloride to 10 mL of dry methanol at 0°) was stirred at 0° for 2 hours then concentrated in vacuo. The resulting oil was purified by flash chromatography (Merck silica, 15 x 3.0 cm, ethyl acetate) to afford 526 mg (1.76 mmol, 74% from Part C compound) of title compound as a colorless oil.

E. [1S-(1α,2α,3α,4α)]-2-[[3-Carboxy-7-oxabicyclo[2.2.1]hept-2-yl]methyl]benzenepropanoic acid, methyl ester

To a solution of 495 mg (1.63 mmol) of Part D compound in 5 mL of reagent acetone cooled to 0° was added rapidly 2.0 mL (2.6M in $Cr^{+6}$) of Jones reagent. The reaction mixture was warmed to room

temperature, stirred for 2 hours then quenched by addition of ~1 mL of isopropanol. After 15 minutes the resulting green slurry was filtered through a pad of Celite. The filtrate was partitioned between 20 mL of diethyl ether and 20 mL of water. The organic layer was separated and the aqueous layer was extracted with an additional 20 mL of diethyl ether. The ether extracts were combined, dried (magnesium sulfate) and concentrated in vacuo to give 560 mg (1.59 mmol, 98%) of crude title compound as a colorless oil.

F. [1S-(1α,2α,3α,4α)]-2-[[3-[[1-(Hydroxymethyl)-2-oxo-2-(phenylmethoxy)ethyl]amino]carbonyl]-7-oxabicyclo-[2.2.1]hept-2-yl]methyl]benzenepropanoic acid, methyl ester

To a solution of 490 mg (1.54 mmol) of Part E acid in 10 mL of dry THF (distilled from ketyl) cooled to 0° was added 392 mg (1.69 mmol, Sigma) of L-serine benzyl ester hydrochloride, 228 mg (1.69 mmol, Aldrich) of 1-hydroxybenzotriazole hydrate and 530 μL (3.8 mmol. distilled from calcium hydride) of triethylamine. The mixture was stirred for 5 minutes then 348 mg (1.69 mmol, Aldrich) of dicyclohexylcarbodiimide was added in one portion. The reaction was stirred at 0° for 3 hours then warmed to room temperature for 16 hours. The resulting slurry was diluted with 10 mL of ethyl acetate, cooled to 0° for 15 minutes then filtered. The filtrate was concentrated in vacuo to give an oil. The crude material was purified by flash chromatography (Merck silica, 15 x 3.0 cm, ethyl acetate) to afford 540 mg (1.09 mmol, 71%) of title compound as a white solid.

G. [1S-(1α,2α,3α,4α)]-2-[[3-[4,5-Dihydro-4-[(phenylmethoxy)carbonyl]-2-oxazolyl]-7-oxabicyclo[2.2.1]hept-2-yl]methyl]benzenepropanoic acid, methyl ester

To a solution of 525 mg (1.06 mmol) of Part F compound, 843 mg (3.10 mmol, Aldrich) of triphenylphosphine and 540 μL (3.1 mmol, Aldrich) of diisopropylethylamine in 6 mL of 5:1 dry acetonitrile/methylene chloride was added at room temperature 300 μL (3.1 mmol, Mallinckrodt) of reagent carbon tetrachloride. The reaction mixture was stirred for 2 hours then diluted with 15 mL of ethyl acetate followed by the slow addition of 15 mL of saturated aqueous sodium bicarbonate solution. The resulting mixture was stirred for 5 minutes then partitioned between 20 mL of ethyl acetate and 20 mL of water. The organic layer was separated, washed with 20 mL of brine, dried (sodium sulfate) and concentrated in vacuo to give a yellow oily solid. The crude material was purified by flash chromatography (Merck silica, 20 x 3.0 cm, 2:1 ethyl acetate/petroleum ether) to afford 380 mg (0.80 mmol, 75%) of title oxazoline as a pale yellow solid.

H. [1S-(1α,2α,3α,4α)]-2-[[3-[4-[(Phenylmethoxy)carbonyl]-2-oxazolyl]-7-oxabicyclo[2.2.1]hept-2-yl]methyl]-benzenepropanoic acid, methyl ester

To a solution of 375 mg (0.79 mmol) of Part G oxazoline in 10 mL of dry methylene chloride (distilled from phosphorous pentoxide) was added 750 mg of Example 1, Part J, nickel peroxide oxidant at room temperature. The reaction mixture was stirred for 1 hour then an additional 190 mg of oxidant was added. After 30 minutes the reaction mixture was diluted with 20 mL of ethyl acetate followed by the addition of 10 mL of 3M aqueous sodium bisulfite solution. The resulting mixture was stirred rapidly for 20 minutes then 10 mL of water was added. The organic layer was separated and the aqueous layer extracted with an additional 20 mL of ethyl acetate. The organic extracts were combined, washed with 25 mL of 1M aqueous sodium citrate solution, dried (magnesium sulfate) and concentrated in vacuo to give an oil. The crude material was purified by flash chromatography (Merck silica, 15 x 5.0 cm, 2:3 ethyl acetate/petroleum ether) to afford 180 mg (0.38 mmol, 48%) of title oxazole as an oil.

I. 4-(4-Chlorophenyl)butylamine

(a) 3-(4-Chloropenyl)propanol

To a stirred solution of 5.0 g (27 mmol) of 3-(4-chlorophenyl)-propionic acid in 30 ml of tetrahydrofuran at 0°C, 30 ml (30 mmol) of boranetetrahydrofuran complex was added dropwise. The reaction was stirred for 15 hours. The reaction mixture was concentrated in vacuo. The residue was quenched with water and partitioned between diethyl ether and saturated sodium bicarbonate. The organic layer was separated and the aqueous layer was extracted twice with 40 ml of diethyl ether. Organic layers were combined and washed with brine, dried over $MgSO_4$ and concentrated in vacuo to obtain 3.9 g of a colorless oil.
$^{13}C$ NMR ($CDCl_3$, 67.8 MHz)δ: 140.0, 131.0, 129.9, 129.0, 161.0, 132.5, 131.0.

(b) 3-(4-Chlorophenyl)propyl bromide

To a stirred solution of 4.15 g (15.8 mmol) of triphenylphosphine in 100 ml of toluene at 0 °C, 1.51 ml (15.8 mmol) of bromine was added dropwise. This mixture was stirred for 3 hours. Then a solution of 3.90 g (22.86 mmol) of Part (a) alcohol and 1.63 ml (15.8 mmol) of pyridine in 20 ml of toluene was added. A solution of 25 ml hexane and 25 ml diethyl ether was added, and a brown mass was formed. The liquid was decanted and concentrated in vacuo. The residue was triturated with hexane:ethyl acetate and triphenylphosphine oxide was precipitated. The solid was filtered and the filtrate was concentrated to give a yellow oil. The oil was purified by flash chromatography to obtain 1.80 g (7.72 mmol, 49%) of the desired product.
$^{13}C$ NMR ($CDCl_3$)δ: 138.7, 131.6, 129.6, 128.3, 33.7, 33.0, 32.5.

(c) 4-(4-Chlorophenyl)butyronitrile

To a solution of 3.10 g (13.3 mmol) of Part (b) bromide in 36 ml of ethanol stirred under argon at room temperature, was added a solution of 4.26 g (65.4 mmol) of potassium cyanide in 12 ml of water. The reaction was incomplete after 5 hours as indicated by TLC. To the reaction mixture 4 ml of THF and 4 ml of water were added, and a homogeneous reaction mixture was obtained. After stirring for 12 hours, water and diethyl ether were added. The organic layer was separated. The aqueous layer was extracted twice with 50 ml of diethyl ether. The organic layers were combined, washed with water, brine, dried over $MgSO_4$ and concentrated in vacuo to give an oil. The oil was purified by flash chromatography (90:10 hexane:ethyl acetate) to obtain 1.80 g (10.1 mmol, 76%)of title nitrile as a clear oil.
$^{13}C$ NMR ($CDCl_3$)δ: 138.0, 132.1, 129.6, 128.6, 33.5, 26.6, 16.2

(d) 4-(4-Chlorophenyl)butylamine

To a solution of 1.80 g (10 mmol) of Part (c) nitrile in 70 ml of diethyl ether stirred under argon at 0 °C, was added 0.38 g (10 mmol) of lithium aluminum hydride. Gas was evolved. After 20 minutes, the reaction mixture was quenched with 0.4 ml of water, then 0.4 ml of 1N NaOH, then 1.2 ml water, stirring for a few minutes after each addition. The white precipitate was filtered and the filtrate was concentrated to obtain 1.5 g (8.20 mmol, 82%) of title amine as a clear oil.
$^{13}C$ NMR ($CDCl_3$)δ: 140.7, 131.2, 129.5, 128.2, 41.8, 34.9, 33.0, 28.4

J. [1S-(1α,2α,3α,4α)]-2-[[3-[4-[[[4-(4-Chlorophenyl)butyl]amino]carbonyl]-2-oxazolyl]-7-oxabicyclo[2.2.1]hept-2-yl]methyl]benzenepropanoic acid, methyl ester

A mixture of 175 mg (0.37 mmol) of Part H oxazole and 30 mg of 20% palladium hydroxide on carbon catalyst (moist, <50% water, Aldrich) in 5 mL of reagent ethyl acetate was stirred under an atmosphere of hydrogen (balloon) for 1 hour. The catalyst was removed by filtration through a 4µM polycarbonate membrane. The filtrate was concentrated in vacuo to afford 141 mg (0.37 mmol, 100%) of the crude acid as a white solid, mp 156-158 °.

To a solution of 135 mg (0.35 mmol) of the crude acid in 3 mL of dry methylene chloride (distilled from phosphorous pentoxide) was added at room temperature a small drop of DMF then 40 µL (0.46 mmol, Aldrich) of oxalyl chloride. The reaction mixture was stirred for 30 minutes then concentrated in vacuo to give the crude acid chloride as a yellow solid. The acid chloride was solubilized in 3 mL of dry methylene chloride then cooled to 0 ° and a solution of 84 mg (0.46 mmol) of Part I amine and 70 µL (0.50 mmol, distilled from calcium hydride) of triethylamine in 1 mL of dry methylene chloride was added rapidly. The reaction mixture was stirred for 30 minutes then partitioned between 25 mL of ethyl acetate and 15 mL of 1M aqueous HCl solution. The organic layer was separated and the aqueous layer was extracted with an additional 10 mL of ethyl acetate. The organic extracts were combined, dried (magnesium sulfate) and concentrated in vacuo to give a yellow solid. The crude material was purified by flash chromatography (Merck silica, 18 x 1.5 cm, 3:1 ethyl acetate/petroleum ether) to afford 161 mg (0.29 mmol. 83%) of title compound as a white solid, mp 140-142 °.

K. [1S-(1α,2α,3α,4α)]-2-[[3-[4-[[[4-(4-Chlorophenyl)butyl]amino]carbonyl]-2-oxazolyl]-7-oxabicyclo[2.2.1]hept-2-yl]methyl]benzenepropanoic acid

A solution of 158 mg (0.29 mmol) of Part J compound and 25 mg (0.60 mmol, Aldrich) of lithium hydroxide monohydrate in 6 mL of 2:1 THF/water was stirred rapidly at room temperature for 1.5 hours. The

reaction was acidified by addition of 2 mL of 1M aqueous HCl solution then partitioned between 20 mL of ethyl acetate and 20 mL of water. The organic layer was separated, washed with 20 mL of brine, dried (magnesium sulfate) and concentrated in vacuo to afford 152 mg (0.28 mmol, 98%) of title product as a solid white foam.

IR (KBr): 3413, 2940, 1724, 1652, 1602, 1522, 1491, 1203, 1175, 1105 $cm^{-1}$

Partial 270 MHz $^1$NMR ($CDCl_3$):

δ2.19 (dd, 1H)
2.32 (t, J=11, 1H)
2.55 (m, 5H)
2.89 (t, J=8, 2H)
3.39 (m, 3H)
4.39 (d, J=4, 1H)
4.96 (d, J=4, 1H)
7.12 (m, 9H)
8.14 (s, 1H)

67.5 MHz $^{13}$C NMR ($CDCl_3$): δ 27.4, 28.5, 28.8, 29.0, 29.9, 32.4, 34.7, 38.8, 46.9, 50.0, 78.7, 79.7, 126.5, 126.7, 128.4, 129.0, 129.7, 131.4, 135.8, 137.7, 138.4, 140.5, 141.1, 160.9, 163.9, 176.5.

MS(CI): 537, 539 $(M+H)^+$

OR: $[\alpha]_D = +9.9°$ (c=1.0 in methanol) closed aperture

TLC: $R_f$ (silica gel, 1:9 methanol/methylene chloride)=0.50, ammonium molybdate/ceric sulfate and UV, homogeneous

| Analysis Calc'd for $C_{30}H_{33}ClN_2O_5$: | | | | |
|---|---|---|---|---|
| | C, 67.09; | H, 6.19; | N, 5.22, | Cl, 6.60 |
| Found: | C, 67.33; | H, 6.35; | N, 5.13; | Cl, 6.45 |

Example 3

[1S-(1α,2α,3α,4α)]-3-[[3-[4-[[(4-Cyclohexylbutyl)amino]carbonyl]-2-oxazolyl]-7-oxabicyclo[2.2.1]hept-2-yl]-methyl]benzenepropanoic acid

A. (3-Bromophenethyl)oxythexyldimethylsilane

To a stirred solution of 3-bromophenylacetic acid (55.8 g, 260 mmol, Aldrich) under argon at 0°C was added 1M diborane/tetrahydrofuran (THF) solution dropwise (300 mL, 300 mmol) over one hour. This mixture was stirred at 0°C for 5.5 hours and then quenched slowly with water. The resulting mixture was concentrated in vacuo and the residue partitioned between 300 mL of saturated $NaHCO_3$ solution and diethyl ether (4 x 40 mL). The combined ether extracts were dried ($MgSO_4$), filtered and concentrated in vacuo to give 51.7 g of crude alcohol. To a stirred solution of this alcohol and triethylamine (75 mL, 538 mmol) in 500 mL of dry $CH_2Cl_2$ under argon at 0°C was added thexyldimethylsilylchloride (56.2 mL, 286 mmol) over 15 minutes. The reaction mixture was stirred at 0°C for 75 minutes and then at room temperature for 15 hours. This mixture was diluted with 500 mL of diethyl ether and the precipitate was filtered off. The solid was rinsed with diethyl ether (3 x 300 mL). The filtrate was concentrated in vacuo and partitioned between 300 mL of saturated $NH_4Cl$ solution and diethyl ether (4 x 300 mL). The combined ether extracts were dried ($MgSO_4$) and concentrated in vacuo. This crude product was distilled to give 76.9 g (87%) of title compound, bp 148-154° (~0.5 mm).

B. [1S-(1α,2α,3α,4α)]-2-[3-[[3-(Hydroxymethyl)-7-oxabicyclo[2.2.1]hept-2-yl]hydroxymethyl]benzene]ethoxy]-dimethyl(1,1,2-trimethylpropyl)silane

To a solution of 10.0 g (29.1 mmol) of Part A compound in 60 mL of dry diethyl ether cooled to -78° was added dropwise 30 mL (1.7m in pentane, 51 mmol, Aldrich) of t-butyllithium solution over ~15 minutes. The reaction mixture was stirred at -78° for 15 minutes then at 0° for 30 minutes. The resulting anion solution was re-cooled to -78°, 40 mL of dry tetrahydrofuran was introduced and then a solution of 1.87 g (12.0 mmol) of [3aR-(3aα,4β,7β,7aα)]octahydro-4,7-epoxyisobenzofuran-1-ol in 20 mL of tetrahydrofuran was added dropwise. After 15 minutes the reaction was warmed to 0°. After an additional 1 hour at 0°, the

reaction was quenched with 5 mL of water, then added to 200 mL of water and extracted with two-75 mL portions of ethyl acetate. The organic extracts were combined, dried (magnesium sulfate) and concentrated in vacuo to give an oil. The crude oil was purified by flash chromatography (Merck silica, 23 x 5.0 cm, 1:4 ethyl acetate/petroleum ether then ethyl acetate) to afford 4.10 g (10.1 mmol, 85%) of title compound as a colorless oil.

C. [1S-(1α,2α,3α,4α)]-2-[3-[[3-(Hydroxymethyl)-7-oxabicyclo[2.2.1]hept-2-yl]methyl]benzene]ethoxy]dimethyl-(1,1,2-trimethylpropyl)silane

A mixture of 4.05 g (10.0 mmol) of Part B compound and 5.50 g of 10% palladium on activated carbon (Aldrich) in 80 mL of glacial acetic acid was shaken under an atmosphere of hydrogen (40 psi) on a Parr apparatus for 24 hours. The resulting mixture was passed through a polycarbonate filter to remove the catalyst and the filtrate was concentrated in vacuo to give an oil. The crude oil was partitioned between 100 mL of ethyl acetate and 100 mL of water. The organic layer was separated, dried (magnesium sulfate) and concentrated in vacuo to afford 3.72 g (9.60 mmol, 96%) of crude title alcohol as a colorless oil.

D. [1S-(1α,2α,3α,4α)]-3-[[3-[(Acetyloxy)methyl]-7-oxabicyclo[2.2.1]hept-2-yl]methyl]benzeneethanol

To a solution of 4.56 g (11.3 mmol) of Part C alcohol and 1.3 mL (16 mmol, Burdick and Jackson) of pyridine in 50 mL of dry methylene chloride (distilled from phosphorous pentoxide) cooled to 0° was added dropwise a solution of 1.0 mL (14 mmol, Mallinckrodt) of acetyl chloride in 3 mL of methylene chloride. The reaction mixture was stirred for 30 minutes then added to 50 mL of 1M aqueous HCl solution. The organic phase was separated, washed with 50 mL of 1M aqueous NaOH solution, dried (magnesium sulfate) and concentrated in vacuo to afford 5.05 g (11.4 mmol, quant) of crude acetate as a colorless oil. To a solution of the crude acetate in 30 mL of acetonitrile (Brudick and Jackson) cooled to 0° was added 1.5 mL of 48% aqueous hydrofluoric acid solution. The reaction was stirred for 1 hour then quenched by slow addition of 20 mL of saturated aqueous sodium bicarbonate solution. The resulting mixture was added to 100 mL of water and extracted with two-50 mL portions of ethyl acetate. The organic extracts were combined, dried (magnesium sulfate) and concentrated in vacuo to give an oil. The crude material was purified by flash chromatography (Merck silica, 20 x 5 cm, ethyl acetate) to afford 3.01 g (9.90 mmol, 88%) of title alcohol as a colorless oil.

E. [1S-(1α,2α,3α,4α)]-2-[[3-[2-[[(1,1-Dimethylethyl)diphenylsilyl]oxy]ethyl]phenyl]methyl]-7-oxabicyclo[2.2.1]-heptane-3-methanol

To a solution of 3.00 g (9.87 mmol) of Part D alcohol and 2.75 g (10.0 mmol, Petrarch) of t-butylchlorodiphenylsilane in 50 mL of dry methylene chloride (distilled from phosphorous pentoxide) was added at 0° 1.7 mL (12 mmol, distilled from calcium hydride) of triethylamine then 200 mg (1.6 mmol, Aldrich) of 4-dimethylaminopyridine. The reaction mixture was warmed to room temperature, stirred for 2 hours and the resulting slurry washed with 50 mL of 1M aqueous hydrochloric acid then 50 mL of water, dried (magnesium sulfate) and concentrated in vacuo to give the crude silyl ether as an oil. To a solution of the crude silyl ether in 50 mL of anhydrous diethyl ether (Mallinckrodt) cooled to -78° was added 20 mL (1.4M in diethyl ether, 28 mmol, Aldrich) of methyllithium solution dropwise over 15 minutes. After 5 minutes the reaction was quenched with 1 mL of methanol then warmed to room temperature and partitioned between 50 mL of diethyl ether and 100 mL of water. The organic layer was separated, washed with 50 mL of brine, dried (magnesium sulfate) and concentrated in vacuo to give an oil. The crude material was purified by flash chromatography (Merck silica, 20 x 5 cm, 1:1 ethyl acetate/petroleum ether) to afford 4.41 g (8.82 mmol, 89%) of title alcohol as a colorless glass.

F. [1S-(1α,2α,3α,4α)]-2-[[3-[2-[[(1,1-Dimethylethyl)diphenylsilyl]oxy]ethyl]phenyl]methyl]-7-oxabicyclo[2.2.1]-heptane-3-carboxylic acid

To a solution of 1.75 g (3.50 mmol) of Part E alcohol in 25 mL of reagent acetone cooled to 0° was added 3.5 mL of Jones reagent (2.6M, Fieser and Fieser, vol. 1, p. 142) dropwise over 5 minutes. The reaction was stirred at 0° for 1.5 hours then quenched by addition of ~1 mL of isopropyl alcohol. The mixture was stirred for 30 minutes then the resulting green slurry was filtered through a pad of Celite. The filtrate was partitioned between 100 mL of water and 75 mL of ethyl acetate. The organic layer was separated, washed with 100 mL of water, 50 mL of brine, dried (magnesium sulfate) and concentrated in

vacuo to give 1.82 g (3.54 mmol, 101%) of crude acid as a solid white foam.

G. [1S-(1α,2α,3α,4α)]-2-[[[2-[[3-[2-[[(1,1-Dimethylethyl)diphenylsilyl]oxy]ethyl]phenyl]methyl]-7-oxabicyclo-[2.2.1]hept-3-yl]carbonyl]amino]-3-hydroxypropanoic acid, methyl ester

To a solution of 1.86 g (3.72 mmol) of Part F acid, 577 mg (3.72 mmol, Aldrich) of L-serine methyl ester hydrochloride and 502 mg (3.72 mmol, Aldrich) of 1-hydroxybenzotriazole hydrate in 20 mL of dry THF (distilled from potassium/benzophenone) cooled to 0° was added 1.1 mL (7.8 mmol, distilled from calcium hydride) of triethylamine, then after 5 minutes, 766 mg (3.72 mmol, Aldrich) of 1,3-dicyclohexylcarbodiimide. The reaction mixture was allowed to warm to room temperature, stirred for 16 hours and the resulting slurry filtered. The filtrate was concentrated in vacuo to give an oil. The crude material was purified by flash chromatography (Merck silica, 15 x 5 cm, ethyl acetate) to afford 1.75 g (2.84 mmol, 76%) of title amide as a white foam.

H. [1S-(1α,2α,3α,4α)]-2-[2-[[3-[2-[[(1,1-Dimethylethyl)diphenylsilyl]oxy]ethyl]phenyl]methyl]-7-oxabicyclo-[2.2.1]hept-3-yl]-4,5-dihydro-4-oxazolecarboxylic acid, methyl ester

To a solution of 1.65 g (2.76 mmol) of Part G amide and 2.89 g (11.0 mmol, Aldrich) of triphenylphosphine in 40 mL of sieve-dried acetonitrile (Burdick and Jackson) was added 0.70 mL (7.2 mmol) of carbon tetrachloride then 1.1 mL (7.8 mmol, distilled from calcium hydride) of triethylamine. The solution was heated to 65° for 30 minutes then cooled to room temperature and the resulting dark solution was partitioned between 120 mL of saturated aqueous sodium bicarbonate solution and 50 mL of ethyl acetate. The organic layer was separated and the aqueous layer was extracted with an additional 50 mL of ethyl acetate. The organic extracts were combined, dried (sodium sulfate) and concentrated in vacuo to give a dark oil. The crude material was purified by flash chromatography (Merck silica, 20 x 5 cm, 1:2 acetone/hexane) to afford 515 mg (0.86 mmol, 31%) of title oxazoline as a yellow oil.

I. [1S-(1α,2α,3α,4α)]-2-[2-[[3-[2-[[(1,1-Dimethylethyl)diphenylsilyl]oxy]ethyl]phenyl]methyl]-7-oxabicyclo-[2.2.1]hept-3-yl]-4-oxazolecarboxylic acid, methyl ester

To a solution of 505 mg (0.85 mmol) of Part H oxazoline in 20 mL of dry methylene chloride (distilled from phosphorous pentoxide) was added at room temperature in one portion 1.0 g of Example 1, Part J, nickel peroxide. The reaction mixture was stirred for 1 hour then an additional 1.0 g portion of nickel peroxide was added. The reaction was again stirred for 1 hour then added was 40 mL of ethyl acetate followed by 25 mL of 3M aqueous sodium bisulfite solution. The mixture was stirred rapidly for 20 minutes then to the resulting blue-green emulsion was added 50 mL of 1M aqueous sodium citrate solution. The mixture was stirred for an additional 20 minutes then the organic layer was separated and the aqueous layer was extracted with 25 mL of ethyl acetate. The organic extracts were combined, dried (magnesium sulfate) and concentrated in vacuo to give an oil. The crude oil was purified by flash chromatography (Merck silica, 15 x 3 cm, 1:1 ethyl acetate/petroleum ether) to yield 247 mg (0.42 mmol, 49%) of title oxazole as a colorless oil.

J. [1S-(1α,2α,3α,4α)]-N-(4-Cyclohexylbutyl)-2-[2-[[3-[2-[[(1,1-dimethylethyl)diphenylsilyl]oxy]ethyl]phenyl]-methyl]-7-oxabicyclo[2.2.1]hept-3-yl]-4-oxazolecarboxamide

A solution of 240 mg (0.40 mmol) of Part I oxazole and 34 mg (0.81 mmol, Aldrich) of lithium hydroxide monohydrate in 5 mL of 4:1 THF/water was stirred rapidly at room temperature for 1.5 hours. The reaction mixture was acidified with 2 mL of 1M aqueous HCl solution then added to 20 mL of water and extracted with two-20 mL portions of ethyl acetate. The organic extracts were combined, dried (magnesium sulfate) and concentrated in vacuo to afford the crude acid as an oil. The acid was solubilized in toluene and concentrated in vacuo to remove residual water. To the resulting oil was added 5 mL of sieve-dried toluene (Burdick and Jackson), a small drop of DMF then dropwise at room temperature 50 µL (0.52 mmol, Aldrich) of oxalyl chloride. The reaction mixture was stirred for 1 hour then concentrated in vacuo to give the crude acid chloride as an oil. To a solution of the crude acid chloride in 5 mL of dry THF (distilled from potassium/benzophenone) cooled to 0° was added 95 mg (0.50 mmol) of cyclohexylbutylamine hydrochloride then 170 µL (1.2 mmol, distilled from calcium hydride) of triethylamine. The reaction was stirred for 1 hour then added to 20 mL of 1M aqueous HCl solution and extracted with two-20 mL portions of ethyl acetate. The organic extracts were combined, dried (magnesium sulfate) and concentrated in vacuo to give

an oil. The oil was purified by flash chromatography (Merck silica, 20 x 3 cm, 2:3 ethyl acetate/petroleum ether) to afford 238 mg (0.33 mmol, 83%) of title oxazole as a colorless oil.

K. [1S-(1α,2α,3α,4α)]-3-[[3-[4-[[(4-Cyclohexylbutyl)amino]carbonyl]-2-oxazolyl]-7-oxabicyclo[2.21]hept-2-yl]-methyl]benzeneacetic acid

To a solution of 235 mg (0.33 mmol) of Part J oxazole in 7.5 mL of 2:1 acetonitrile/methylene chloride was added at room temperature 0.35 mL of 48% aqueous hydrofluoric acid. The reaction mixture was stirred for 2 hours then added to 20 mL of saturated aqueous sodium bicarbonate solution and extracted with 20 mL of ethyl acetate and 20 mL of methylene chloride. The organic extracts were combined, dried (magnesium sulfate) and concentrated in vacuo to give an oil. The oil was purified by flash chromatography (Merck silica, 15 x 3 cm, ethyl acetate) to afford 147 mg (0.31 mmol, 94%) of the alcohol as a white solid.

To a solution of 135 mg (0.28 mmol) of the alcohol from above in 5 mL of reagent acetone at room temperature was added 0.35 mL (2.6M in $Cr^{+6}$, Fieser & Fieser, Vol. 1, p. 142) of Jones reagent. The reaction mixture was stirred for 45 minutes then quenched with several drops of isopropyl alcohol. After 15 minutes 20 mL of ethyl acetate was added followed by 20 mL of 3M aqueous sodium bisulfite solution. The mixture was stirred rapidly for 15 minutes then the organic layer was separated and the aqueous layer extracted with 20 mL of methylene chloride. The organic extracts were combined, dried (magnesium sulfate) and concentrated in vacuo to a volume of ~10 mL. The resulting solution was cooled to 0° and treated with excess ethereal diazomethane (until a yellow color persisted). The excess diazomethane was quenched with glacial acetic acid and the solution concentrated in vacuo to give an oil. The oil was purified by flash chromatography (Merck silica, 15 x 1.5 cm, 3:2 ethyl acetate/petroleum ether) to yield 63 mg (0.12 mmol, 43%) of methyl ester of the title acid as a white foam.

A solution of 60 mg (0.12 mmol) of methyl ester and 20 mg (0.48 mmol, Aldrich) of lithium hydroxide monohydrate in 2.5 ml of 4:1 THF/water was stirred at room temperature for 3 hours. The reaction was acidified by addition of 1 ml of 1M aqueous HCl solution then partitioned between 20 ml of ethyl acetate and 20 ml of water. The organic layer was separated, dried (magnesium sulfate) and concentrated in vacuo to give 58 mg (0.58 mmol, 100% from methyl ester) of title acid as a solid white foam.

IR (KBr): 3409 (broad), 2923, 1712, 1649, 1604, 1513 $cm^{-1}$

Partial 270 MHz $^1H$ NMR ($CDCl_3$):

δ2.20 (m, 1H)
2.40 (t, J = 10, 1H)
2.62 (m, 1H)
3.32 (d, J = 9, 1H)
3.36 (m, 2H)
3.56 (s, 2H)
4.39 (d, J = 4, 1H)
4.90 (d, J = 4, 1H)
6.90-7.30 (m, 4H)
8.04 (s, 1H)

67.5 MHz $^{13}C$ NMR ($CDCl_3$): δ 24.2, 26.3, 26.7, 29.0, 29.7, 29.8, 33.3, 35.9, 37.0, 37.5, 39.2, 41.0, 46.6, 50.6, 79.2, 79.9, 127.2, 127.3, 128.6, 129.7, 133.9, 135.7, 140.0, 140.8, 161.0, 163.9, 175.0.

MS(CI): 495 $(M + H)^+$

TLC: $R_f$ (silica gel, 1:9 methanol/methylene chloride) = 0.38, ammonium molybdate/ceric sulfate and UV

| Analysis for $C_{29}H_{38}N_2O_5$: | | | |
|---|---|---|---|
| | C, 70.42; | H, 7.74; | N, 5.67 |
| Found: | C, 70.54; | H, 7.78; | N, 5.57 |

Example 4

[1S-(1α,2α,3α,4α)]-2-[[3[4-[[(4-Cyclohexylbutyl)amino]carbonyl]-2-thiazolyl]-7-oxabicyclo[2.2.1]hept-2-yl]-methyl]benzenepropanoic acid

A. [1S-(1α,2α,3α,4α)]-2-[[3-(Aminocarbonyl)-7-oxabicyclo[2.2.1]hept-2-yl]methyl]benzenepropanoic acid, methyl ester

To a solution of Example 2 Part E acid (9.13 mmol) in dry benzene (100 mL) is added, dropwise over a 10 minute period, oxalyl chloride (0.96 mL) 11 mmol). After stirring for 5 hours, the reaction is concentrated in vacuo, dissolved in dry THF (10 mL) and added dropwise over a 5 minute period to a 0°C solution of concentrated ammonium hydroxide (3 mL) in THF (100 mL). The reaction is then concentrated in vacuo. The residual solid is partitioned between ethyl acetate (150 mL) and 0.25 M $K_2CO_3$ (25 mL). The aqueous layer is extracted with ethyl acetate (25 mL). The combined organic layers are dried ($Na_2SO_4$) and concentrated in vacuo. The residue is suspended in boiling diethyl ether (100 mL). Ethyl acetate (ca. 10 mL) is added to effect solution. The mixture is concentrated to ca. 50 mL on a steam bath, cooled to room temperature, seeded and chilled overnight. Title amide is obtained by filtration.

B. [1S-(1α,2α,3α,4α)]-2-[[3-(Aminothiocarbonyl)-7-oxabicyclo[2.2.1]hept-2-yl]methyl]benzenepropanoic acid, methyl ester

To a 60°C solution of Part A amide (0.999 mmol) in dry toluene (10 mL) is added Lawesson's Reagent (222 mg, 0.55 mmol). The reaction is stirred at 60°C for 30 minutes, diluted with diethyl ether (50 mL), and washed with half-saturated $NaHCO_3$ (2 x 5 mL). The organic layer is dried ($Na_2SO_4$) and concentrated in vacuo. The residue is passed through a short silica plug using 50% ethyl acetate/hexanes to yield the title thioamide.

C. [1S-(1α,2α,3α,4α)]-2-[[3-(4-Carboxy-2-thiazolyl)-7-oxabicyclo[2.2.1]hept-2-yl]methyl]benzenepropanoic acid, methyl ester

To a solution of Part B thioamide (1.41 mmol) and powdered anhydrous $K_2CO_3$ (390 mg, 2.82 mmol) in dry DMF (10 mL) is added, in several portions, bromopyruvic acid (contains 0.4 mol water/mol bromoacid, 295 mg, 1.69 mmol). The reaction is allowed to stir at room temperature for 30 minutes. After this time, an additional 29.5 mg portion of bromopyruvic acid is added. After an additional 1 hour, the solvent is removed in vacuo below 30°C. The residue is suspended/dissolved in methylene chloride (10 mL). Triethylamine (0.59 mL, 4.2 mmol) is added followed by the dropwise addition of methanesulfonyl chloride (0.33 mL, 4.2 mmol). After stirring for 5 minutes, the reaction is diluted with diethyl ether (40 mL). The organic layer is extracted with 0.5M $K_2CO_3$ (9 x 10 mL). The combined aqueous layers are brought to pH 1.5 with 6 N HCl and extracted with diethyl ether (6 x 25 mL). These combined organic layers are dried ($Na_2SO_4$) and concentrated in vacuo.

D. [1S-(1α,2α,3α,4α)]-2-[[3-[4-[[(4-Cyclohexylbutyl)amino]carbonyl]-2-thiazolyl]-7-oxabicyclo[2.2.1]hept-2-yl]-methyl]benzenepropanoic acid, methyl ester

To a solution of Part C acid (0.120 mmol) in dry DMF (1 mL) is added 1,1′-carbonyldiimidazole (20.3 mg, 0.125 mmol). The reaction is allowed to stir for 1 hour. A solution of (cyclohexylbutyl)amine hydrochloride (23.4 mg, 0.131 mmol) and triethylamine (0.020 mL, 0.14 mmol) in dry DMF (0.5 mL) is then added. The reaction is stirred for 1 hour, and concentrated to remove DMF. The residue is taken up in diethyl ether (20 mL) and 0.5 N HCl (5 mL). The organic layer is dried ($Na_2SO_4$) and concentrated in vacuo. The resultant material is chromatographed (silica, ethyl acetate/hexanes) to yield title ester.

E. [1S-(1α,2α,3α,4α)]-2-[[3-[4-[[(4-Cyclohexylbutyl)amino]carbonyl]-2-thiazolyl]-7-oxabicyclo[2.2.1]hept-2-yl]-methyl]benzenepropanoic acid

To a solution of Part D amide in methanol (1 mL) is added 2 N KOH (0.3 mL). The reaction is stirred for 2 hours. An additional 0.3 mL portion of KOH is added. After an additional 1 hour, the reaction is concentrated to remove methanol. The residue is dissolved in water (1 mL) and 1 N HCl is added to bring the pH to 2. The mixture is extracted with methylene chloride (3 x 5 mL). the combined organic layers are

dried ($Na_2SO_4$) and concentrated in vacuo to yield title compound.

Example 5

[1S-(1α,2α,3α,4α)]-2-[[3-[4-[[(4-Cyclohexylbutylamino]carbonyl]-1H-imidazol-2-yl]-7-oxabicyclo[2.2.1]hept-2-yl]methyl]benzenepropanoic acid

A. [1S-(1α,2α,3α,4α)]-2-[[3-Cyano-7-oxabicyclo[2.2.1]hept-2-yl]methyl]benzenepropanoic acid, methyl ester

Vilsmeier reagent is prepared by the addition of oxalyl chloride (1 mmol) to a 0°C acetonitrile solution of DMF (1 mmol). To the reagent is added Example 4, Part A amide (1 mmol). After 45 minutes, pyridine (2 mmol) is added. The reaction is then partitioned between diethyl ether and 1N HCl. The title nitrile is isolated by drying and concentrating the organic phase.

B. [1S-(1α,2α,3α,4α)]-2-[[3-(Iminomethoxymethyl)-7-oxabicyclo-[2.2.1]hept-2-yl]methyl]benzenepropanoic acid, methyl ester HCl salt

Gaseous HCl is bubbled into a 0°C solution of part A nitrile (1 mmol) and methanol (2 mmol) in diethyl ether (10 mL) for 5 minutes. The mixture is allowed to stand at 0°C for 7 days and is then concentrated to yield the title compound.

C. [1S-(1α,2α,3α,4α)]-2-[[3-[Methoxy[[2-oxo-2-[2-(trimethylsilyl)ethoxy]ethyl]imino]methyl]-7-oxabicyclo-[2.2.1]hept-2-yl]methyl]benzenepropanoic acid, methyl ester

Part B iminoether (1 mmol) is suspended in 10 mL of THF and stirred with glycine, 2-(trimethylsilyl)-ethyl ester HCl salt (2 mmol) and triethylamine (2 mmol). After 1 hour, the organic layer is diluted with diethyl ether, washed with $NaHCO_3$ solution, dried and concentrated to yield title ester which is used for the subsequent step.

D. [1S-(1α,2α,3α,4α)]-2-[[3-[[[1-(Hydroxymethylene)-2-oxo-2-[2-(trimethylsilyl)ethoxy]ethyl]imino]-methoxymethyl]-7-oxabicyclo[2.2.1]hept-2-yl]methyl]benzenepropanoic acid, methyl ester, monopotassium salt

To a 0°C solution of potassium methoxide (2 mmol) is added dropwise, a solution of ethyl formate (4 mmol) and Part C ester (1 mmol). After 3 hours, the potassium salt is collected by filtration and used immediately for the next step.

E. [1S-(1α,2α,3α,4α)]-2-[[3-[4-[[2-(Trimethylsilyl)ethoxy]carbonyl]-1H-imidazol-2-yl]-7-oxabicyclo[2.2.1]hept-2-yl]methyl]benzenepropanoic acid, methyl ester

The Part D ester is dissolved in concentrated ammonia. After 3 hours, the solution is bubbled with $N_2$ to purge excess $NH_3$ and the residue neutralized with 1N HCl. The mixture is extracted with $CH_2Cl_2$. The organic layers are concentrated in vacuo after drying to yield title imidazole.

F. [1S-(1α,2α,3α,4α)]-2-[[3-(4-Carboxy-1H-imidazol-2-yl)-7-oxabicyclo[2.2.1]hept-2-yl]methyl]-benzenepropanoic acid, methyl ester

To a solution of Part E imidazole (1 mmol) in THF (10 mL) is added 1.1 mL of 1M tetra-n-butylammonium fluoride (in THF). After stirring for 1 hour, the reaction is concentrated, diluted with water, brought to pH 7 with 1N HCl, and extracted with methylene chloride. The organic layer is concentrated after drying to yield title acid.

G. [1S-(1α,2α,3α,4α)]-2-[3-[[(4-Cyclohexylbutyl)amino]carbonyl]-1H-imidazol-2-yl]-7-oxabicyclo[2.2.1]hept-2-yl]methyl]benzenepropanoic acid, methyl ester

To a stirred mixture of 1.0 mmol of Part F acid, 1.0 mmol of 4-cyclohexylbutylamine hydrochloride, 1.0 mmol of 1-hydroxybenzotriazole in 10 mL of dry dimethylformamide under argon at 0°C is added triethylamine (1.5 mmol). The mixture is stirred at 0°C for 10 minutes at which time 1-(3-dimethylaminopropyl)-3-ethylcarbodiimide hydrochloride (1 mmol) is added. The reaction mixture is stirred at 23 °C for 24 hours and concentrated in vacuo. The residue is dissolved in ethyl acetate and washed with saturated $NaHCO_3$, dried ($MgSO_4$), filtered and concentrated in vacuo to provide title amide.

H. [1S-(1α,2α,3α,4α)]-2-[[3-[4-[[(4-Cyclohexylbutyl)amino]carbonyl]-1H-imidazol-2-yl]-7-oxabicyclo[2.2.1]-hept-2-yl]methyl]benzenepropanoic acid

To a solution of Part G amide (1 mmol) in 10 mL of methanol is added 2 mL of 2N KOH. After stirring for 4 hours, the reaction is concentrated to remove methanol. The residue is diluted with water and brought to pH 2 with 1N HCl. The mixture is concentrated and the solid is extracted into methanol. The methanol is concentrated and the residue is extracted into 5:1 chloroform/methanol. The organics are concentrated to yield crude title acid which can be purified by chromatography on silica using ethyl acetate/pyridine/acetic acid/water.

Example 6

[1S-(1α,2α,3α,4α)]-2-[3-[4-[[(4-Cyclohexylbutyl)amino]carbonyl]-2-oxazolyl]-7-oxabicyclo[2.2.1]hept-2-yl]-N-(phenylsulfonyl)benzene propionamide

To a stirred mixture of 1-(3-dimethylaminopropyl)-3-ethylcarbodiimide hydrochloride (1.0 mmol) and 4-dimethylaminopyridine (1.0 mmol) in 8 mL of dimethylformamide is added 1.0 mmol of benzenesulfonamide and 2.0 mmol of triethylamine followed by 1.0 mmol of Example 1 title acid. This mixture is stirred at room temperature for 48 hours and concentrated in vacuo. The residue is partitioned between water and ethyl acetate. The aqueous layer is acidified and extracted with ethyl acetate. Combined ethyl acetate layers are concentrated in vacuo to afford title sulfonamide.

Example 7

[1S-(1α,2α,3α,4α)]-3-[[3-[4-[[[4-(4-Chlorophenyl)butyl]amino]carbonyl]-2-oxazolyl]-7-oxabicyclo[2.2.1]hept-2-yl]methyl]benzoic acid

A. 3-Bromobenzenemethanol

To a solution of 50.0 g (0.25 mols, Aldrich) of 3-bromobenzoic acid in 100 ml of dry tetrahydrofuran (THF) cooled to 0° is added slowly 100 ml (1.0 M in THF) 100 mmols, Aldrich) of borane-tetrahydrofuran complex. The reaction is stirred at 0° for 1 hour then at room temperature for 18 hours. The reaction is quenched by dropwise addition of water then concentrated in vacuo to remove solvent. The residue is partitioned between diethyl ether and 1 M aqueous HCl. The organic layer is separated, washed with 1 M aqueous sodium hydroxide, brine, dried (magnesium sulfate) and concentrated in vacuo to afford title compound.

B. 3-Bromo-1-[[[dimethyl(1,1,2-trimethylpropyl)silyl]oxy]methyl]benzene

To a solution of 25.2 g (135 mmol) of crude Part A alcohol and 24.1 g (135 mmol, Petrarch) of thexyldimethylchlorosilane in 200 ml of dry methylene chloride (distilled from phosphorous pentoxide) is added at room temperature 20 ml (143 mmol, distilled from calcium hydride) of triethylamine then 200 mg (1.64 mmol, Aldrich) of 4-dimethylaminopyridine. The reaction mixture is stirred at room temperature for 18 hours. The resulting slurry is diluted with 100 ml of hexane, cooled to 0° with stirring for 15 minutes then filtered to remove solid triethylamine hydrochloride. The filtrate is concentrated in vacuo to give an oil. The crude oil is purified by flash chromatography (Merck silica) to afford title compound.

C. [1S-(1α,2α,3α,4α)]-[3-[[[Dimethylsilyl(1,1,2-trimethylpropyl)]oxy]methyl]phenyl]-7-oxabicyclo[2.2.1]-heptane-2,3-dimethanol

To a solution of 4.61 g (14.0 mmol) of Part B compound in 30 ml of dry diethyl ether (distilled from ketyl) cooled to -100° is added dropwise 15 ml (1.7 M in pentane, 25 mmol, Aldrich) of t-butyllithium solution over 15 minutes. The reaction mixture is stirred at -100° for 15 minutes then at 0° for 15 minutes. The resulting anion solution is re-cooled to -78° then 30 ml of dry THF (distilled from ketyl) is introduced

followed by the rapid addition of a solution of 875 mg (5.61 mmol) of [3aR-(3aα,4β,7β,7aα)]-octahydro-4,7-epoxyisobenzofuran-1-ol in 10 ml of THF. The reaction mixture is warmed to 0°, stirred for 1 hour, quenched with 5 ml of water then partitioned between 100 ml of water and 25 ml of ethyl acetate. The organic layer is separated and the aqueous layer is extracted with an additional 25 ml of ethyl acetate. The organic extracts are combined, dried (magnesium sulfate) and concentrated in vacuo give an oil. The crude oil is purified by flash chromatography (Merck silica) to afford title diasteromeric alcohols.

D. [1S-(1α,2α,3α,4α)]-[3-[[[Dimethylsilyl(1,1,2-trimethylpropyl)]oxy]methyl]phenyl]-7-oxabicyclo[2.2.1]-heptane-2,3-dimethanol, diacetate

A solution of 2.20 g (5.41 mmol) of Part C diol and 50 mg (0.41 mmol, Aldrich) of 4-dimethylaminopyridine in 10 ml of 1:1 acetic anhydride/pyridine is stirred at room temperature for 6 hours. The reaction mixture is concentrated in vacuo and the residue partitioned between 25 ml of ethyl acetate and 25 ml of 1 M aqueous HCl. The organic layer is separated, washed with 25 ml of 1 M aqueous sodium hydroxide, dried (magnesium sulfate) and concentrated in vacuo to give title compound.

E. [1S-(1α,2α,3α,4α)]-3-[Hydroxy[3-(hydroxymethyl)-7-oxabicyclo[2.2.1]hept-2-yl]methyl]benzoic acid, methyl ester

To a solution of 1.10 g (2.24 mmol) of Part D diacetate in 15 ml of reagent acetone cooled to 0° is added rapidly 3.3 ml (2.6 M in $Cr^{+6}$, for preparation see Fieser and Fieser, "Reagents for Organic Synthesis", vol. 1, p. 142) of Jones reagent. The reaction mixture is stirred for 2 hours, quenched by addition of 1 ml of isopropanol and stirred for an additional 30 minutes. The resulting green slurry is filtered through a pad of Celite. The filtrate is concentrated in vacuo and the residue partitioned between 25 ml of diethyl ether and 25 ml of water. The organic layer is separated and concentrated in vacuo to give the crude diacetate-acid as an oil.

A solution of the crude diacetate-acid in 15 ml of 2:1 1 M aqueous NaOH/THF is stirred at room temperature for 90 minutes. The reaction mixture is cooled in an ice-bath, quenched by addition of 15 ml of 1 M aqueous HCl solution then extracted with two-25 ml portions of diethyl ether. The ether extracts are combined, washed with 25 ml of brine and concentrated in vacuo to give the crude diol-acid.

A solution of the crude diol-acid in 10 ml of diethyl ether is treated with ethereal diazomethane at 0° then concentrated in vacuo. The resulting oil is purified by flash chromatography (Merck silica) to afford title compound.

F. [1S-(1α,2α,3α,4α)]-3-[[3-(Hydroxymethyl)-7-oxabicyclo[2.2.1]hept-2-yl]methyl]benzoic acid, methyl ester

A mixture of 450 mg (1.54 mmol) of Part E diol and 450 mg of 10% palladium on carbon catalyst (Aldrich) in 10 ml of glacial acetic acid is shaken under an atmosphere of hydrogen (50 psi) for 24 hours. The reaction is filtered and the filtrate concentrated in vacuo to give an oil. The crude material is purified by flash chromatography (Merck silica) to give title product.

G. [1S-(1α,2α,3α,4α)]-3-[[3-Carboxy-7-oxabicyclo[2.2.1]hept-2-yl]methyl]benzoic acid, methyl ester

To a solution of 450 mg (1.63 mmol) of Part F alcohol in 5 ml of reagent acetone cooled to 0° is added rapidly 2.0 ml (2.6 M in $Cr^{+6}$ of Jones reagent. The reaction mixture is warmed to room temperature, stirred for 2 hours then quenched by addition of ~1 ml of isopropanol. After 15 minutes the resulting green slurry is filtered through a pad of Celite. The filtrate is partitioned between 20 ml of diethyl ether and 20 ml of water. The organic layer is separated and the aqueous layer is extracted with an additional 20 ml of diethyl ether. The ether extracts are combined, dried (magnesium sulfate) and concentrated in vacuo to give the crude title acid.

H. [1S-(1α,2α,3α,4α)]-3-[[3-[[[1-(Hydroxymethyl)-2-oxo-2-(phenylmethoxy)ethyl]amino]carbonyl]-7-oxabicyclo[2.2.1]hept-2-yl]methyl]benzoic acid, methyl ester

To a solution of 447 mg (1.54 mmol) of Part G acid in 10 ml of dry THF (distilled from ketyl) cooled to 0° is added 392 mg (1.69 mmol, Sigma) of L-serine benzyl ester hydrochloride, 228 mg (1.69 mmol, Aldrich) of 1-hydroxybenzotriazole hydrate and 530 µl (3.8 mmol, distilled from calcium hydride) of triethylamine. The mixture is stirred for 5 minutes then 348 mg (1.69 mmol, Aldrich) of dicyclohexylcarbodiimide is added in one portion. The reaction is stirred at 0° for 3 hours then warmed to room temperature for 16 hours. The resulting slurry is diluted with 10 ml of ethyl acetate, cooled to 0° for 15 minutes then filtered. The filtrate is concentrated in vacuo to give an oil. The crude material is purified by flash chromatography (Merck silica) to afford title ester.

I. [1S-(1α,2α,3α,4α)]-3-[[3-[4,5-Dihydro-4-[(phenylmethoxy)carbonyl]-2-oxazolyl]-7-oxabicyclo[2.2.1]hept-2-yl]methyl]benzoic acid, methyl ester

To a solution of 495 mg (1.06 mmol) of Part H ester, 843 mg (3.10 mmol, Aldrich) of triphenylphosphine and 540 µL (3.1 mmol, Aldrich) of diisopropylethylamine in 6 ml of 5:1 dry acetonitrile/methylene chloride is added at room temperature 300 µL (3.1 mmol, Mallinckrodt) of reagent carbon tetrachloride. The reaction mixture is stirred for 2 hours then diluted with 15 ml of ethyl acetate followed by the slow addition of 15 ml of saturated aqueous sodium bicarbonate solution. The resulting mixture is stirred for 5 minutes then partitioned between 20 ml of ethyl acetate and 20 ml of water. The organic layer is separated, washed with 20 ml of brine, dried (sodium sulfate) and concentrated in vacuo. The crude material is purified by flash chromatography (Merck silica) to afford title oxazoline.

J. [1S-(1α,2α,3α,4α)]-3-[[3-[4-[(Phenylmethoxy)carbonyl]-2-oxazolyl]-7-oxabicyclo[2.2.1]hept-2-yl]methyl]-benzoic acid, methyl ester

To a solution of 355 mg (0.79 mmol) of Part I oxazoline in 10 ml of dry methylene chloride (distilled from phosphorous pentoxide) is added 750 mg of nickel peroxide oxidant (prepared as described in Example 1 Part J) at room temperature. The reaction mixture is stirred for 1 hour then an additional 750 mg of oxidant is added. After 30 minutes the reaction mixture is diluted with 20 ml of ethyl acetate followed by the addition of 10 ml of 3M aqueous sodium bisulfite solution. The resulting mixture is stirred rapidly for 20 minutes then 10 ml of water is added. The organic layer is separated and the aqueous layer extracted with an additional 20 ml of ethyl acetate. The organic extracts are combined, washed with 25 ml of 1M aqueous sodium citrate solution, dried (magnesium sulfate) and concentrated in vacuo to give an oil. The crude material is purified by flash chromatography (Merck silica) to afford title oxazole.

K. [1S-(1α,2α,3α,4α)]-3-[[3-[4-[[[4-(4-chlorophenyl)butyl]amino]carbonyl]-2-oxazolyl]-7-oxabicyclo[2.2.1]hept-2-yl]methyl]benzoic acid, methyl ester

A mixture of 165 mg (0.37 mmol) of Part J oxazole and 30 mg of 20% palladium hydroxide on carbon catalyst (moist, <50% water, Aldrich) in 5 ml of reagent ethyl acetate is stirred under an atmosphere of hydrogen (balloon) for 1 hour. The catalyst is removed by filtration through a 4µM polycarbonate membrane. The filtrate is concentrated in vacuo to afford crude acid.

To a solution of 125 mg (0.35 mmol) of the crude acid in 3 ml of dry methylene chloride (distilled from phosphorous pentoxide) is added at room temperature a small drop of dimethylformamide (DMF) then 40 µl (0.46 mmol, Aldrich) of oxalyl chloride. The reaction mixture is stirred for 30 minutes then concentrated in vacuo to give the crude acid chloride. The acid chloride is solubilized in 3 ml of dry methylene chloride then cooled to 0° and a solution of 84 mg (0.46 mmol) of 4-chlorophenylbutylamine and 70 µl (0.50 mmol, distilled from calcium hydride) of triethylamine in 1 ml of dry methylene chloride is added rapidly. The reaction mixture is stirred for 30 minutes then partitioned between 25 ml of ethyl acetate and 15 ml of 1M aqueous HCl solution. The organic layer is separated and the aqueous layer is extracted with an additional 10 ml of ethyl acetate. The organic extracts are combined, dried (magnesium sulfate) and concentrated in vacuo. The crude material is purified by flash chromatography (Merck silica) to afford title ester.

L. [1S-(1α,2α,3α,4α)]-3-[[3-[4-[[[4-(4-Chlorophenyl)butyl]amino]carbonyl]-2-oxazolyl]-7-oxabicyclo[2.2.1]hept-2-yl]methyl]benzoic acid

A solution of 151 mg (0.29 mmol) of Part K ester and 25 mg (0.60 mmol, Aldrich) of lithium hydroxide monohydrate in 6 ml of 2:1 THF/water is stirred rapidly at room temperature for 1.5 hours. The reaction is acidified by addition of 2 ml of 1M aqueous HCl solution then partitioned between 20 ml of ethyl acetate and 20 ml of water. The organic layer is separated, washed with 20 ml of brine, dried (magnesium sulfate) and concentrated in vacuo to afford title product.

Example 8

[1S-(1α,2α,3α,4α)]-5-[[3-[[3-[4-[[(4-Cyclohexylbutyl)amino]carbonyl]-2-oxazolyl]-7-oxabicyclo[2.2.1]hept-2-yl]-methyl]phenyl]methyl]-1H-tetrazole

A. [1S-(1α,2α,3α,4α)]-3-[[3-[4-[[(4-Cyclohexylbutyl)amino]carbonyl]-2-oxazolyl]-7-oxabicyclo[2.2.1]hept-2-yl]-methyl]benzeneacetonitrile

To a solution of 1 mmol of Example 7, Part K ester in 10 mL of tetrahydrofuran is added 2 mmol of lithium borohydride. The reaction mixture is stirred for 1 hour at 23 °C and then is quenched by the addition of 1M HCl solution. The aqueous layer is extracted with ethyl acetate. The organic layers are dried, filtered and concentrated in vacuo to afford the crude alcohol. This alcohol is converted to the title nitrile using the procedures of Example 2 Part I(b) and I(c).

B. [1S-(1α,2α,3α,4α)]-5-[[3-[[3-[4-[[(4-Cyclohexylbutyl)amino]carbonyl]-2-oxazolyl]-7-oxabicyclo[2.2.1]hept-2-yl]methyl]phenyl]methyl]-1H-tetrazole

A mixture of 300 mg (0.61 mmol) of Part A nitrile, 65 mg (1.0 mmol) of sodium azide, 53 mg (1.0 mmol) of ammonium chloride and 42 mg (1.0 mmol) of lithium chloride in 5 ml of dry dimethylformamide is heated to 125° for 24 hours. The reaction is cooled and filtered. The filtrate is concentrated in vacuo and the residue partitioned between water and ethyl acetate. The aqueous layer is adjusted to pH = 2 with 1M aqueous HCl. The organic layer is separated, dried ($MgSO_4$) and concentrated in vacuo to give an oil. The crude material is purified by flash chromatography (Merck silica) to give title compound.

Example 9

[1S-(1α,2α,3α,4α)]-[2-[[3-[4-[[(4-Cyclohexylbutyl)amino]carbonyl]-2-oxazolyl]-7-oxabicyclo[2.2.1]hept-2-yl]-methyl]phenoxy]aceticacid

A. 1-Bromo-2-(methoxymethoxy)benzene

The oil was removed from 4.5 g (60% in oil, 112 mmol, Aldrich) of sodium hydride dispersion by three-20 mL washes with hexane then the residue was covered with 75 mL of dimethylformamide (Burdick and Jackson). The resulting mixture was heated to about 50° and 18.1 g (105 mmol, Aldrich) of 2-bromophenol was added dropwise over 15 minutes. Vigorous gas evolution was observed. The reaction was stirred for an additional 30 minutes then the resulting gray-brown solution was cooled to 0° and 9.6 mL (117 mmol, Aldrich) of bromomethyl methyl ether was added dropwise over 15 minutes. The reaction mixture was stirred for 1 hour at 0° then at room temperature for 16 hours. The resulting slurry was partitioned between 200 mL of 1M aqueous sodium hydroxide solution and 150 mL of 4:1 hexane/diethyl ether. The aqueous layer was separated and extracted with an additional 100 mL of 4:1 hexane/diethyl ether. The organic extracts were combined, washed with two-200 mL portions of water, dried (magnesium sulfate) and concentrated in vacuo to give 22.2 g (102 mmol, 97%) of title compound as a pale yellow liquid.

B. [1S-(1α,2α,3α,4α)]-[2-(Methoxymethoxy)phenyl]-7-oxabicyclo[2.2.1]heptane-2,3-dimethanol

To a solution of 16.7 g (77.0 mmol) of Part A aryl bromide in 150 mL of dry THF (distilled from potassium/benzophenone) cooled to -78° was added dropwise 48 mL (1.6M in hexane, 77 mmol, Aldrich) of n-butyllithium over 30 minutes. The reaction mixture was stirred at -78° for 1 hour. To the resulting white slurry of the anion was added a solution of 4.80 g (30.8 mmol, of [3aR-(3aα,4β,7β,7aα)]-octahydro-4,7-epoxyisobenzofuran-1-ol in 30 mL of dry THF over 5 minutes. The reaction was warmed to 0° (becomes homogeneous), stirred for 2 hours then quenched with 5 mL of methanol and concentrated in vacuo. The residue was partitioned between 100 mL of brine and 100 mL of ethyl acetate then an additional 50 mL of water was added. The aqueous layer was separated and extracted with 100 mL of ethyl acetate. The organic extracts were combined, dried (magnesium sulfate) and concentrated in vacuo to give an oil. The crude material was purified by flash chromatography (Merck silica, 22 x 5.0 cm, 1:2 ethyl acetate/petroleum ether then ethyl acetate) to afford 8.49 g (28.9 mmol, 94%) of title diol as an oil.

C. [1S-(1α,2α,3α,4α)]-2-[[2-(Methoxymethoxy)phenyl]methyl]-7-oxabicyclo[2.2.1]heptane-3-methanol

A mixture of 8.40 g (28.6 mmol) of Part B diol and 8.0 g of 10% palladium on carbon catalyst (Aldrich) in 75 mL of glacial acetic acid was stirred under an atmosphere of hydrogen (balloon) for 18 hours. The resulting mixture was filtered on a Buchner funnel then passed through a 4 μM polycarbonate membrane. The filtrate was concentrated in vacuo (oil pump vacuum) to give an oil. The oil was partitioned between 75 mL of ethyl acetate and 100 mL of 1M aqueous sodium hydroxide solution (pH=12 of aqueous) then an equal volume of brine was added (100 mL). The aqueous layer was separated and extracted with an additional 50 mL of ethyl acetate. The organic extracts were combined, dried (magnesium sulfate) and concentrated in vacuo to afford 7.56 g (27.2 mmol, 95%) of title alcohol as a colorless oil.

D. [1S-(1α,2α,3α,4α)]-2-[[2-(Methoxymethoxy)phenyl]methyl]-3-[(phenylmethoxy)methyl]-7-oxabicyclo[2.2.1]-heptane

The oil was removed from 552 mg (60% in oil, 13.8 mmol, Aldrich) of sodium hydride dispersion by three washes with petroleum ether then the residue was covered with 15 mL of dry THF (distilled from potassium/benzophenone). The mixture was heated to about 50° then added dropwise was a solution of 3.50 g (12.6 mmol) of Part C alcohol in 15 mL of dry THF. Vigorous gas evolution was observed. The reaction was stirred for an additional 30 minutes then cooled to 0°. To the resulting anion solution was added 465 mg (1.26 mmol, Fluka) of tetra-n-butylammonium iodide then dropwise 1.6 mL (14 mmol, Aldrich) of benzyl bromide. The reaction was stirred at 0° for 2 hours then at room temperature for 16 hours. The resulting mixture was quenched with 5 mL of water then partitioned between 100 mL of 1M aqueous HCl solution and 50 mL of ethyl acetate. The aqueous layer was separated and extracted with an additional 50 mL of ethyl acetate. The organic extracts were combined, washed with 100 mL of 1M aqueous sodium hydroxide solution, dried (magnesium sulfate) and concentrated in vacuo to give 4.55 g (12.4 mmol, 98%) of crude title compound as a yellow oil.

E. [1S-(1α,2α,3α,4α)]-2-[[3-[(Phenylmethoxy)methyl]-7-oxabicyclo[2.2.1]hept-2-yl]methyl]phenol

To a solution of 4.53 g (12.3 mmol) of Part D title compound in 12 mL of dioxane (Burdick and Jackson) was added at room temperature 30 mL of 1:4 concentrated HCl/methanol. The reaction was stirred for 5 hours then concentrated in vacuo. The residue was partitioned between 50 mL of 1M aqueous HCl solution and 75 mL of ethyl acetate then 50 mL of brine was added. The organic layer was separated, dried (magnesium sulfate) and concentrated in vacuo to give an orange oil. The crude oil was purified by flash chromatography (Merck silica, 12 x 5.0 cm, 1:1:3 ethyl acetate/methylene chloride/hexane) to afford 3.46 g (10.7 mmol, 87%) of title phenol as a pale yellow glass.

F. [1S-(1α,2α,3α,4α)]-[2-[[3-[(Phenylmethoxy)methyl]-7-oxabicyclo[2.2.1]hept-2-yl]methyl]phenoxy]acetic acid, ethyl ester

The oil was removed from 420 mg (60% in oil. 11 mmol, Aldrich) of sodium hydride dispersion by three washes with hexane then 15 mL of dry THF (distilled from potassium/benzophenone) was added. To the resulting stirred mixture at room temperature was added dropwise a solution of 3.30 g (10.2 mmol) of Part E phenol in 20 mL of dry THF over about 15 minutes. Vigorous gas evolution was observed. The reaction was stirred for an additional 30 minutes then cooled to 0° and a solution of 1.75 g (10.5 mmol, Aldrich) of ethyl bromoacetate in 2 mL of THF was added dropwise. The reaction mixture was stirred for 1.5 hours then quenched with 50 mL of 1M aqueous HCl solution. The resulting mixture was added to 50 mL of brine then extracted with 75 mL of ethyl acetate. The organic extract was dried (magnesium sulfate) and concentrated in vacuo to give an oil. The crude oil was purified by flash chromatography (Merck silica, 12 x 5.0 cm, 1:2 ethyl acetate/hexane) to afford 3.87 g (9.44 mmol, 93%) of title ester as a pale yellow oil.

G. [1S-(1α,2α,3α,4α)]-[2-[[3-(Hydroxymethyl)-7-oxabicyclo[2.2.1]hept-2-yl]methyl]phenoxy]acetic acid ethyl ester

A mixture of 3.60 g (8.78 mmol) Part F ester and 180 mg of 20% palladium hydroxide on carbon catalyst (moist, Aldrich) in 25 mL of ethyl acetate was stirred under hydrogen (balloon) for 2 hours (TLC showed little reaction). Added to the reaction was 12 mL of absolute ethanol then 0.3 mL of concentrated HCl. The reaction was stirred for 2 hours (TLC showed little reaction) then an additional 360 mg of catalyst

was added. The resulting mixture was stirred for 20 hours, filtered on a Buchner funnel then through a 4μM polycarbonate membrane. The filtrate was concentrated in vacuo to give an oil. The crude oil was purified by flash chromatography (Merck silica, 15 x 5.0 cm, 2:1 ethyl acetate/hexane) to afford 1.20 g (3.75 mmol, 43%) of desired title alcohol as an oil and 1.48 g (4.09 mmol, 47%) of corresponding acetate as an oil.

H. [1S-(1α,2α,3α,4α)]-[2-[[3-Carboxy-7-oxabicyclo[2.2.1]hept-2-yl]methyl]phenoxy]acetic acid, ethyl ester

To a solution of 1.17 g (3.66 mmol) of Part G alcohol in 15 mL of reagent acetone cooled to 0° was added rapidly 2.5 mL (2.6M in $Cr^{+6}$, 6.5 mmol, prepared as described in Fieser & Fieser, Vol. 1, p 142) of Jones reagent. The reaction was stirred for 1 hour at 0° then 30 minutes at room temperature. The mixture was re-cooled to 0°, quenched with 2 mL of isopropanol and stirred for an additional 30 minutes. The resulting green slurry was filtered through a pad of Celite. The filtrate was concentrated in vacuo and the residue partitioned between 20 mL of 1M HCl solution and 20 mL of ethyl acetate. The organic extract was separated, washed with 20 mL of brine, dried (magnesium sulfate) and concentrated in vacuo to give 1.19 g (3.56 mmol, 97%) of crude title acid as an oil.

I. [1S-(1α,2α,3α,4α)]-[2-[[3-[[[1-(Hydroxymethyl)-2-oxo-2-(phenylmethoxy)ethyl]amino]carbonyl]-7-oxabicyclo-[2.2.1]hept-2-yl]methyl]phenoxy]acetic acid, ethyl ester

To a solution of 1.19 g (3.69 mmol) of crude Part H acid in 15 mL of dry THF (distilled from potassium/benzophenone) cooled to 0° was added 540 mg (4.00 mmol, Aldrich) of 1-hydroxybenzotriazole hydrate, 928 mg (4.00 mmol, Sigma) of L-serine benzyl ester hydrochloride then 1.2 mL (8.5 mmol, distilled from calcium hydride) of triethylamine. The slurry was stirred for 5 minutes then 824 mg (4.00 mmol, Aldrich) of dicyclohexylcarbodiimide was added. The reaction mixture was stirred at 0° for 3 hours then at room temperature for 16 hours. The resulting slurry was diluted with 15 mL of ethyl acetate then filtered. The filtrate was concentrated in vacuo to give an oil. The crude material was purified by flash chromatography (Merck silica, 12 x 5.0 cm, ethyl acetate) to afford 1.33 g (2.60 mmol, 73%) of title amide as a solid.

J. [1S-(1α,2α,3α,4α)]-[2-[[3-[4,5-Dihydro-4-[(phenylmethoxy)carbonyl]-2-oxazolyl]-7-oxabicyclo[2.2.1]hept-2-yl]methyl]phenoxy]acetic acid, ethyl ester

To a solution of 1.32 g (2.58 mmol) of Part I amide, 1.02 g (3.90 mmol, Aldrich) of triphenylphosphine and 0.70 mL (4.0 mmol, Aldrich) of diisopropylethylamine in 12 mL of 5:1 dry acetonitrile/methylene chloride was added at room temperature 380 mL (3.9 mmol) of reagent carbon tetrachloride. The reaction was stirred for 2.5 hours then added was 30 mL of ethyl acetate and 30 mL of saturated sodium bicarbonate solution. The organic layer was separated, washed with 30 mL of brine, dried (sodium sulfate) and concentrated in vacuo to give an oily solid. The crude material was purified by flash chromatography (Merck silica, 15 x 5.0 cm, 4:1 ethyl acetate/hexane) to afford 883 mg (1.79 mmol, 69%) of title oxazoline as an oil.

K. [1S-(1α,2α,3α,4α)]-[2-[[3-[4-[(Phenylmethoxy)carbonyl]-2-oxazolyl]-7-oxabicyclo[2.2.1]hept-2-yl]methyl]-phenoxy]acetic acid, ethyl ester

To a solution of 860 mg (1.74 mmol) of Part J oxazoline in 15 mL of dry methylene chloride (distilled from phosphorous pentoxide) was added at room temperature 1.7 g of nickel peroxide oxidant (prepared as described in Example 1, Part J), in one portion. The reaction was stirred for 45 minutes (incomplete by TLC) then an additional 1.7 g of oxidant was added. After 45 minutes an additional 0.85 g of oxidant was added. The reaction mixture was stirred for 45 minutes (starting material consumed by TLC) then 50 mL of ethyl acetate was added followed by 100 mL of 3M aqueous sodium bisulfite solution. The mixture was stirred rapidly for 30 minutes (green emulsion) then 50 mL of 1M aqueous sodium citrate solution was added. After stirring for 15 minutes two layers formed. The organic layer was separated and the aqueous layer was extracted with an additional 50 mL of ethyl acetate. The combined organic extracts were dried (magnesium sulfate) and concentrated in vacuo to give an oil. The crude material was purified by flash chromatography (Merck silica, 15 x 3.0 cm, 2:3 ethyl acetate/hexane) to afford 390 mg (0.79 mmol, 45%) of title oxazole as a foam.

L. [1S-(1α,2α,3α,4α)]-[2-[[3-(4-Carboxy-2-oxazolyl)-7-oxabicyclo[2.2.1]hept-2-yl]methyl]phenoxy]acetic acid, ethyl ester

A mixture of 385 mg (0.78 mmol) of Part K oxazole and 38 mg of 20% palladium hydroxide on carbon catalyst (moist, Aldrich) in 10 mL of ethyl acetate was stirred rapidly under an atmosphere of hydrogen (balloon) for 1.5 hours then magnesium sulfate was added. The reaction mixture was passed through a 4 µM polycarbonate membrane. The filtrate was concentrated in vacuo to give 314 mg (0.78 mmol, 100%) of title oxazole acid as a white solid, mp 151-154°.

M. [1S-(1α,2α,3α,4α)]-[2-[[3-[4-[[(4-Cyclohexylbutyl)amino]carbonyl]-2-oxazolyl]-7-oxabicyclo[2.2.1]hept-2-yl]methyl]phenoxy]acetic acid, ethyl ester

To a solution of 310 mg (0.77 mmol) of Part L oxazole acid in 5 mL of dry methylene chloride (distilled from phosphorous pentoxide) was added a small drop of dimethylformamide then 90 µL (1.0 mmol, Aldrich) of oxalyl chloride. The reaction was stirred until gas evolution ceased, about 30 minutes, then concentrated in vacuo to give the crude acid chloride as a yellow foam. The acid chloride was solubilized in 3 mL of dry methylene chloride then cooled to 0° and added dropwise was a solution of 192 mg (1.00 mmol) of cyclohexylbutylamine hydrochloride and 280 µL (2.0 mmol, distilled from calcium hydride) of triethylamine in 5 mL of dry methylene chloride. The reaction mixture was stirred for 15 minutes then partitioned between 15 mL of 1M aqueous HCl solution and 25 mL of ethyl acetate. The organic layer was separated, washed with 15 mL of brine, dried (magnesium sulfate) and concentrated in vacuo to give a solid. The crude material was purified by flash chromatography (Merck silica, 10 x 3.0 cm, 1:1 ethyl acetate/hexane) to afford 332 mg (0.62 mmol, 80%) of title oxazole amide as a white solid, mp 135-136°.

N. [1S-(1α,2α,3α,4α)]-[2-[[3-[4-[[(4-Cyclohexylbutyl)amino]carbonyl]-2-oxazolyl]-7-oxabicyclo[2.2.1]hept-2-yl]-methyl]phenoxy]acetic acid,

A mixture of 295 mg (0.55 mmol) of Part M ester and 46 mg (1.1 mmol, Aldrich) of lithium hydroxide monohydrate in 2.5 mL of 4:1 THF/water was stirred at room temperature for 1.5 hours. The reaction mixture was acidified with 2.2 mL of 1M aqueous HCl solution then partitioned between 20 mL of ethyl acetate and 20 mL of water. The organic layer was separated, dried (magnesium sulfate) and concentrated in vacuo to afford 281 mg (0.55 mmol, 100%) of title product as a white solid, mp 190-191°.
IR (KBr): 3416, 2921, 1741, 1644, 1602, 1520, 1492 $cm^1$.
270 MHz $^1H$ NMR ($CDCl_3$):
0.70-1.90 (m, 22H)
2.18 (dd, 1H)
2.37 (dd, J=12,12,1H)
2.70 (m, 1H)
3.40 (m, 3H)
4.53 (d, J=15, 1H)
4.67 (d, J=15, 1H)
5.04 (d, 1H)
6.76 (d, J=8, 1H)
6.93 (dd, J=7,7, 1H)
7.15 (m, 3H)
8.17 (s, 1H)
67.5 MHZ $^{13}C$ NMR ($CDCl_3$):
170.4, 164.2, 161.1, 155.4, 141.2, 135.7, 131.0, 128.8, 127.7, 121.7, 111.3, 80.0, 78.9, 65.1, 49.2, 46.9, 39.3, 37.5, 37.1, 33.3, 30.7, 29.9, 29.8, 28.7, 26.7, 26.4, 24.2. MS(CI): 511 $(M+H)^+$
OR: $[\alpha]_D = +41.8°$ (c=1.0 in chloroform)
TLC: $R_f$ (silica gel, 1:10:90, acetic acid/methanol/methylene chloride)=0.46, ammonium molybdate/ceric sulfate and UV, homogeneous.

| Analysis Calc'd for $C_{29}H_{38}N_2O_6$: | | | |
|---|---|---|---|
| | C, 68.21; | H, 7.50; | N, 5.49 |
| Found: | C, 68.35; | H, 7.81; | N, 5.42 |

Example 10

[1S-(1α,2α,3α,4α)]-2-[[3-[4-[[(7,7-Dimethyloctyl)amino]carbonyl]-2-oxazolyl]-7-oxabicyclo[2.2.1]hept-2-yl]-methyl]benzenepropanoic acid

A. 7,7-Dimethyl-1-octanamine

(1) 3,3-Dimethylbutanal

To a solution of 9.4 mL (107 mmol) of oxalyl chloride in 500 mL of dry $CH_2Cl_2$ at -60° was added a solution of 15.4 mL (18.5 g, 235 mmol) of dimethyl sulfoxide in 25 mL of $CH_2Cl_2$ dropwise over 10 minutes. The reaction mixture was stirred for 10 minutes then 10 g (98 mmol, Aldrich) of 3,3-dimethyl-1-butanol was added slowly. Stirring was continued for an additional 20 minutes then 68.1 mL (489 mmol) of triethylamine was added, and the reaction mixture was allowed to warm to room temperature. Water (50 mL) was then added, the organic layer was separated and the aqueous layer was extracted with $CH_2Cl_2$ (30 mL). The organic layers were combined, washed sequentially with 1% aqueous HCl, water, aqueous saturated $NaHCO_3$, water and brine, dried (magnesium sulfate) and concentrated in vacuo to obtain 3.3 g (33%) of title compound as a volatile yellow oil.

(2) (3-Carboxypropyl)triphenylphosphonium bromide

A mixture of 100 g (599 mmol, Aldrich) of 4-bromobutyric acid and 157 g (599 mmol, Aldrich) of triphenylphosphine was heated to 130° for 2 hours. The reaction was cooled to room temperature. The resulting solid was solubilized in 250 mL of hot chloroform then diluted with 200 mL of diethyl ether. The mixture was cooled to room temperature then 0°. The solid which formed was collected by filtration and then dried under vacuum to afford 240 g (559 mmol, 93%) of title compound.

(3) 7,7-Dimethyl-4-octenoic acid

To a stirred solution of 13.7 g (31.9 mmol) of Part A(2) phosphonium bromide in 60 mL of dry THF under argon at -15° was added dropwise 32 mL (1.72 M in toluene, 57.9 mmol, Callery Chem) of potassium t-amylate solution over 10 minutes. The mixture was stirred for 0.5 hour then to the resulting orange reaction mixture was added slowly a solution of 2.00 g (19.9 mmol) of Part A(1) aldehyde in 5 mL of THF. The reaction mixture was stirred at -15°C for 1 hour then at room temperature for 20 hours and quenched with 12 mL of glacial acetic acid. The resulting solution was concentrated in vacuo and the residue was partitioned between ethyl acetate (100 mL) and saturated $NaHCO_3$ (100 mL). The organic layer was separated and the aqueous layer was extracted twice with ethyl acetate (100 mL). The combined organic layers were washed sequentially with 1% aqueous HCl, water, saturated aqueous $NaHCO_3$, water, and brine, then dried (magnesium sulfate), and concentrated in vacuo. The residue was purified by flash chromatography (Merck silica, gradient: 1% to 100% ethyl acetate in hexane, with 0.3% glacial acetic acid) to obtain 1.75 g (51%) of title compound.

(4) 7,7-Dimethyloctanoic acid

To a stirred solution of 1.2 g (7.0 mmol) of Part A(3) compound in 8 mL of glacial acetic acid was added 0.2 g of platinum oxide catalyst. This mixture was stirred for 14 hours under 1 atmosphere of hydrogen (balloon). The reaction mixture was filtered through a Celite pad and the filtrate was concentrated in vacuo. The residue was diluted with 50 mL toluene and concentrated again. This process was repeated to obtain 1.2 g (100%) of title compound as oil.

(5) 7,7-Dimethyloctanamide

To a stirred solution of 1.21 g (7.02 mmol) of Part A(4) compound in 50 mL of toluene was added 3 mL (134 mmol) of oxalyl chloride. The reaction mixture was stirred for 1 hour at room temperature under argon and then concentrated in vacuo. The residue was diluted with 20 mL of toluene and concentrated again. This was repeated to remove traces of oxalyl chloride. The residue of the crude acid chloride was stirred in 5 mL of methanol and then 1.17 mL (8.43 mmol) of triethylamine and 2 mL (9M, 18 mmol) of methanolic ammonia were added at room temperature under argon. After stirring for 16 hours, the reaction mixture was

partitioned between 3 mL water and 20 mL ethyl acetate. The organic layer was separated and the aqueous layer was extracted twice with ethyl acetate (20 mL). The combined organic layers were washed with brine then dried (magnesium sulfate) and concentrated in vacuo to obtain a semi-solid. The semi-solid was crystallized by trituration with hexane to obtain 0.5 g (42%) of title compound as a solid.

(6) 7,7-Dimethyl-1-octanamine

To a solution of 0.45 g (2.62 mmol) of Part A(5) compound in 50 mL of dry diethyl ether stirred under argon at 0 °C was added 0.11 g (2.9 mmol) of lithium aluminum hydride. Gas was evolved. The reaction mixture was stirred at room temperature for 4 days. While stirring vigorously, the reaction was cautiously quenched by sequential addition of 0.02 mL of $H_2O$, 0.02 mL of 15% aqueous NaOH, 0.072 mL of $H_2O$, and 1 mL of diethyl ether. A white precipitate formed. After stirring for 0.5 hours the mixture was filtered and the filtrate was concentrated in vacuo to obtain 0.4 g (89%) of a yellow oil. This was crystallized by trituration with hexane and $CHCl_3$ to give title amine.

B. [1S-(1α,2α,3α,4α)]-2-[[3-[4-[[(7,7-Dimethyloctyl)amino]carbonyl]-2-oxazolyl]-7-oxabicyclo[2.2.1]hept-2-yl]-methyl]benzenepropanoic acid, methyl ester

To a solution of 140 mg (0.36 mmol), of [1S-(1α,2α,3α,4α)]-2-[[3-[4-(carboxy)-2-oxazolyl]-7-oxabicyclo-[2.2.1]hept-2-yl]methyl]benzenepropanoic acid, prepared as described in Example 2, part J, first paragraph, in 4 mL dry $CH_2Cl_2$ (distilled from $P_2O_5$) was added 1 small drop of dimethylformamide, followed by 40 μL (0.46 mmol, Aldrich) of oxalyl chloride. The reaction was stirred until gas evolution ceased (about 20 minutes), then the mixture was concentrated in vacuo to give the crude acid chloride as a pale yellow solid.

To a solution of crude acid chloride in 3 mL dry $CH_2Cl_2$ (distilled from $P_2O_5$), cooled to 0 °C, was added 63 mg (80%) (equivalent to 51 mg pure amine, 0.32 mmol) of Part A amine, then a solution of 65 μL (0.46 mmol, distilled from $CaH_2$) triethylamine in 1 mL dry $CH_2Cl_2$ was added to the reaction mixture. After 30 minutes the reaction mixture was partitioned between 15 mL $CH_2Cl_2$ and 15 mL $H_2O$; the organic layer was separated and the aqueous layer was extracted with an additional 15 mL $CH_2Cl_2$. The combined organic layers were dried ($MgSO_4$) and concentrated in vacuo to give a crude solid. The crude solid was flash chromatographed (Merck Silica, 12 x 2.5 cm, 1:1 ethyl acetate:hexane) to give 135 mg (0.26 mmol, 81%) of title amine as a white solid.

C. [1S-(1α,2α,3α,4α)]-2-[[3-[4-[[(7,7-Dimethyloctyl)amino]carbonyl]-2-oxazolyl]-7-oxabicyclo[2.2.1]hept-2-yl]-methyl]benzenepropanoic acid

To a mixture of 134 mg (0.26 mmol) of Part B amide in 4 mL THF/1 mL $H_2O$ was added 22 mg (0.51 mmol, Aldrich) of lithium hydroxide monohydrate. The reaction was stirred at room temperature for 2.5 hours, then quenched by the addition of 1 mL 1M HCl. The mixture was partitioned between 20 mL $H_2O$ and 20 mL ethyl acetate; the organic layer was separated, dried ($MgSO_4$) and concentrated in vacuo to give a crude white solid. The crude solid was recrystallized (hot ethyl acetate/hexane) to give 100 mg (0.20 mmol, 77%) of title acid as a white solid, mp 151-153 °C.
IR (KBr): 3409, 2950, 2932, 1726, 1649, 1603, 1520 $cm^{-1}$
270 MHz $^1$H NMR ($CDCl_3$) δ:
8.15 (s, 1H)
7.27 (s, 1H)
7.10 (m, 4H)
4.98 (d, 1H)
4.39 (d, 1H)
3.38 (m, 3H)
2.90 (t, 2H)
2.55 (t, 3H)
2.34 (t, 1H)
2.20 (dd, 1H)
1.90-1.00 (m, 14H)
0.81 (s, 9H)
67.5 MHz $^{13}$C NMR ($CDCl_3$) δ: 176.2, 163.9, 160.8, 141.0, 138.5, 137.8, 136.0, 129.7, 129.0, 126.7, 126.5, 79.7, 78.7, 50.0, 47.0, 44.2, 39.2, 34.7, 32.5, 30.3, 29.9, 29.6, 29.4, 28.9, 27.4, 27.0, 24.5
MS (CL): 511 (M + H) +

TLC: $R_f$ (silica gel, 1:9 methanol/methylene chloride) = 0.20, ammonium molybdate/ceric sulfate and UV, homogeneous.

| Analysis Calc'd for $C_{30}H_{42}N_2O_5$: | | | |
|---|---|---|---|
| | C, 70.56; | H, 8.29; | N, 5.50 |
| Found: | C, 70.44; | H, 8.42; | N, 5.50 |

Examples of additional compounds in accordance with the present invention which may be prepared following the procedures outlined in the specification and working Examples include, but are not limited to the following:

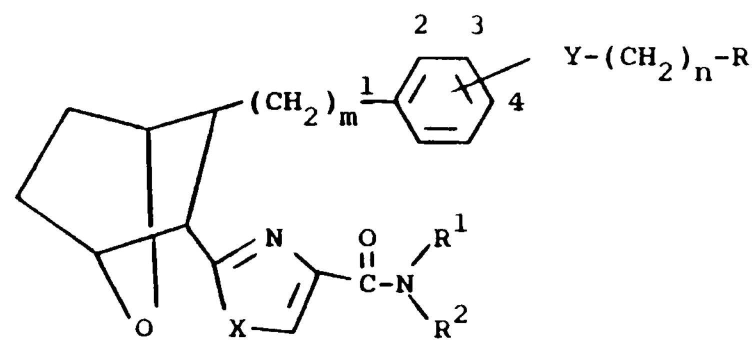

| Ex. No. | m | n | X | Y (position) | $R^2$ | R | $R^1$ | m.p. °C |
|---|---|---|---|---|---|---|---|---|
| 11. | 1 | 2 | 0 | -(2) | H | COOH | 1-piperidyl-4-butyl | |
| 12. | 1 | 2 | 0 | -(2) | H | COOH | 4-OH-phenylbutyl | d. 212 |
| 13. | 1 | 2 | 0 | -(2) | H | COOH | propyl | 165-168 |
| 14. | 1 | 2 | 0 | -(2) | H | COOH | pentyl | 136-139 |
| 15. | 1 | 2 | 0 | -(2) | H | COOH | cyclohexylethyl | 158-161 |
| 16. | 1 | 2 | 0 | -(2) | H | COOH | t-butylbutyl | 147-149 |
| 17. | 1 | 2 | 0 | -(2) | H | COOH | 4-methoxyphenylbutyl | 125-127 |
| 18. | 1 | 2 | 0 | -(2) | H | COOH | 4-cyclohexylbuten-2-yl | 167-169 |
| 19. | 1 | 2 | 0 | -(2) | H | COOH | 5-cyclohexylpenten-4-yl | 145-147 |
| 20. | 1 | 2 | 0 | -(2) | H | COOH | heptyl | 134-137 |
| 21. | 1 | 2 | 0 | -(2) | H | COOH | cyclohexyl | 58-62 |
| 22. | 1 | 2 | 0 | -(2) | H | COOH | isopropyl | 57-63 |
| 23. | 1 | 2 | 0 | -(2) | H | COOH | cyclohexyloctyl | 148-149 |

| Ex. No. | m | n | X | Y (position) | $R^2$ | R | $R^1$ | m.p. °C |
|---|---|---|---|---|---|---|---|---|
| 24. | 1 | 2 | 0 | -(2) | H | COOH | 4-methylsulfonylphenylbutyl | 159-161 |
| 25. | 1 | 2 | 0 | -(2) | H | COOH | 4-methylmercaptophenylbutyl | 155-158 |
| 26. | 1 | 2 | 0 | -(2) | H | COOH | phenyl | 170-172 |
| 27. | 1 | 2 | 0 | -(2) | H | COOH | p-biphenyl | 204-206 |
| 28. | 1 | 2 | 0 | -(2) | H | COOH | 4-phenylbutyl | 148-150 |
| 29. | 1 | 2 | 0 | -(2) | H | COOH | 4-benzyloxyphenyl | 192-193 |
| 30. | 1 | 2 | 0 | -(2) | H | COOH | 4-hydroxyphenyl | 185-186 |
| 31. | 1 | 2 | 0 | -(2) | H | COOH | 3-iodo-propen-2-yl | 142-145 |
| 32. | 1 | 2 | 0 | -(2) | H | COOH | 4-(m-OH, p-iodophenyl)butyl | 84-91 |
| 33. | 1 | 2 | 0 | -(2) | H | COOH | 4-cyclohexylbutyl | 274-275 (Na salt) |
| 34. | 1 | 2 | 0 | -(2) | H | COOH | p-methoxyphenyl | foam |
| 35. | 1 | 2 | 0 | -(2) | H | COOH | p-chlorophenyl | 220-221 |
| 36. | 1 | 2 | 0 | -(2) | H | COOH | 1-imidazolylpropyl | 215-216 |
| 37. | 1 | 2 | 0 | -(2) | H | COOH | p-chlorophenethyl | 187-188 |
| 38. | 1 | 2 | 0 | -(2) | H | COOH | t-butyl | 155-156 |
| 39. | 1 | 2 | 0 | -(2) | H | COOH | 1,1,dimethylpropyl | 128-132 |
| 40. | 1 | 2 | 0 | -(2) | H | COOH | n-$C_{18}H_{37}$ | 135-136 |
| 41. | 1 | 2 | 0 | -(2) | H | COOH | 5-(N-cyclohexyl)pentamido | 168-169 |
| 42. | 1 | 2 | 0 | -(2) | H | COOH | 5-hydroxy-5-methyl hexyl | 149-150 |
| 43. | 1 | 2 | 0 | -(2) | H | COOH | 5-carboxy-5-methyl hexyl | 123-125 |
| 44. | 1 | 2 | 0 | -(2) | H | COOH | H | >200 |

| Ex. No. | m | n | X | Y (position) | $R^2$ | R | $R^1$ | m.p.°C |
|---|---|---|---|---|---|---|---|---|
| 45. | 1 | 2 | 0 | -(2) | H | COOH | p-F-phenylbutyl | 141-143 |
| 46. | 1 | 2 | 0 | -(2) | H | COOH | $C_{10}C_{21}$ | 145-147 |
| 47. | 1 | 2 | 0 | -(2) | H | COOH | 2,2-dimethylbutyl | --- |
| 48. | 1 | 2 | 0 | -(2) | H | COOH | 2,2-dimethylpropyl | --- |
| 49. | 1 | 2 | 0 | -(2) | H | COOH | 3,3-dimethylbutyl | 160-162 |
| 50. | 1 | 2 | 0 | -(2) | H | COOH | 2-(4-fluorophenyl)ethyl | 187-189 |
| 51. | 1 | 2 | 0 | -(2) | H | COOH | 2-phenyethyl | 181-183 |
| 52. | 1 | 2 | 0 | -(2) | H | $-CONHC_2H_5$ | cyclohexylbutyl | 155-156 |
| 53. | 1 | 2 | 0 | -(2) | H | $-CONH_2$ | cyclohexybutyl | 174-175 |
| 54. | 1 | 2 | 0 | -(2) | H | $-CO_2C_2H_5$ | cyclohexybutyl | 130-131 |
| 55. | 1 | 2 | 0 | -(2) | H | $-CH_2OH$ | cyclohexybutyl | 119-121 |
| 56. | 1 | 2 | 0 | -(2) | $CH_3$ | -COOH | pentyl | oil |
| 57. | 1 | 2 | 0 | -(2) | H | $-CONHSO_2CH_3$ | cyclohexylbutyl | 154-156 |
| 58. | 1 | 2 | 0 | -(2) | $CH_3$ | -COOH | cyclohexylbutyl | 60-65 |
| 59. | 2 | 1 | 0 | -(2) | H | COOH | cyclohexylbutyl | 58-63 |
| 60. | 2 | 1 | 0 | -(3) | H | COOH | cyclohexylbutyl | 50-58 |
| 61. | 2 | 2 | 0 | -(2) | H | COOH | cyclohexylbutyl | oil |
| 62. | 1 | 0 | 0 | -(2) | H | COOH | cyclohexylbutyl | 139-141 |
| 63. | 1 | 1 | 0 | -(2) | H | COOH | cyclohexylbutyl | 130-132 |
| 64. | 2 | 0 | 0 | -(3) | H | COOH | cyclohexylbutyl | 160-162 |
| 65. | 1 | 2 | N | -(2) | H | COOH | cyclohexylbutyl | 229-232 |
| 66. | 1 | 2 | 0 | vinyl(2) | H | COOH | cyclohexylbutyl | 210-211 |
| 67. | 1 | 2 | 0 | -(2) | H | COOH | 6-heptynyl | --- |

## Claims

**Claims for the following Contracting States : AT, BE, DK, FR, DE, GB, NL, IT, LU, SE, CH, LI**

**1.** A compound having the formula

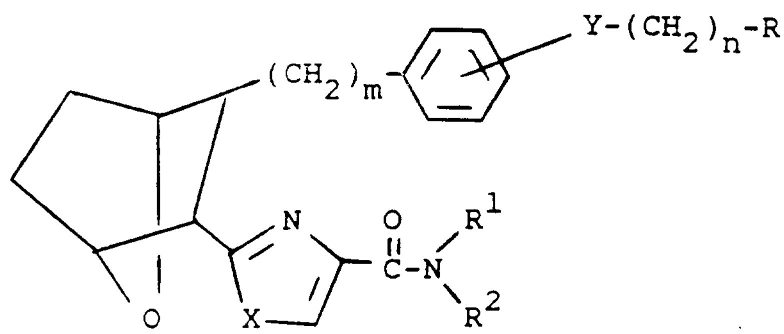

including all stereoisomers thereof, wherein

m is 1, 2 or 3;

n is 0, 1, 2, 3 or 4;

Y is O, vinyl or a single bond, with the proviso that when n is 0, Y is a single bond;

R is $CO_2H$, $CO_2$alkali metal, $CO_2$ alkyl, $CH_2OH$, $CONHSO_2R^3$, $CONHR^{3a}$ or 5-tetrazolyl, with the proviso that when R is 5-tetrazolyl, n is other than 0;

X is O, S or NH;

$R^1$ is hydrogen, alkyl, alkenyl, alkynyl, aryl, aralkyl, cycloalkyl, cycloalkylalkyl, saturated heterocycle, saturated heterocyclicalkyl, aromatic heterocycle, aromatic heterocyclicalkyl or amido which is optionally substituted with an alkyl, aryl, cycloalkyl, or cycloalkylalkyl; and

$R^2$ is hydrogen, alkyl, aryl, or aralkyl, or $R^1$ and $R^2$ together with the N to which they are linked form a 5- to 8- membered ring;

$R^3$ is alkyl, aryl or aralkyl and $R^{3a}$ is hydrogen, alkyl, aryl or aralkyl;

and wherein:

"alkyl" means a $C_1$ to $C_{12}$ alkyl group which is optionally substituted by 1, 2 or 3 halo substituents, an aryl substituent, an alkyl-aryl substituent, a haloaryl substituent, a cycloalkyl substituent or an alkylcycloalkyl substituent;

"cycloalkyl" means a $C_3$ to $_{12}$ cycloalkyl group which is optionally substituted by halogen, alkyl and/or alkoxy;

"aryl" means phenyl or naphthyl, each of which optionally has 1 or 2 substituents selected from alkyl, trifluoromethyl, halogen (Cl, Br or F), alkoxy, alkylthio, alkylsulfinyl, and alkylsulfonyl;

"aralkyl" means "alkyl" as defined above but having an "aryl" substituent as defined above;

"alkoxy" means "alkyl" linked to an oxygen atom;

"saturated heterocycle" means a 5-, 6- or 7-membered saturated ring which includes 1 or 2 hetero atoms selected from nitrogen, oxygen and sulfur and which is optionally substituted by "alkyl", "aryl", "cycloalkyl" or "cycloalkylalkyl";

"aromatic heterocycle" means a 5- or 6-membered aromatic ring which includes 1 or 2 hetero atoms selected from nitrogen, oxygen and sulfur and which is optionally substituted by "alkyl", "aryl", "cycloalkyl" or "cycloalkylalkyl".

2. The compound as defined in Claim 1 having the formula

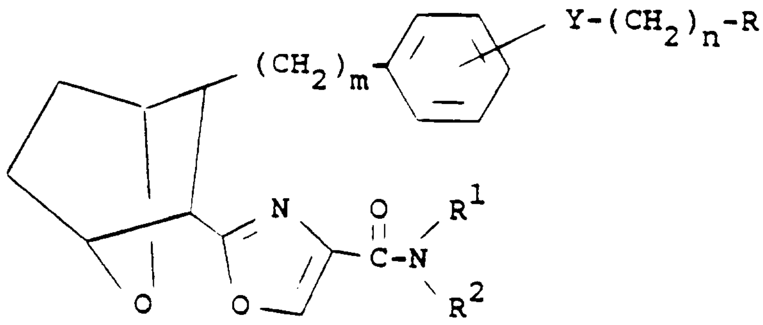

3. The compound as defined in Claim 1 where m = 1 and n = 2.

4. The compound as defined in Claim 1 having the formula

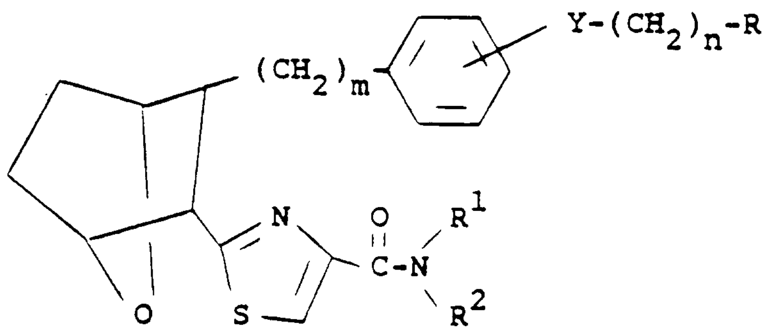

5. The compound as defined in Claim 1 having the formula

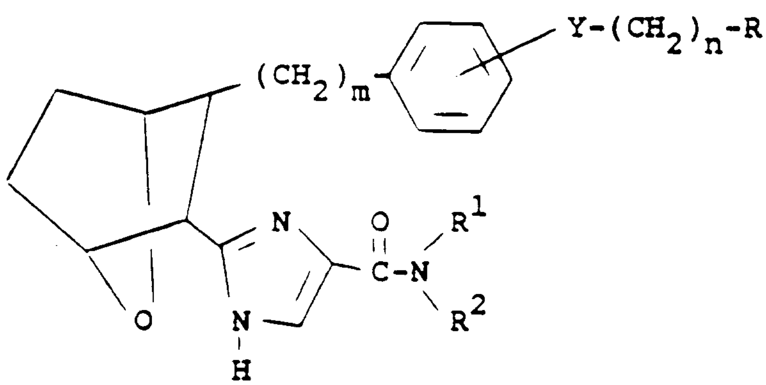

6. The compound as defined in any preceding claim wherein R is $CO_2H$, $CONHSO_2R^3$ or 5-tetrazolyl.

7. The compound as defined in Claim 2 having the formula

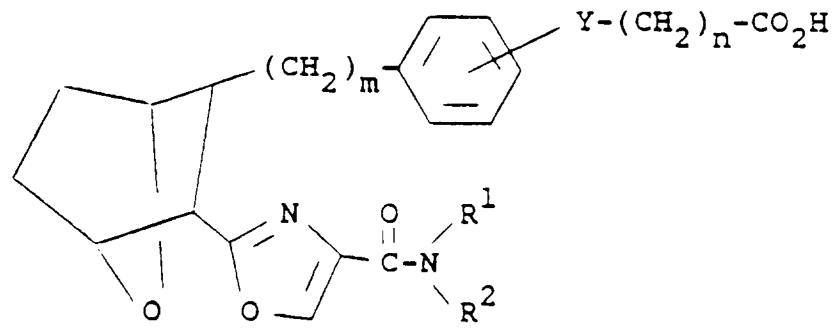

8. The compound as defined in Claim 1 having the name [1S-(1α,2α,3α,4α)]-2-[[3-[4-[[(4-cyclohexylbutyl)-amino]carbonyl]-2-oxazolyl]-7-oxabicyclo[2.2.1]hept-2-yl]methyl]benzenepropanoic acid, or an ester or salt thereof.

9. The compound as defined in Claim 1 having the name [1S-(1α,2α,3α,4α)]-2-[[3-[4-[[[4-chlorophenyl)-butyl]amino]carbonyl]-2-oxazolyl]-7-oxabicyclo[2.2.1]hept-2-yl]methyl]benzenepropanoic acid or an ester or salt thereof.

10. The compound as defined in Claim 1 having the name [1S-(1α,2α,3α,4α)]-3-[[3-[4-[[(4-cyclohexylbutyl)-amino]carbonyl]-2-oxazolyl]-7-oxabicyclo[2.2.1]hept-2-yl]methyl]benzeneacetic acid, or an ester or salt thereof.

11. The compound as defined in Claim 1 having the name [1S-(1α,2α,3α,4α)]-[2-[[3-[4-[[(4-cyclohexylbutyl)-amino]carbonyl]-2-oxazolyl]-7-oxabicyclo[2.2.1]hept-2-yl]methyl]phenoxy]acetic acid, or an ester or salt thereof.

12. The compound as defined in Claim 1 having the name [1S-(1α,2α,3α,4α)]-2-[[3-[4-[[(7,7-dimethyloctyl) amino]carbonyl]-2-oxazolyl]-7-oxabicyclo[2.2.1]hept-2-yl]methyl]benzenepropanoic acid, or an ester or salt thereof.

13. The compound as defined in Claim 1 wherein m = 1, n = 2, X is O, Y is a single bond at the 2-position, $R^2$ is H, R is COOH and $R^1$ is pentyl.

14. The compound as defined in Claim 1 wherein m = 1, n = 2, X is O, Y is a single bond at the 2-position, $R^2$ is H, R is COOH and $R^1$ is p-chlorophenethyl.

15. The compound of the formula given in Claim 1 wherein m = 1, n = 2, X is O, Y is a single bond at the 2-position, $R^2$ is H, R is COOH and $R^1$ is 4-cyclohexylbuten-2-yl.

16. The compound of the formula given in Claim 1 wherein m = 1, n = 2, X is O, Y is a single bond at the 2-position, $R^2$ is H, R is COOH and $R^1$ is 5-cyclohexylpenten-4-yl.

17. The compound of the formula given in Claim 1 wherein m = 1, n = 2, X is O, Y is a single bond at the 2-position, $R^2$ is H, R is COOH and $R^1$ is 4-benzyloxyphenyl.

18. The compound of the formula given in Claim 1 wherein m = 1, n = 2, X is O, Y is a single bond at the 2-position, $R^2$ is H, R is COOH and $R^1$ is 3-iodo-propen-2-yl.

19. A composition for inhibiting platelet aggregation and bronchoconstriction comprising an effective amount of a compound as defined in any preceding claim and a pharmaceutically acceptable carrier therefor.

**20.** Use of a compound as defined in any one of Claims 1-18 for the manufacture of a medicament for inhibiting platelet aggregation and bronchoconstriction, by administration to the circulatory system of a mammalian host.

**21.** The use as defined in Claim 20 wherein said compound is in a form suitable to be administered in an amount within the range of from 0.1 to 100 mg/kg.

**22.** Use of a compound as defined in any one of Claims 1-18 for the manufacture of a medicament for inhibiting platelet aggregation.

**23.** Use of a compound as defined in any one of Claims 1-18 for the manufacture of a medicament for inhibiting bronchoconstriction associated with asthma.

**24.** Use of a compound as defined in any one of Claims 1-18 for the manufacture of a medicament for improving post-ischemic myocardial dysfunction.

**25.** Use of a compound as defined in any one of Claims 1-18 for the manufacture of a medicament for treating toxemia during pregnancy.

**26.** Use of a compound as defined in any one of Claims 1-18 for the manufacture of a medicament for preventing or reducing venous thrombosis.

**27.** Use of a compound as defined in any one of Claims 1-18 for the manufacture of a medicament for preventing or reducing platelet loss during extracorporeal circulation.

**28.** Use of a compound as defined in any one of Claims 1-18 for the manufacture of a medicament for treating burn injuries and/or promoting wound healing, in systemic or topical form.

**29.** Use of a compound as defined in any one of Claims 1-18 for the manufacture of a medicament for reducing post-ischemic myocardial injury within 6 hours of a myocardial infarction while an effective amount of a thrombolytic agent is also administered.

**30.** The use as defined in Claim 29 wherein said thrombolytic is t-PA, streptokinase, urokinase, prourokinase or anisoylated plasminogen-streptokinase activator complex.

**31.** A process for preparing a compound having the formula

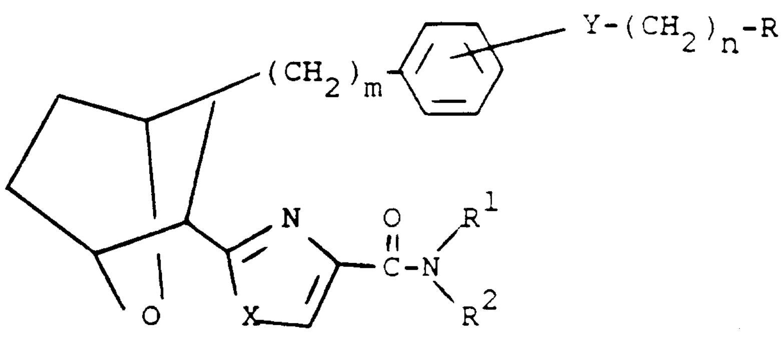

including all stereoisomers thereof, wherein

m is 1, 2 or 3;

n is 0, 1, 2, 3 or 4;

Y is O, vinyl or a single bond, with the proviso that when n is 0, Y is a single bond;

R is $CO_2H$, $CO_2$alkali metal, $CO_2$ alkyl, $CH_2OH$, $CONHSO_2R^3$, $CONHR^{3a}$ or 5-tetrazolyl, with the proviso that when R is 5-tetrazolyl, n is other than 0;

X is O, S or NH;

$R^1$ is hydrogen, alkyl, alkenyl, alkynyl, aryl, aralkyl, cycloalkyl, cycloalkylalkyl, saturated heterocycle, saturated heterocyclicalkyl, aromatic heterocycle, aromatic heterocyclicalkyl or amido which is optionally substituted with an alkyl, aryl, cycloalkyl, or cycloalkylalkyl; and

$R^2$ is hydrogen, alkyl, aryl, or aralkyl, or $R^1$ and $R^2$ together with the N to which they are linked form a 5- to 8- membered ring;

$R^3$ is alkyl, aryl or aralkyl and $R^{3a}$ is hydrogen, alkyl, aroyl or aralkyl,

and wherein:

"alkyl" means a $C_1$ to $C_{12}$ alkyl group which is optionally substituted by 1, 2 or 3 halo substituents, an aryl substituent, an alkyl-aryl substituent, a haloaryl substituent, a cycloalkyl substituent or an alkylcycloalkyl substituent;

"cycloalkyl" means a $C_3$ to $_{12}$ cycloalkyl group which is optionally substituted by halogen, alkyl and/or alkoxy;

"aryl" means phenyl or naphthyl, each of which optionally had 1 or 2 substituents selected from alkyl, trifluoromethyl, halogen (Cl, Br or F), alkoxy, alkylthio, alkylsulfinyl, and alkylsulfonyl;

"aralkyl" means "alkyl" as defined above but having an "aryl" substituent as defined above;

"alkoxy" means "alkyl" linked to an oxygen atom;

"saturated heterocycle" means a 5-, 6- or 7-membered saturated ring which includes 1 or 2 hetero atoms selected from nitrogen, oxygen and sulfur and which is optionally substituted by "alkyl", "aryl", "cycloalkyl" or "cycloalkylalkyl";

"aromatic heterocycle" means a 5- or 6-membered aromatic ring which includes 1 or 2 hetero atoms selected from nitrogen, oxygen and sulfur and which is optionally substituted by "alkyl", "aryl", "cycloalkyl" or "cycloalkylalkyl";

characterized by converting a compound of the formula

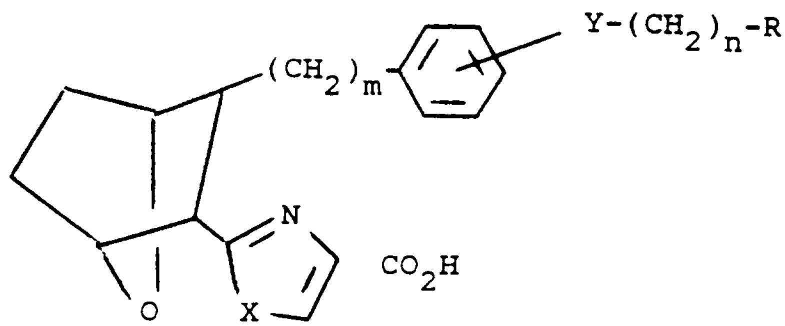

where R is other than $CO_2H$ or its alkali metal salt, to its acid halide form and then reacting said acid halide with an amine of the formula $HNR_1R_2$ according to conventional procedures to form products where R is other than $CO_2H$ or its alkali metal salt and hydrolyzing a product wherein R is $CO_2$ alkyl to form a product where R is $CO_2H$ and forming an alkali metal salt thereof according to conventional procedures.

**Claims for the following Contracting States : ES, GR**

**1.** A process for preparing a compound having the formula

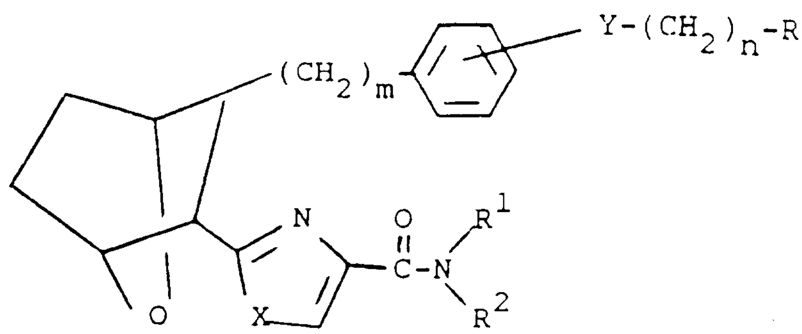

including all stereoisomers thereof, wherein

m is 1, 2 or 3;

n is 0, 1, 2, 3 or 4;

Y is O, vinyl or a single bond, with the proviso that when n is 0, Y is a single bond;

R is $CO_2H$, $CO_2$alkali metal, $CO_2$ alkyl, $CH_2OH$, $CONHSO_2R^3$, $CONHR^{3a}$ or 5-tetrazolyl, with the proviso that when R is 5-tetrazolyl, n is other than 0;

X is O, S or NH;

$R^1$ is hydrogen, alkyl, alkenyl, alkynyl, aryl, aralkyl, cycloalkyl, cycloalkylalkyl, saturated heterocycle, saturated heterocyclicalkyl, aromatic heterocycle, aromatic heterocyclicalkyl or amido which is optionally substituted with an alkyl, aryl, cycloalkyl, or cycloalkylalkyl; and

$R^2$ is hydrogen, alkyl, aryl, or aralkyl, or $R^1$ and $R^2$ together with the N to which they are linked form a 5- to 8- membered ring;

$R^3$ is alkyl, aryl or aralkyl and $R^{3a}$ is hydrogen, alkyl, aryl or aralkyl;

and wherein:

"alkyl" means a $C_1$ to $C_{12}$ alkyl group which is optionally substituted by 1, 2 or 3 halo substituents, an aryl substituent, an alkyl-aryl substituent, a haloaryl substituent, a cycloalkyl substituent or an alkylcycloalkyl substituent;

"cycloalkyl" means a $C_3$ to $_{12}$ cycloalkyl group which is optionally substituted by halogen, alkyl and/or alkoxy;

"aryl" means phenyl or naphthyl, each of which optionally has 1 or 2 substituents selected from alkyl, trifluoromethyl, halogen (Cl, Br or F), alkoxy, alkylthio, alkylsulfinyl, and alkylsulfonyl;

"aralkyl" means "alkyl" as defined above but having an "aryl" substituent as defined above;

"alkoxy" means "alkyl" linked to an oxygen atom;

"saturated heterocycle" means a 5-, 6- or 7-membered saturated ring which includes 1 or 2 hetero atoms selected from nitrogen, oxygen and sulfur and which is optionally substituted by "alkyl", "aryl", "cycloalkyl" or "cycloalkylalkyl";

"aromatic heterocycle" means a 5- or 6-membered aromatic ring which includes 1 or 2 hetero atoms selected from nitrogen, oxygen and sulfur and which is optionally substituted by "alkyl", "aryl", "cycloalkyl" or "cycloalkylalkyl";

characterized by converting a compound of the formula

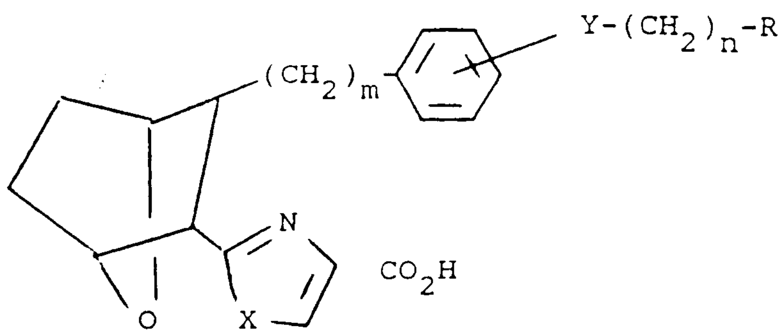

where R is other than $CO_2H$ or its alkali metal salt, to its acid halide form and then reacting said acid halide with an amine of the formula $HNR_1R_2$ according to conventional procedures to form products where R is other than $CO_2H$ or its alkali metal salt and hydrolyzing a product wherein R is $CO_2$ alkyl to form a product where R is $CO_2H$ and forming an alkali metal salt thereof according to conventional procedures.

2. The process of Claim 1 wherein the product has the formula

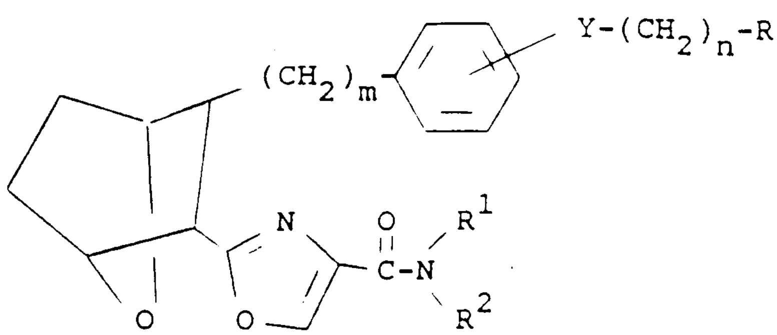

3. The process of Claim 1 where m = 1 and n = 2.

4. The process of Claim 1 wherein the product has the formula

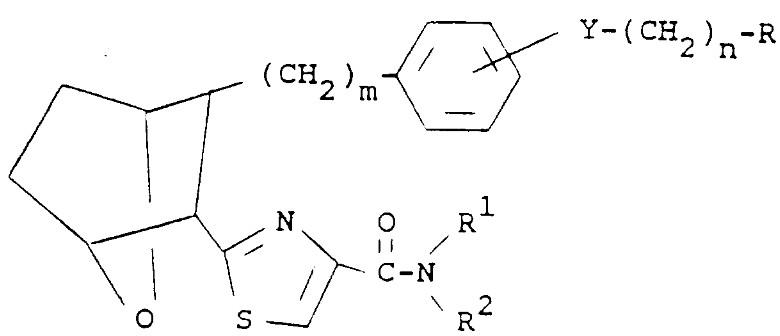

5. The process of Claim 1 wherein the product has the formula

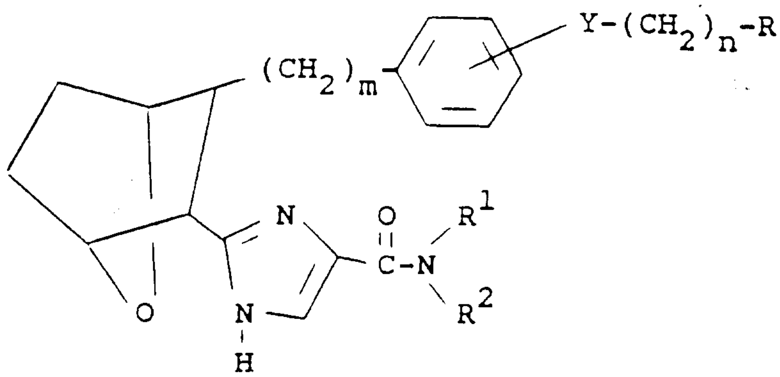

6. The process of Claim 1 wherein R is $CO_2H$, $CONHSO_2R^3$ or 5-tetrazolyl.

7. The process of Claim 1 wherein the product has the formula

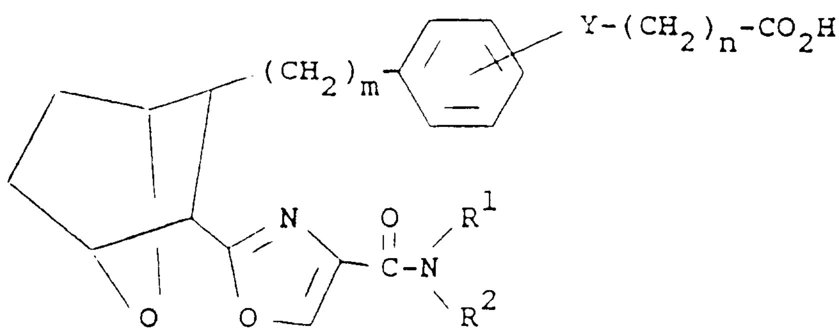

8. The process of Claim 1 wherein the product is [1S-(1α,2α,3α,4α)]-2-[[3-[4-[[(4-cyclohexylbutyl)amino]-carbonyl]-2-oxazolyl]-7-oxabicyclo[2.2.1]hept-2-yl]methyl]benzenepropanoic acid, or an ester or salt thereof.

9. The process of Claim 1 wherein the product is [1S-(1α,2α,3α,4α)]-2-[[3-[4-[[[4-chlorophenyl)butyl]-amino]carbonyl]-2-oxazolyl]-7-oxabicyclo[2.2.1]hept-2-yl]methyl]benzenepropanoic acid or an ester or salt thereof.

10. The process of Claim 1 wherein the product is [1S-(1α,2α,3α,4α)]-3-[[3-[4-[[(4-cyclohexylbutyl)amino]-carbonyl]-2-oxazolyl]-7-oxabicyclo[2.2.1]hept-2-yl]methyl]benzeneacetic acid, or an ester or salt thereof.

11. The process of Claim 1 wherein the product is [1S-(1α,2α,3α,4α)]-[2-[[3-[4-[[(4-cyclohexylbutyl)amino]-carbonyl]-2-oxazolyl]-7-oxabicyclo[2.2.1]hept-2-yl]methyl]phenoxy]acetic acid, or an ester or salt thereof.

12. The process of Claim 1 wherein the product is [1S-(1α,2α,3α,4α)]-2-[[3-[4-[[(7,7-dimethyloctyl)amino]-carbonyl]-2-oxazolyl]-7-oxabicyclo[2.2.1]hept-2-yl]methyl]benzenepropanoic acid, or an ester or salt thereof.

13. The process of Claim 1 wherein, in the product, m = 1, n = 2, X is O, Y is a single bond at the 2-position, $R^2$ is H, R is COOH and $R^1$ is pentyl.

14. The process of Claim 1 wherein, in the product, m = 1, n = 2, X is O, Y is a single bond at the 2-position, $R^2$ is H, R is COOH and $R^1$ is p-chlorophenethyl.

15. A process for preparing a compound of the first formula in Claim 1 wherein m = 1, n = 2, X is O, Y is a single bond at the 2-position, $R^2$ is H, R is COOH and $R^1$ is 4-cyclohexylbuten-2-yl, the process steps being as defined in Claim 1.

16. A process for preparing a compound of the first formula in Claim 1 wherein m = 1, n = 2, X is O, Y is a single bond at the 2-position, $R^2$ is H, R is COOH and $R^1$ is 5-cyclohexylpenten-4-yl, the process steps being as defined in Claim 1.

17. A process for preparing a compound of the first formula in Claim 1 wherein m = 1, n = 2, X is O, Y is a single bond at the 2-position, $R^2$ is H, R is COOH and $R^1$ is 4-benzyloxyphenyl, the process steps being as defined in Claim 1.

18. A process for preparing a compound of the first formula in Claim 1 wherein m = 1, n = 2, X is O, Y is a single bond at the 2-position, $R^2$ is H, R is COOH and $R^1$ is 3-iodo-propen-2-yl, the process steps being as defined in Claim 1.

**Patentansprüche**
**Patentansprüche für folgende Vertragsstaaten : AT, BE, DK, FR, DE, GB, NL, IT, LU, SE, CH, LI**

1. Verbindung der Formel

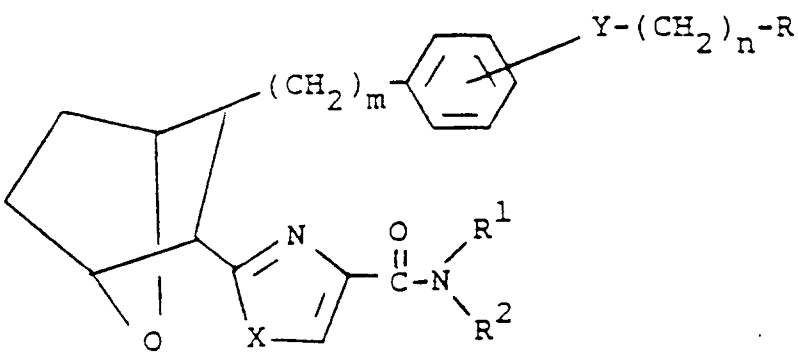

umfassend alle Stereoisomere davon, in der

m 1, 2 oder 3 ist;

n 0, 1, 2, 3 oder 4 ist;

Y ein Sauerstoffatom, eine Vinylgruppe oder eine Einfachbindung ist, mit der Maßgabe, daß Y eine Einfachbindung ist, wenn n 0 ist;

R eine $CO_2H$-Gruppe, eine Gruppe der Formel $CO_2$-Alkalimetall, ein Rest der Formel $CO_2$-Alkyl, eine $CH_2OH$-Gruppe, ein Rest der Formel $CONHSO_2R^3$, ein Rest der Formel $CONHR^{3a}$ oder eine 5-Tetrazolylgruppe ist, mit der Maßgabe, daß n nicht Null ist, wenn R eine 5-Tetrazolylgruppe ist;

X ein Sauerstoffatom, Schwefelatom oder eine NH-Gruppe ist;

$R^1$ ein Wasserstoffatom, ein Alkyl-, Alkenyl-, Alkinyl-, Aryl-, Aralkyl-, Cycloalkyl-, Cycloalkylalkylrest, ein gesättigter Heterocyclus, ein gesättigter heterocyclischer Alkylrest, ein aromatischer Heterocyclus, ein aromatischer heterocyclischer Alkylrest oder eine Amidgruppe ist, die gegebenenfalls mit einem Alkyl-, Aryl-, Cycloalkyl- oder Cycloalkylalkylrest substituiert ist; und

$R^2$ ein Wasserstoffatom, ein Alkyl-, Aryl- oder Aralkylrest ist, oder $R^1$ und $R^2$ zusammen mit dem Stickstoffatom, an das sie gebunden sind, einen 5- bis 8-gliedrigen Ring bilden;

$R^3$ ein Alkyl-, Aryl- oder Aralkylrest ist, und $R^{3a}$ ein Wasserstoffatom, ein Alkyl-, Aryl- oder Aralkylrest ist;

und wobei:

"Alkylrest" einen $C_1$- bis $C_{12}$-Alkylrest bedeutet, der gegebenenfalls mit 1, 2 oder 3 Halogensubstituenten, einem Arylsubstituenten, einem Alkylarylsubstituenten, einem Halogenarylsubstituenten, einem Cycloalkylsubstituenten oder einem Alkylcycloalkylsubstituenten substituiert ist;

"Cycloalkylrest" einen $C_3$- bis $C_{12}$-Cycloalkylrest bedeutet, der gegebenenfalls mit (einem) Halogenatom(en), (einem) Alkylrest(en) und/oder (einem) Alkoxyrest(en) substituiert ist;

"Arylrest" eine Phenyl- oder Naphthylgruppe bedeutet, von denen jede gegebenenfalls 1 oder 2 Substituenten aufweist, ausgewählt aus einem Alkylrest, einer Trifluormethylgruppe, einem Halogenatom (Cl, Br oder F), einem Alkoxy-, Alkylthio-, Alkylsulfinyl- und Alkylsulfonylrest;

"Aralkylrest" einen wie vorstehend definierten "Alkylrest" bedeutet, der jedoch einen wie vorstehend definierten "Aryl"substituenten aufweist;

"Alkoxyrest" einen "Alkylrest" bedeutet, der an ein Sauerstoffatom gebunden ist;

"gesättigter Heterocyclus" einen 5-, 6- oder 7-gliedrigen gesättigten Ring bedeutet, der 1 oder 2 Heteroatome umfaßt, ausgewählt aus einem Stickstoff-, Sauerstoff- und Schwefelatom, und der gegebenenfalls mit (einem) "Alkyl-", "Aryl-", "Cycloalkyl-" oder "Cycloalkylalkylrest(en)" substituiert ist;

"aromatischer Heterocyclus" einen 5- oder 6-gliedrigen aromatischen Ring bedeutet, der 1 oder 2 Heteroatome umfaßt, ausgewählt aus einem Stickstoff-, Sauerstoff- und Schwefelatom, und der gegebenenfalls mit (einem) "Alkyl-", "Aryl-", "Cycloalkyl-" oder "Cycloalkylalkylrest(en)" substituiert ist;

**2.** Verbindung nach Anspruch 1 der Formel

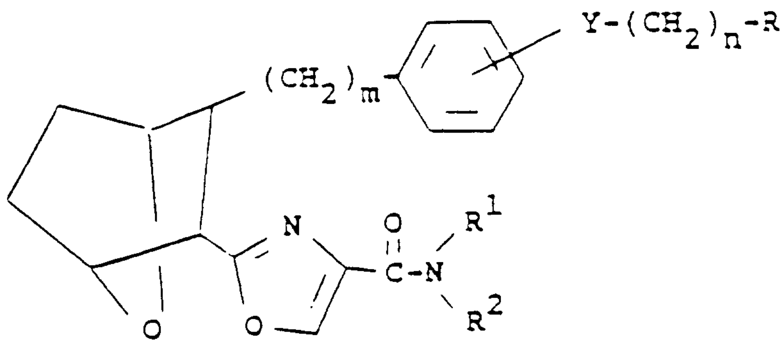

**3.** Verbindung nach Anspruch 1, wobei m = 1 und n = 2 ist.

**4.** Verbindung nach Anspruch 1 der Formel

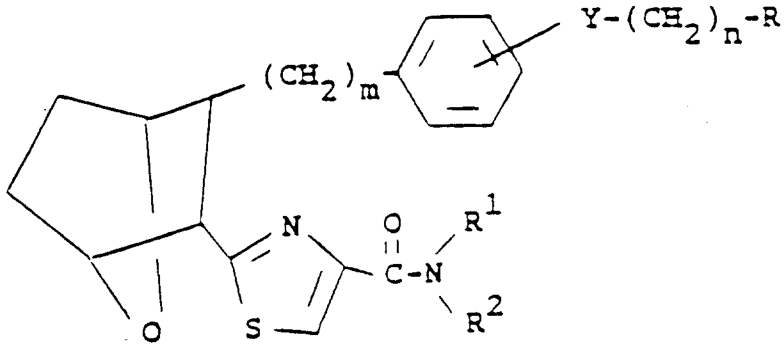

**5.** Verbindung nach Anspruch 1 der Formel

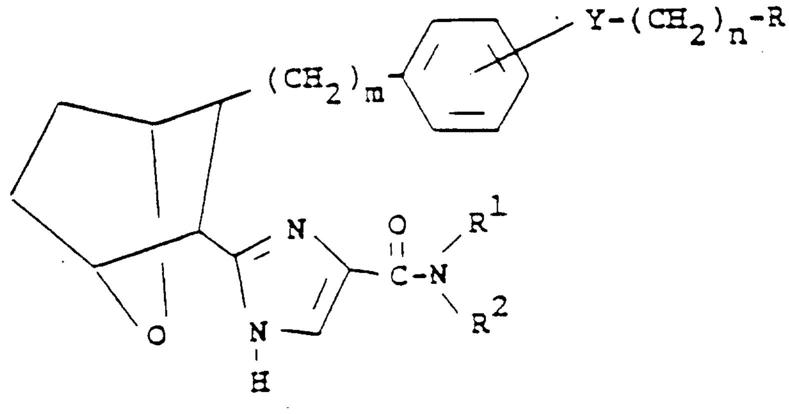

**6.** Verbindung nach einem der vorstehenden Ansprüche, wobei R eine $CO_2H$-Gruppe, ein Rest der Formel $CONHSO_2R^3$ oder eine 5-Tetrazolylgruppe ist.

**7.** Verbindung nach Anspruch 2 der Formel

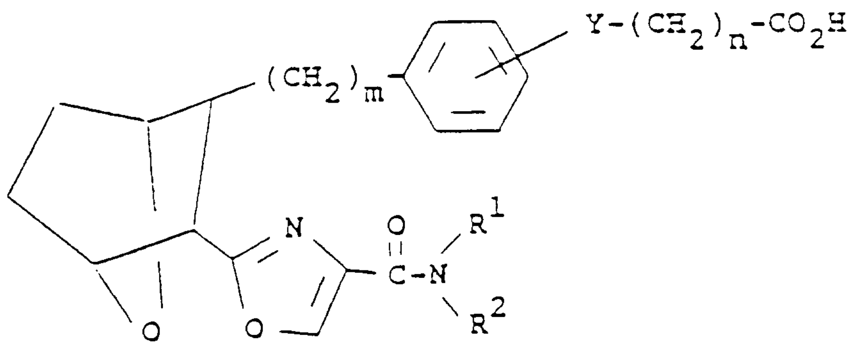

**8.** Verbindung nach Anspruch 1 mit der Bezeichnung [1S-(1α,2α,3α,4α)]-2-[[3-[4-[[(4-Cyclohexylbutyl)-amino]carbonyl]-2-oxazolyl]-7-oxabicyclo[2.2.1]hept-2-yl]methyl]benzolpropionsäure oder ein Ester oder Salz davon.

**9.** Verbindung nach Anspruch 1 mit der Bezeichnung [1S-(1α,2α,3α,4α)]-2-[[3-[4-[[[(4-Chlorphenyl)butyl]-amino]carbonyl]-2-oxazolyl]-7-oxabicyclo[2.2.1]hept-2-yl]methyl]benzolpropionsäure oder ein Ester oder Salz davon.

**10.** Verbindung nach Anspruch 1 mit der Bezeichnung [1S-(1α,2α,3α,4α)]-3-[[3-[4-[[(4-Cyclohexylbutyl)-amino]carbonyl]-2-oxazolyl]-7-oxabicyclo[2.2.1]hept-2-yl]methyl]benzolessigsäure oder ein Ester oder Salz davon.

**11.** Verbindung nach Anspruch 1 mit der Bezeichnung [1S-(1α,2α,3α,4α)]-[2-[[3-[4-[[(4-Cyclohexylbutyl)-amino]carbonyl]-2-oxazolyl]-7-oxabicyclo[2.2.1]hept-2-yl]methyl]phenoxy]essigsäure oder ein Ester oder Salz davon.

**12.** Verbindung nach Anspruch 1 mit der Bezeichnung [1S-(1α,2α,3α,4α)]-2-[[3-[4-[[(7,7-Dimethyloctyl)-amino]carbonyl]-2-oxazolyl]-7-oxabicyclo[2.2.1]hept-2-yl]methyl]benzolpropionsäure oder ein Ester oder Salz davon.

**13.** Verbindung nach Anspruch 1, wobei m = 1, n = 2, X ein Sauerstoffatom ist, Y eine Einfachbindung an der 2-Stellung ist, $R^2$ ein Wasserstoffatom ist, R eine COOH-Gruppe ist, und $R^1$ eine Pentylgruppe ist.

**14.** Verbindung nach Anspruch 1, wobei m = 1, n = 2, X ein Sauerstoffatom ist, Y eine Einfachbindung an der 2-Stellung ist, $R^2$ ein Wasserstoffatom ist, R eine COOH-Gruppe ist, und $R^1$ eine p-Chlorphenethylgruppe ist.

**15.** Verbindung der Formel nach Anspruch 1, in der m = 1, n = 2, X ein Sauerstoffatom ist, Y eine Einfachbindung an der 2-Stellung ist, $R^2$ ein Wasserstoffatom ist, R eine COOH-Gruppe ist, und $R^1$ eine 4-Cyclohexylbuten-2-ylgruppe ist.

**16.** Verbindung der Formel nach Anspruch 1, in der m = 1, n = 2, X ein Sauerstoffatom ist, Y eine Einfachbindung an der 2-Stellung ist, $R^2$ ein Wasserstoffatom ist, R eine COOH-Gruppe ist, und $R^1$ eine 5-Cyclohexylpenten-4-ylgruppe ist.

**17.** Verbindung der Formel nach Anspruch 1, in der m = 1, n = 2, X ein Sauerstoffatom ist, Y eine Einfachbindung an der 2-Stellung ist, $R^2$ ein Wasserstoffatom ist, R eine COOH-Gruppe ist, und $R^1$ eine 4-Benzyloxyphenylgruppe ist.

**18.** Verbindung der Formel nach Anspruch 1, in der m = 1, n = 2, X ein Sauerstoffatom ist, Y eine Einfachbindung an der 2-Stellung ist, $R^2$ ein Wasserstoffatom ist, R eine COOH-Gruppe ist, und $R^1$ eine 3-Iodpropen-2-ylgruppe ist.

**19.** Mittel zur Inhibierung der Plättchenaggregation und Bronchialverengerung, umfassend eine wirksame Menge einer Verbindung nach einem der vorstehenden Ansprüche und einen pharmazeutisch verträglichen Träger dafür.

**20.** Verwendung einer Verbindung nach einem der Ansprüche 1-18 zur Herstellung eines Arzneimittels zur Inhibierung der Plättchenaggregation und Bronchialverengerung durch die Verabreichung an den Blutkreislauf eines Säugerwirtes.

**21.** Verwendung nach Anspruch 20, wobei die Verbindung in einer Form vorliegt, die zur Verabreichung in einer Menge innerhalb des Bereiches von 0,1 bis 100 mg/kg geeignet ist.

**22.** Verwendung einer Verbindung nach einem der Ansprüche 1-18 zur Herstellung eines Arzneimittels zur Inhibierung der Plättchenaggregation.

**23.** Verwendung einer Verbindung nach einem der Ansprüche 1-18 zur Herstellung eines Arzneimittels zur Inhibierung der mit Asthma verbundenen Bronchialverengerung.

**24.** Verwendung einer Verbindung nach einem der Ansprüche 1-18 zur Herstellung eines Arzneimittels zur Verbesserung der postischämischen Myokarddysfunktion.

**25.** Verwendung einer Verbindung nach einem der Ansprüche 1-18 zur Herstellung eines Arzneimittels zur Behandlung der Toxanämie während der Schwangerschaft.

**26.** Verwendung einer Verbindung nach einem der Ansprüche 1-18 zur Herstellung eines Arzneimittels zur Vorbeugung oder Verringerung einer venösen Thrombose.

**27.** Verwendung einer Verbindung nach einem der Ansprüche 1-18 zur Herstellung eines Arzneimittels zur Vorbeugung oder Verringerung des Verlustes von Blutplättchen während der extrakorporalen Zirkulation.

**28.** Verwendung einer Verbindung nach einem der Ansprüche 1-18 zur Herstellung eines Arzneimittels zur Behandlung von Verbrennungen und/oder zur Förderung der Wundheilung in systemischer oder topischer Form.

**29.** Verwendung einer Verbindung nach einem der Ansprüche 1-18 zur Herstellung eines Arzneimittels zur Verringerung der postischämischen Schädigung des Myokards innerhalb von 6 Stunden nach einem Myokardinfarkt, während eine wirksame Menge eines thrombolytischen Mittels ebenfalls verabreicht wird.

**30.** Verwendung nach Anspruch 29, wobei das thrombolytische Mittel t-PA, Streptokinase, Urokinase, Prourokinase oder ein anisoylierter Plasminogen-Streptokinase-Aktivator-Komplex ist.

**31.** Verfahren zur Herstellung einer Verbindung der Formel

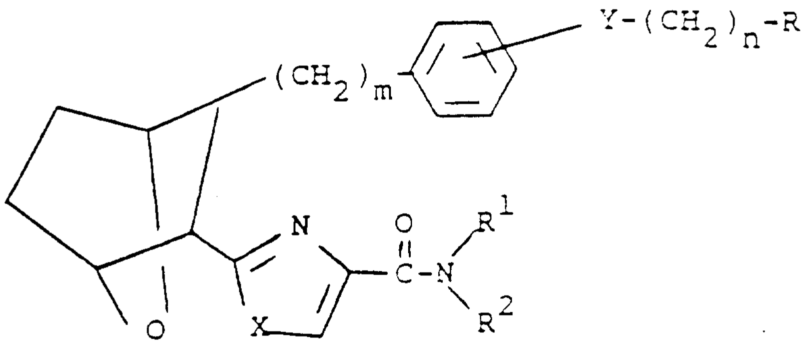

umfassend alle Stereoisomere davon, in der

m 1, 2 oder 3 ist;

n 0, 1, 2, 3 oder 4 ist;

Y ein Sauerstoffatom, eine Vinylgruppe oder eine Einfachbindung ist, mit der Maßgabe, daß Y eine Einfachbindung ist, wenn n 0 ist;

R eine $CO_2H$-Gruppe, eine Gruppe der Formel $CO_2$-Alkalimetall, ein Rest der Formel $CO_2$-Alkyl, eine $CH_2OH$-Gruppe, ein Rest der Formel $CONHSO_2R^3$, ein Rest der Formel $CONHR^{3a}$ oder eine 5-Tetrazolylgruppe ist, mit der Maßgabe, daß n nicht null ist, wenn R eine 5-Tetrazolylgruppe ist;

X ein Sauerstoffatom, Schwefelatom oder eine NH-Gruppe ist;

$R^1$ ein Wasserstoffatom, ein Alkyl-, Alkenyl-, Alkinyl-, Aryl-, Aralkyl-, Cycloalkyl-, Cycloalkylalkylrest, ein gesättigter Heterocyclus, ein gesättigter heterocyclischer Alkylrest, ein aromatischer Heterocyclus, ein aromatischer heterocyclischer Alkylrest oder eine Amidgruppe ist, die gegebenenfalls mit einem Alkyl-, Aryl-, Cycloalkyl- oder Cycloalkylalkylrest substituiert ist; und

$R^2$ ein Wasserstoffatom, ein Alkyl-, Aryl- oder Aralkylrest ist, oder $R^1$ und $R^2$ zusammen mit dem Stickstoffatom, an das sie gebunden sind, einen 5- bis 8-gliedrigen Ring bilden;

$R^3$ ein Alkyl-, Aryl- oder Aralkylrest ist, und $R^{3a}$ ein Wasserstoffatom, ein Alkyl-, Aryl- oder Aralkylrest ist;

und wobei:

"Alkylrest" einen $C_1$- bis $C_{12}$-Alkylrest bedeutet, der gegebenenfalls mit 1, 2 oder 3 Halogensubstituenten, einem Arylsubstituenten, einem Alkylarylsubstituenten, einem Halogenarylsubstituenten, einem Cycloalkylsubstituenten oder einem Alkylcycloalkylsubstituenten substituiert ist;

"Cycloalkylrest" einen $C_3$- bis $C_{12}$-Cycloalkylrest bedeutet, der gegebenenfalls mit (einem) Halogenatom(en), (einem) Alkylrest(en) und/oder (einem) Alkoxyrest(en) substituiert ist;

"Arylrest" eine Phenyl- oder Naphthylgruppe bedeutet, von denen jede gegebenenfalls 1 oder 2 Substituenten aufweist, ausgewählt aus einem Alkylrest, einer Trifluormethylgruppe, einem Halogenatom (Cl, Br oder F), einem Alkoxy-, Alkylthio-, Alkylsulfinyl- und Alkylsulfonylrest;

"Aralkylrest" einen wie vorstehend definierten "Alkylrest" bedeutet, der jedoch einen wie vorstehend definierten "Aryl"substituenten aufweist;

"Alkoxyrest" einen "Alkylrest" bedeutet, der an ein Sauerstoffatom gebunden ist;

"gesättigter Heterocyclus" einen 5-, 6- oder 7-gliedrigen gesättigten Ring bedeutet, der 1 oder 2 Heteroatome umfaßt, ausgewählt aus einem Stickstoff-, Sauerstoff- und Schwefelatom, und der gegebenenfalls mit (einem) "Alkyl-", "Aryl-", "Cycloalkyl-" oder "Cycloalkylalkylrest(en)" substituiert ist;

"aromatischer Heterocyclus" einen 5- oder 6-gliedrigen aromatischen Ring bedeutet, der 1 oder 2 Heteroatome umfaßt, ausgewählt aus einem Stickstoff-, Sauerstoff- und Schwefelatom, und der gegebenenfalls mit (einem) "Alkyl-", "Aryl-", "Cycloalkyl-" oder "Cycloalkylalkylrest(en)" substituiert ist;
gekennzeichnet durch die Umwandlung einer Verbindung der Formel

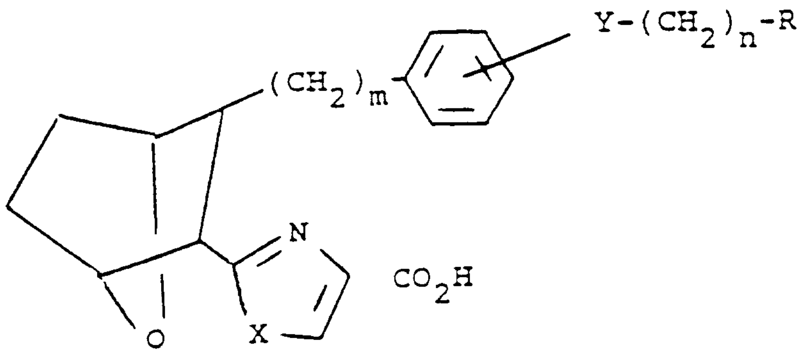

in der R keine $CO_2H$-Gruppe oder ihr Alkalimetallsalz ist, in ihre Säurehalogenidform und anschließende Umsetzung des Säurehalogenids mit einem Amin der Formel $HNR_1R_2$ nach herkömmlichen Verfahren, wobei Produkte gebildet werden, in denen R keine $CO_2H$-Gruppe oder ihr Alkalimetallsalz ist, und das Hydrolysieren eines Produktes, in dem R ein Rest der Formel $CO_2$-Alkyl ist, wobei ein Produkt gebildet wird, in dem R eine $CO_2H$-Gruppe ist, und die Bildung eines Alkalimetallsalzes davon nach herkömmlichen Verfahren.

**Patentansprüche für folgende Vertragsstaaten : ES, GR**

**1.** Verfahren zur Herstellung einer Verbindung der Formel

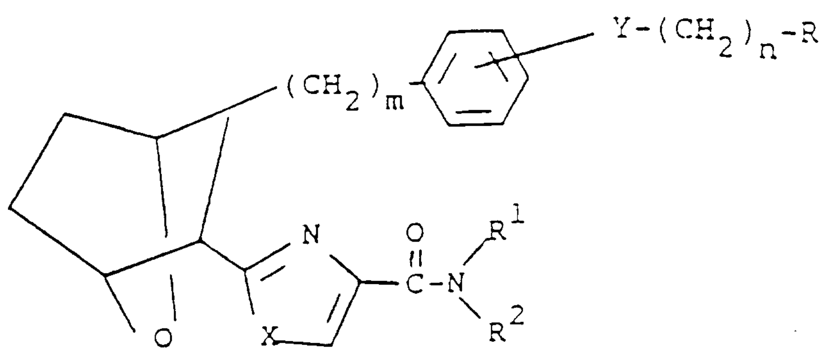

umfassend alle Stereoisomere davon, in der
m 1, 2 oder 3 ist;
n 0, 1, 2, 3 oder 4 ist;
Y ein Sauerstoffatom, eine Vinylgruppe oder eine Einfachbindung ist, mit der Maßgabe, daß Y eine Einfachbindung ist, wenn n 0 ist;
R eine $CO_2H$-Gruppe, eine Gruppe der Formel $CO_2$-Alkalimetall, ein Rest der Formel $CO_2$-Alkyl, eine $CH_2OH$-Gruppe, ein Rest der Formel $CONHSO_2R^3$, ein Rest der Formel $CONHR^{3a}$ oder eine 5-Tetrazolylgruppe ist, mit der Maßgabe, daß n nicht Null ist, wenn R eine 5-Tetrazolylgruppe ist;
X ein Sauerstoffatom, Schwefelatom oder eine NH-Gruppe ist;
$R^1$ ein Wasserstoffatom, ein Alkyl-, Alkenyl-, Alkinyl-, Aryl-, Aralkyl-, Cycloalkyl-, Cycloalkylalkylrest, ein gesättigter Heterocyclus, ein gesättigter heterocyclischer Alkylrest, ein aromatischer Heterocyclus, ein aromatischer heterocyclischer Alkylrest oder eine Amidgruppe ist, die gegebenenfalls mit einem Alkyl-, Aryl-, Cycloalkyl- oder Cycloalkylalkylrest substituiert ist; und
$R^2$ ein Wasserstoffatom, ein Alkyl-, Aryl- oder Aralkylrest ist, oder $R^1$ und $R^2$ zusammen mit dem Stickstoffatom, an das sie gebunden sind, einen 5- bis 8-gliedrigen Ring bilden;
$R^3$ ein Alkyl-, Aryl- oder Aralkylrest ist, und $R^{3a}$ ein Wasserstoffatom, ein Alkyl-, Aryl- oder Aralkylrest ist;
und wobei:

"Alkylrest" einen $C_1$- bis $C_{12}$-Alkylrest bedeutet, der gegebenenfalls mit 1, 2 oder 3 Halogensubstituenten, einem Arylsubstituenten, einem Alkylarylsubstituenten, einem Halogenarylsubstituenten, einem Cycloalkylsubstituenten oder einem Alkylcycloalkylsubstituenten substituiert ist;

"Cycloalkylrest" einen $C_3$- bis $C_{12}$-Cycloalkylrest bedeutet, der gegebenenfalls mit (einem) Halogenatom(en), (einem) Alkylrest(en) und/oder (einem) Alkoxyrest(en) substituiert ist;

"Arylrest" eine Phenyl- oder Naphthylgruppe bedeutet, von denen jede gegebenenfalls 1 oder 2 Substituenten aufweist, ausgewählt aus einem Alkylrest, einer Trifluormethylgruppe, einem Halogenatom (Cl, Br oder F), einem Alkoxy-, Alkylthio-, Alkylsulfinyl- und Alkylsulfonylrest;

"Aralkylrest" einen wie vorstehend definierten "Alkylrest" bedeutet, der jedoch einen wie vorstehend definierten "Aryl"substituenten aufweist;

"Alkoxyrest" einen "Alkylrest" bedeutet, der an ein Sauerstoffatom gebunden ist;

"gesättigter Heterocyclus" einen 5-, 6- oder 7-gliedrigen gesättigten Ring bedeutet, der 1 oder 2 Heteroatome umfaßt, ausgewählt aus einem Stickstoff-, Sauerstoff- und Schwefelatom, und der gegebenenfalls mit (einem) "Alkyl-", "Aryl-", "Cycloalkyl-" oder "Cycloalkylalkylrest(en)" substituiert ist;

"aromatischer Heterocyclus" einen 5- oder 6-gliedrigen aromatischen Ring bedeutet, der 1 oder 2 Heteroatome umfaßt, ausgewählt aus einem Stickstoff-, Sauerstoff- und Schwefelatom, und der gegebenenfalls mit (einem) "Alkyl-", "Aryl-", "Cycloalkyl-" oder "Cycloalkylalkylrest(en)" substituiert ist;

gekennzeichnet durch die Umwandlung einer Verbindung der Formel

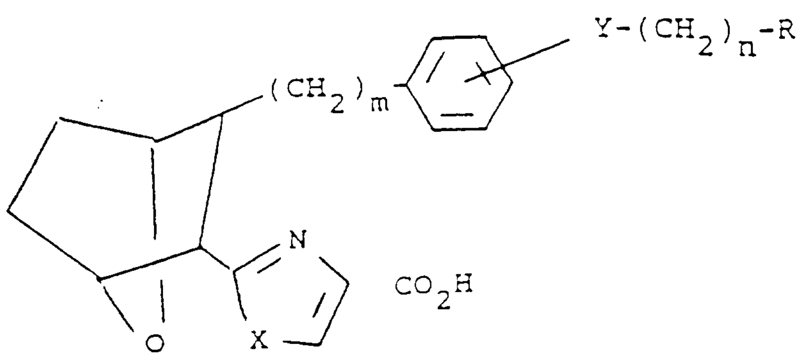

in der R keine $CO_2H$-Gruppe oder ihr Alkalimetallsalz ist, in ihre Säurehalogenidform und anschließende Umsetzung des Säurehalogenids mit einem Amin der Formel $HNR_1R_2$ nach herkömmlichen Verfahren, wobei Produkte gebildet werden, in denen R keine $CO_2H$-Gruppe oder ihr Alkalimetallsalz ist, und das Hydrolysieren eines Produktes, in dem R ein Rest der Formel $CO_2$-Alkyl ist, wobei ein Produkt gebildet wird, in dem R eine $CO_2H$-Gruppe ist, und die Bildung eines Alkalimetallsalzes davon nach herkömmlichen Verfahren.

**2.** Verfahren nach Anspruch 1, wobei das Produkt die Formel

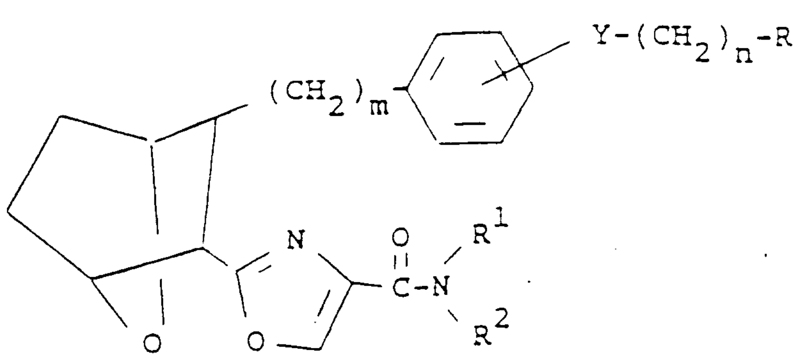

aufweist.

3. Verfahren nach Anspruch 1, wobei m = 1 und n = 2 ist.

4. Verfahren nach Anspruch 1, wobei das Produkt die Formel

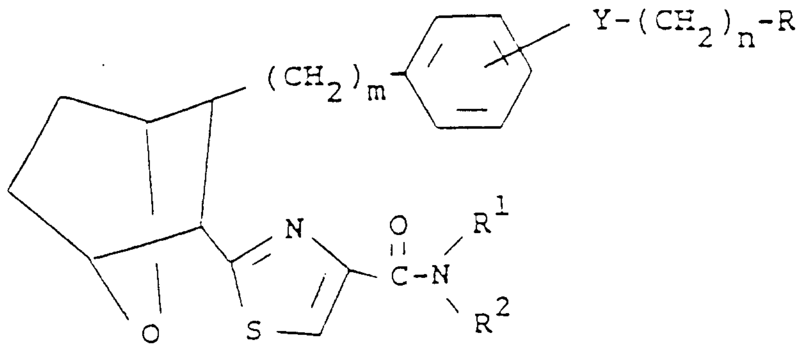

aufweist.

5. Verfahren nach Anspruch 1, wobei das Produkt die Formel

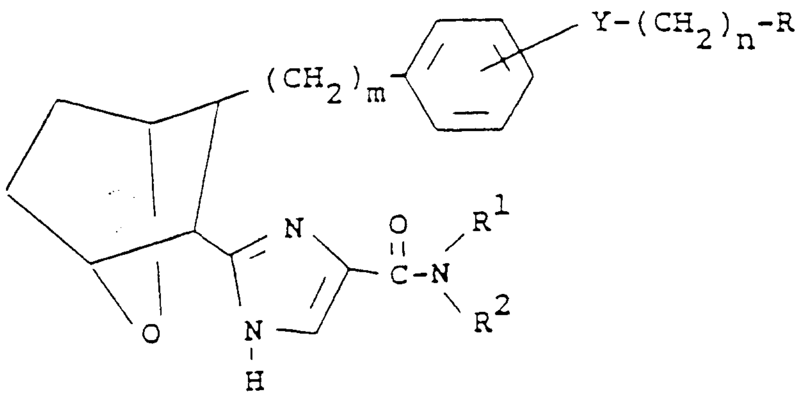

aufweist.

6. Verfahren nach Anspruch 1, wobei R eine $CO_2H$-Gruppe, ein Rest der Formel $CONHSO_2R^3$ oder eine 5-Tetrazolylgruppe ist.

7. Verfahren nach Anspruch 1, wobei das Produkt die Formel

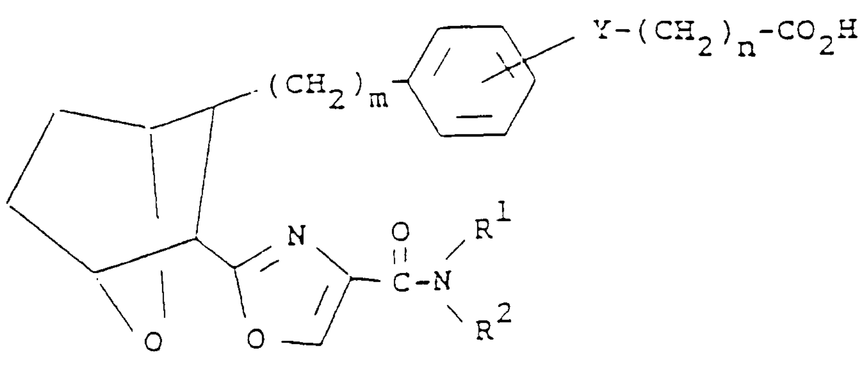

aufweist.

**8.** Verfahren nach Anspruch 1, wobei das Produkt [1S-(1α,2α,3α,4α)]-2-[[3-[4-[[(4-Cyclohexylbutyl)amino]-carbonyl]-2-oxazolyl]-7-oxabicyclo[2.2.1]hept-2-yl]methyl]benzolpropionsäure oder ein Ester oder Salz davon ist.

**9.** Verfahren nach Anspruch 1, wobei das Produkt [1S-(1α,2α,3α,4α)]-2-[[3-[4-[[[(4-Chlorphenyl)butyl]-amino]carbonyl]-2-oxazolyl]-7-oxabicyclo[2.2.1]hept-2-yl]methyl]benzolpropionsäure oder ein Ester oder Salz davon ist.

**10.** Verfahren nach Anspruch 1, wobei das Produkt [1S-(1α,2α,3α,4α)]-3-[[3-[4-[[(4-Cyclohexylbutyl)amino]-carbonyl]-2-oxazolyl]-7-oxabicyclo[2.2.1]hept-2-yl]methyl]benzolessigsäure oder ein Ester oder Salz davon ist.

**11.** Verfahren nach Anspruch 1, wobei das Produkt [1S-(1α,2α,3α,4α)]-[2-[[3-[4-[[(4-Cyclohexylbutyl)amino]-carbonyl]-2-oxazolyl]-7-oxabicyclo[2.2.1]hept-2-yl]methyl]phenoxy]essigsäure oder ein Ester oder Salz davon ist.

**12.** Verfahren nach Anspruch 1, wobei das Produkt [1S-(1α,2α,3α,4α)]-2-[[3-[4-[[(7,7-Dimethyloctyl)amino]-carbonyl]-2-oxazolyl]-7-oxabicyclo[2.2.1]hept-2-yl]methyl]benzolpropionsäure oder ein Ester oder Salz davon ist.

**13.** Verfahren nach Anspruch 1, wobei in dem Produkt $m = 1$, $n = 2$, X ein Sauerstoffatom ist, Y eine Einfachbindung an der 2-Stellung ist, $R^2$ ein Wasserstoffatom ist, R eine COOH-Gruppe ist, und $R^1$ eine Pentylgruppe ist.

**14.** Verfahren nach Anspruch 1, wobei in dem Produkt $m = 1$, $n = 2$, X ein Sauerstoffatom ist, Y eine Einfachbindung an der 2-Stellung ist, $R^2$ ein Wasserstoffatom ist, R eine COOH-Gruppe ist, und $R^1$ eine p-Chlorphenethylgruppe ist.

**15.** Verfahren zur Herstellung einer Verbindung der ersten Formel in Anspruch 1, in der $m = 1$, $n = 2$, X ein Sauerstoffatom ist, Y eine Einfachbindung an der 2-Stellung ist, $R^2$ ein Wasserstoffatom ist, R eine COOH-Gruppe ist, und $R^1$ eine 4-Cyclohexylbuten-2-ylgruppe ist, wobei die Schritte des Verfahrens wie in Anspruch 1 definiert sind.

**16.** Verfahren zur Herstellung einer Verbindung der ersten Formel in Anspruch 1, in der $m = 1$, $n = 2$, X ein Sauerstoffatom ist, Y eine Einfachbindung an der 2-Stellung ist, $R^2$ ein Wasserstoffatom ist, R eine COOH-Gruppe ist, und $R^1$ eine 5-Cyclohexylpenten-4-ylgruppe ist, wobei die Schritte des Verfahrens wie in Anspruch 1 definiert sind.

**17.** Verfahren zur Herstellung einer Verbindung der ersten Formel in Anspruch 1, in der $m = 1$, $n = 2$, X ein Sauerstoffatom ist, Y eine Einfachbindung an der 2-Stellung ist, $R^2$ ein Wasserstoffatom ist, R eine COOH-Gruppe ist, und $R^1$ eine 4-Benzyloxyphenylgruppe ist, wobei die Schritte des Verfahrens wie in Anspruch 1 definiert sind.

**18.** Verfahren zur Herstellung einer Verbindung der ersten Formel in Anspruch 1, in der $m = 1$, $n = 2$, X ein Sauerstoffatom ist, Y eine Einfachbindung an der 2-Stellung ist, $R^2$ ein Wasserstoffatom ist, R eine COOH-Gruppe ist, und $R^1$ eine 3-Iodpropen-2-ylgruppe ist, wobei die Schritte des Verfahrens wie in Anspruch 1 definiert sind.

**Revendications**

**Revendications pour les Etats contractants suivants : AT, BE, DK, FR, DE, GB, NL, IT, LU, SE, CH, LI**

1. Composé ayant pour formule

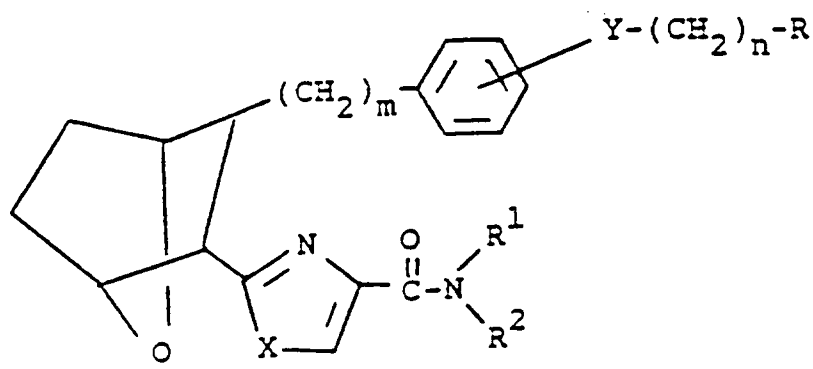

y compris tous ses stéréo-isomères, formule dans laquelle

m est égal à 1, 2 ou 3;

n est égal à 0, 1, 2, 3 ou 4;

Y est O, le groupe vinyle ou une liaison simple, à condition que, quand n est égal à 0, Y soit une liaison simple;

R est $CO_2H$, $CO_2$-métal alcalin, $CO_2$-alkyle, $CH_2OH$, $CONHSO_2R^3$, $CONHR^{3a}$ ou 5-tétrazolyle, à condition que, quand R est le groupe 5-tétrazolyle, n soit autre que 0;

X est O, S ou NH;

$R^1$ est l'hydrogène ou un groupe alkyle, alcényle, alcynyle, aryle, aralkyle, cycloalkyle, cycloalkylalkyle, un hétérocycle saturé, un groupe hétérocycle saturé-alkyle, un hétérocycle aromatique, un groupe hétérocycle aromatique-alkyle, ou un groupe amide qui est facultativement substitué par un groupe alkyle, aryle, cycloalkyle ou cycloalkylalkyle; et

$R^2$ est l'hydrogène ou un groupe alkyle, aryle ou aralkyle, ou bien $R^1$ et $R^2$ forment, avec l'atome d'azote auquel ils sont liés, un cycle de 5 à 8 chaînons;

$R^3$ est un groupe alkyle, aryle ou aralkyle et $R^{3a}$ est l'hydrogène ou un groupe alkyle, aryle ou aralkyle;

et dans laquelle:

"alkyle" désigne un groupe alkyle en $C_1$ à $C_{12}$ qui est facultativement substitué par 1, 2 ou 3 substituants halo, un substituant aryle, un substituant alkyl-aryle, un substituant haloaryle, un substituant cycloalkyle ou un substituant alkylcycloalkyle;

"cycloalkyle" désigne un groupe cycloalkyle en $C_3$ à $C_{12}$ qui est facultativement substitué par halogène, alkyle et/ou alcoxy;

"aryle" désigne un groupe phényle ou naphtyle, dont chacun porte facultativement 1 ou 2 substituants choisis entre les groupes alkyle et trifluorométhyle, les atomes d'halogène (Cl, Br ou F) et les groupes alcoxy, alkylthio, alkylsulfinyle et alkylsulfonyle;

"aralkyle" désigne un groupe "alkyle" tel que défini ci-dessus mais portant un substituant "aryle" tel qu'il est défini ci-dessus;

"alcoxy" désigne un groupe "alkyle" lié à un atome d'oxygène;

"hétérocycle saturé" désigne un cycle saturé à 5, 6 ou 7 chaînons qui comporte 1 ou 2 hétéro-atomes choisis entre l'azote, l'oxygène et le soufre, et qui est facultativement substitué par "alkyle", "aryle", "cycloalkyle" ou "cycloalkylalkyle";

"hétérocycle aromatique" désigne un cycle aromatique à 5 ou 6 chaînons qui comporte 1 ou 2 hétéro-atomes choisis entre l'azote, l'oxygène et le soufre et qui est facultativement substitué par "alkyle", "aryle", "cycloalkyle" ou "cycloalkylalkyle".

2. Composé selon la revendication 1, ayant pour formule

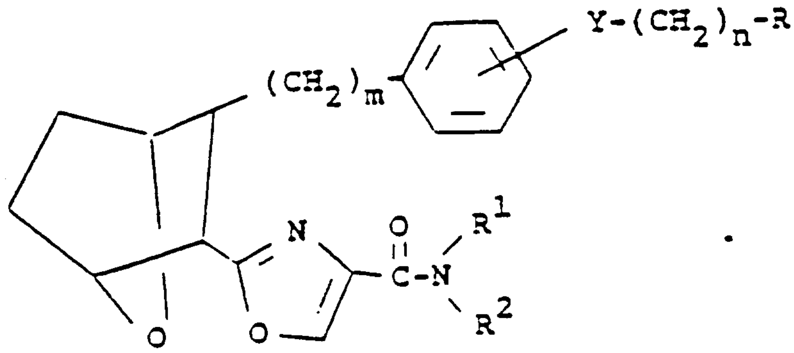

.

3. Composé selon la revendication 1, dans lequel m = 1 et n = 2.

4. Composé selon la revendication 1, ayant pour formule

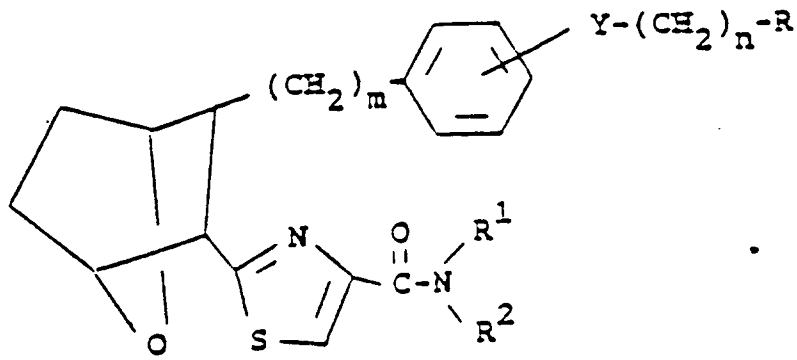

.

5. Composé selon la revendication 1, ayant pour formule

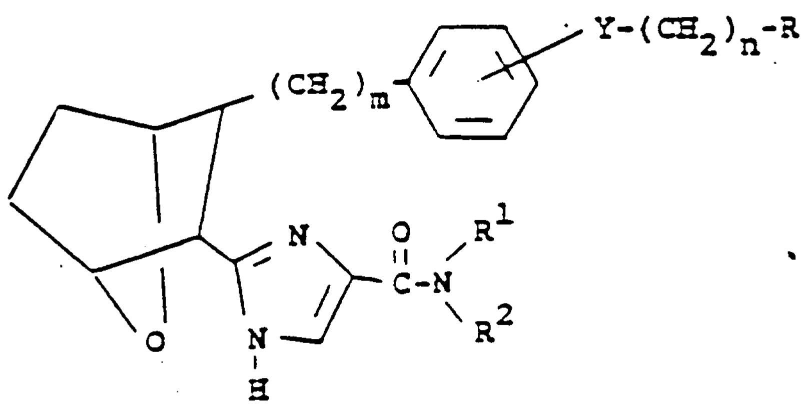

.

6. Composé selon l'une quelconque des revendications précédentes, dans lequel R est $CO_2H$, $CONHSO_2R^3$ ou 5-tétrazolyle.

**7.** Composé selon la revendication 2, ayant pour formule

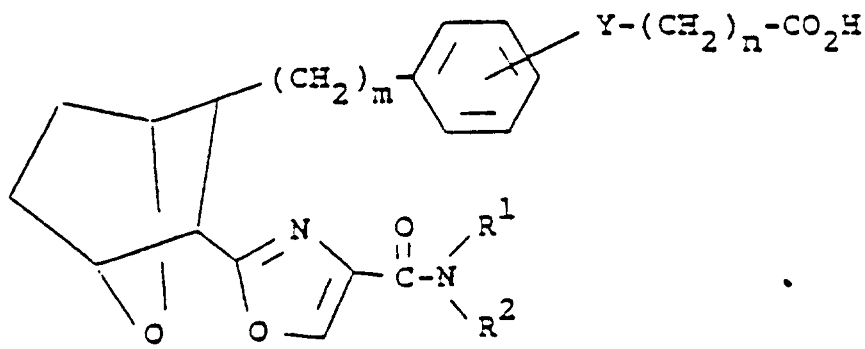

**8.** Composé selon la revendication 1, qui est l'acide [1S-(1α,2α,3α,4α)]-2-[[3-[4-[[(4-cyclohexylbutyl)-amino]carbonyl]-2-oxazolyl]-7-oxabicyclo[2.2.1]-hept-2-yl]méthyl]benzènepropanoïque, ou un ester ou un sel de celui-ci.

**9.** Composé selon la revendication 1, qui est l'acide [1S-(1α,2α,3α,4α)]-2-[[3-[4-[[[4-chlorophényl)butyl]-amino]carbonyl]-2-oxazolyl]-7-oxabicyclo[2.2.1]-hept-2-yl]méthyl]benzènepropanoïque, ou un ester ou un sel de celui-ci.

**10.** Composé selon la revendication 1, qui est l'acide [1S-(1α,2α,3α,4α)]-3-[[3-[4-[[(4-cyclohexylbutyl)-amino]carbonyl]-2-oxazolyl]-7-oxabicyclo[2.2.1]-hept-2-yl]méthyl]benzèneacétique, ou un ester ou un sel de celui-ci.

**11.** Composé selon la revendication 1, qui est l'acide [1S-(1α,2α,3α,4α)]-[2-[[3-[4-[[(4-cyclohexylbutyl)-amino]carbonyl]-2-oxazolyl]-7-oxabicyclo[2.2.1]-hept-2-yl]méthyl]phénoxy]acétique, ou un ester ou un sel de celui-ci.

**12.** Composé selon la revendication 1, qui est l'acide [1S-(1α,2α,3α,4α)]-2-[[3-[4-[[(7,7-diméthyloctyl)amino]-carbonyl]-2-oxazolyl]-7-oxabicyclo[2.2.1]-hept-2-yl]méthyl]benzènepropanoïque, ou un ester ou un sel de celui-ci.

**13.** Composé selon la revendication 1, dans lequel m = 1, n = 2, X est O, Y est une liaison simple en position 2, $R^2$ est H, R est COOH et $R^1$ est le groupe pentyle.

**14.** Composé selon la revendication 1, dans lequel m = 1, n = 2, X est O, Y est une liaison simple en position 2, $R^2$ est H, R est COOH et $R^1$ est le groupe p-chlorophénéthyle.

**15.** Composé de la formule donnée dans la revendication 1, dans lequel m = 1, n = 2, X est O, Y est une liaison simple en position 2, $R^2$ est H, R est COOH et $R^1$ est le groupe 4-cyclohexylbutén-2-yle.

**16.** Composé de la formule donnée dans la revendication 1, dans lequel m = 1, n = 2, X est O, Y est une liaison simple en position 2, $R^2$ est H, R est COOH et $R^1$ est le groupe 5-cyclohexylpentén-4-yle.

**17.** Composé de la formule donnée dans la revendication 1, dans lequel m = 1, n = 2, X est O, Y est une liaison simple en position 2, $R^2$ est H, R est COOH et $R^1$ est le groupe 4-benzyloxyphényle.

**18.** Composé de la formule donnée dans la revendication 1, dans lequel m = 1, n = 2, X est O, Y est une liaison simple en position 2, $R^2$ est H, R est COOH et $R^1$ est le groupe 3-iodo-propén-2-yle.

**19.** Composition permettant d'inhiber l'agrégation plaquettaire et la bronchoconstriction, comprenant une quantité efficace d'un composé tel qu'il est défini dans l'une quelconque des revendications précédentes, et un porteur pharmaceutiquement acceptable pour celui-ci.

**20.** Utilisation d'un composé tel qu'il est défini dans l'une quelconque des revendications 1 à 18 pour la fabrication d'un médicament permettant d'inhiber l'agrégation plaquettaire et la bronchoconstriction, par administration au système circulatoire d'un mammifère hôte.

**21.** Utilisation selon la revendication 20, dans laquelle ledit composé est administré sous une forme convenant à son administration dans une quantité comprise dans l'intervalle de 0,1 à 100 mg/kg.

**22.** Utilisation d'un composé selon l'une quelconque des revendications 1 à 18 pour la fabrication d'un médicament permettant d'inhiber l'agrégation plaquettaire.

**23.** Utilisation d'un composé selon l'une quelconque des revendications 1 à 18 pour la fabrication d'un médicament permettant d'inhiber la bronchoconstriction associée à l'asthme.

**24.** Utilisation d'un composé selon l'une quelconque des revendications 1 à 18 pour la fabrication d'un médicament permettant d'améliorer un dysfonctionnement myocardique post-ischémique.

**25.** Utilisation d'un composé selon l'une quelconque des revendications 1 à 18 pour la fabrication d'un médicament permettant de traiter la néphropathie gravidique.

**26.** Utilisation d'un composé selon l'une quelconque des revendications 1 à 18 pour la fabrication d'un médicament permettant de prévenir ou de réduire la thrombose veineuse.

**27.** Utilisation d'un composé selon l'une quelconque des revendications 1 à 18 pour la fabrication d'un médicament permettant de prévenir ou de réduire la perte de plaquettes pendant la circulation extracorporelle.

**28.** Utilisation d'un composé selon l'une quelconque des revendications 1 à 18 pour la fabrication d'un médicament permettant de traiter les blessures par brûlure et/ou de favoriser la cicatrisation des plaies, sous une forme systémique ou topique.

**29.** Utilisation d'un composé selon l'une quelconque des revendications 1 à 18 pour la fabrication d'un médicament permettant de réduire une atteinte myocardique post-ischémique dans les 6 heures d'un infarctus du myocarde, en même temps qu'une quantité efficace d'un agent fibrinolytique est également administrée.

**30.** Utilisation d'un composé selon la revendication 29, dans laquelle ledit agent fibrinolytique est un t-PA, la streptokinase, l'urokinase, la pro-urokinase ou un complexe activateur de plasminogène anisoylé et de streptokinase.

**31.** Procédé de préparation d'un composé ayant pour formule

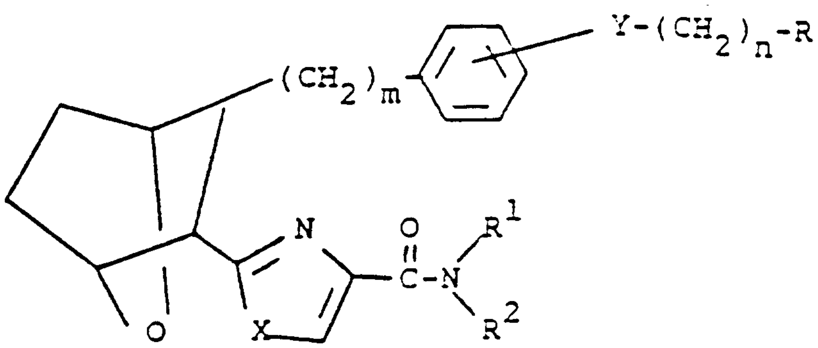

y compris tous ses stéréo-isomères, formule dans laquelle
m est égal à 1, 2 ou 3;
n est égal à 0, 1, 2, 3 ou 4;

Y est O, un groupe vinyle ou une liaison simple, à cette condition que, quand n est égal à 0, Y soit une liaison simple;

R est $CO_2H$, $CO_2$-métal alcalin, $CO_2$-alkyle, $CH_2OH$, $CONHSO_2R^3$, $CONHR^{3a}$ ou 5-tétrazolyle, à cette condition que, quand R est le groupe 5-tétrazolyle, n soit autre que 0;

X est O, S ou NH;

$R^1$ est l'hydrogène ou un groupe alkyle, alcényle, alcynyle, aryle, aralkyle, cycloalkyle, cycloalkylalkyle, un hétérocycle saturé, un groupe hétérocycle saturé-alkyle, un hétérocycle aromatique, un groupe hétérocycle aromatique-alkyle, ou un groupe amide qui est facultativement substitué par un groupe alkyle, aryle, cycloalkyle ou cycloalkylalkyle, et

$R^2$ est l'hydrogène ou un groupe alkyle, aryle ou aralkyle, ou bien $R^1$ et $R^2$ forment, avec l'atome d'azote auquel ils sont liés, un cycle de 5 à 8 chaînons;

$R^3$ est un groupe alkyle, aryle ou aralkyle et $R^{3a}$ est l'hydrogène ou un groupe alkyle, aryle ou aralkyle,

et dans laquelle:

"alkyle" désigne un groupe alkyle en $C_1$ à $C_{12}$ qui est facultativement substitué par 1, 2 ou 3 substituants halo, un substituant aryle, un substituant alkyl-aryle, un substituant haloaryle, un substituant cycloalkyle ou un substituant alkylcycloalkyle;

"cycloalkyle" désigne un groupe cycloalkyle en $C_3$ à $C_{12}$ qui est facultativement substitué par halogène, alkyle et/ou alcoxy;

"aryle" désigne un groupe phényle ou naphtyle, dont chacun porte facultativement 1 ou 2 substituants choisis entre les groupes alkyle et trifluorométhyle, les atomes d'halogène (Cl, Br ou F), et les groupes alcoxy, alkylthio, alkylsulfinyle et alkylsulfonyle;

"aralkyle" désigne un groupe "alkyle" tel que défini ci-dessus mais portant un substituant "aryle" tel que défini ci-dessus;

"alcoxy" désigne un groupe "alkyle" lié à un atome d'oxygène;

"hétérocycle saturé" désigne un cycle saturé de 5, 6 ou 7 chaînons qui comporte 1 ou 2 hétéro-atomes choisis entre l'azote, l'oxygène et le soufre et qui est facultativement substitué par "alkyle", "aryle", "cycloalkyle" ou "cycloalkylalkyle";

"hétérocyclique aromatique" désigne un cycle aromatique de 5 ou 6 chaînons qui comporte 1 ou 2 hétéro-atomes choisis entre l'azote, l'oxygène et le soufre et qui est facultativement substitué par "alkyle", "aryle" "cycloalkyle" ou "cycloalkylalkyle", caractérisé en ce qu'on transforme un composé de formule

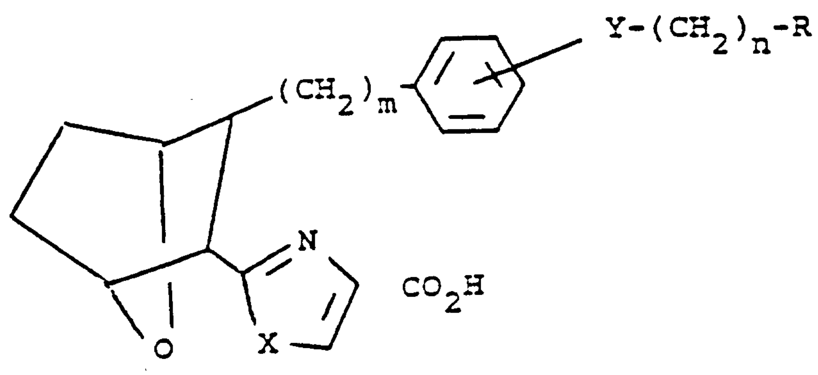

dans laquelle R est autre que $CO_2H$ ou son sel de métal alcalin, en sa forme halogénure d'acide, puis on fait réagir ledit halogénure d'acide avec une amine de formule $HNR_1R_2$, selon des modes opératoires classiques, pour former des produits dans lesquels R est autre que $CO_2H$ ou son sel de métal alcalin, et on hydrolyse un produit dans lequel R est $CO_2$-alkyle pour former un produit dans lequel R est $CO_2H$, et on forme un sel de métal alcalin de celui-ci, selon des modes opératoires classiques.

**Revendications pour les Etats contractants suivants : ES, GR**

**1.** Procédé de préparation d'un composé ayant pour formule

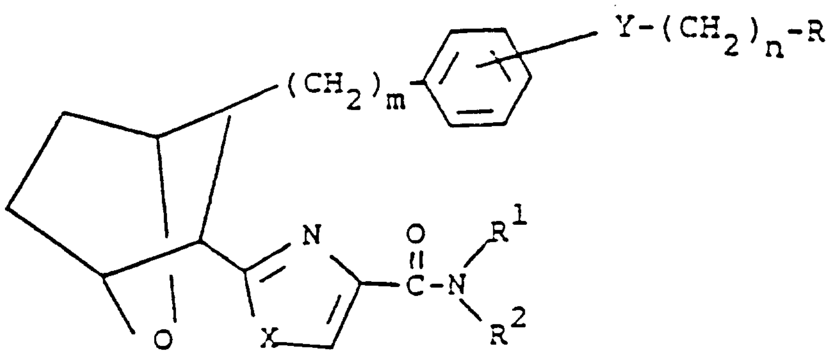

y compris tous ses stéréo-isomères, formule dans laquelle

m est égal à 1, 2 ou 3;

n est égal à 0, 1, 2, 3 ou 4;

Y est O, le groupe vinyle ou une liaison simple, à condition que, quand n est égal à 0, Y soit une liaison simple;

R est $CO_2H$, $CO_2$-métal alcalin, $CO_2$-alkyle, $CH_2OH$, $CONHSO_2R^3$, $CONHR^{3a}$ ou 5-tétrazolyle, à condition que, quand R est le groupe 5-tétrazolyle, n soit autre que 0;

X est O, S ou NH;

$R^1$ est l'hydrogène ou un groupe alkyle, alcényle, alcynyle, aryle, aralkyle, cycloalkyle, cycloalkylalkyle, un hétérocycle saturé, un groupe hétérocycle saturé-alkyle, un hétérocycle aromatique, un groupe hétérocycle aromatique-alkyle, ou un groupe amide qui est facultativement substitué par un groupe alkyle, aryle, cycloalkyle ou cycloalkylalkyle; et

$R^2$ est l'hydrogène ou un groupe alkyle, aryle ou aralkyle, ou bien $R^1$ et $R^2$ forment, avec l'atome d'azote auquel ils sont liés, un cycle de 5 à 8 chaînons;

$R^3$ est un groupe alkyle, aryle ou aralkyle et $R^{3a}$ est l'hydrogène ou un groupe alkyle, aryle ou aralkyle,

et dans laquelle:

"alkyle" désigne un groupe alkyle en $C_1$ à $C_{12}$ qui est facultativement substitué par 1, 2 ou 3 substituants halo, un substituant aryle, un substituant alkyl-aryle, un substituant haloaryle, un substituant cycloalkyle ou un substituant alkylcycloalkyle;

"cycloalkyle" désigne un groupe cycloalkyle en $C_3$ à $C_{12}$ qui est facultativement substitué par halogène, alkyle et/ou alcoxy;

"aryle" désigne un groupe phényle ou naphtyle, dont chacun porte facultativement 1 ou 2 substituants choisis entre les groupes alkyle et trifluorométhyle, les atomes d'halogène (Cl, Br ou F), et les groupes alcoxy, alkylthio, alkylsulfinyle et alkylsulfonyle;

"aralkyle" désigne un groupe "alkyle" tel que défini ci-dessus mais portant un substituant "aryle" tel que défini ci-dessus;

"alcoxy" désigne un groupe "alkyle" lié à un atome d'oxygène;

"hétérocycle saturé" désigne un cycle saturé de 5, 6 ou 7 chaînons qui comporte 1 ou 2 hétéro-atomes choisis entre l'azote, l'oxygène et le soufre et qui est facultativement substitué par "alkyle", "aryle", "cycloalkyle" ou "cycloalkylalkyle";

"hétérocyclique aromatique" désigne un cycle aromatique de 5 ou 6 chaînons qui comporte 1 ou 2 hétéro-atomes choisis entre l'azote, l'oxygène et le soufre et qui est facultativement substitué par "alkyle", "aryle", "cycloalkyle" ou "cycloalkylalkyle", caractérisé en ce qu'on transforme un composé de formule

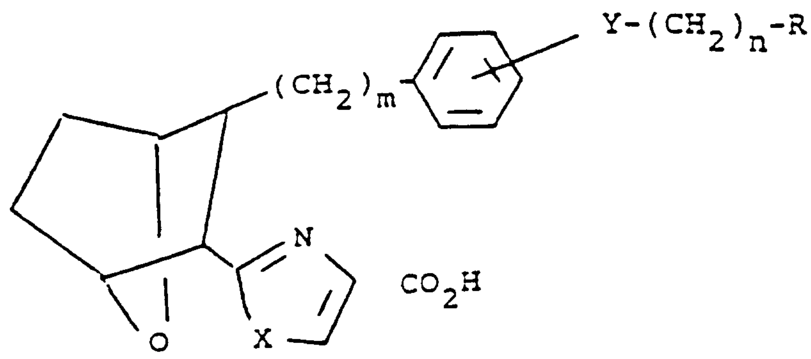

dans laquelle R est autre que $CO_2H$ ou son sel de métal alcalin, en sa forme halogénure d'acide, puis on fait réagir ledit halogénure d'acide avec une amine de formule $HNR_1R_2$, selon des modes opératoires classiques, pour former des produits dans lesquels R est autre que $CO_2H$ ou son sel de métal alcalin, et on hydrolyse un produit dans lequel R est $CO_2$-alkyle pour former un produit dans lequel R est $CO_2H$, et on forme un sel de métal alcalin de celui-ci, selon des modes opératoires classiques.

**2.** Procédé selon la revendication 1, dans lequel le produit a pour formule

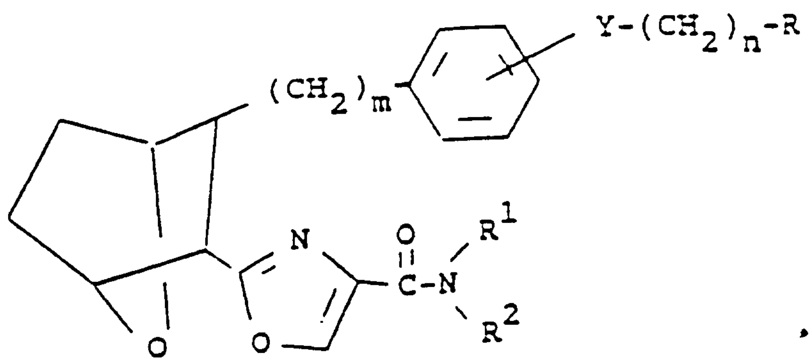

.

**3.** Procédé selon la revendication 1, dans lequel m = 1 et n = 2.

**4.** Procédé selon la revendication 1, dans lequel le produit a pour formule

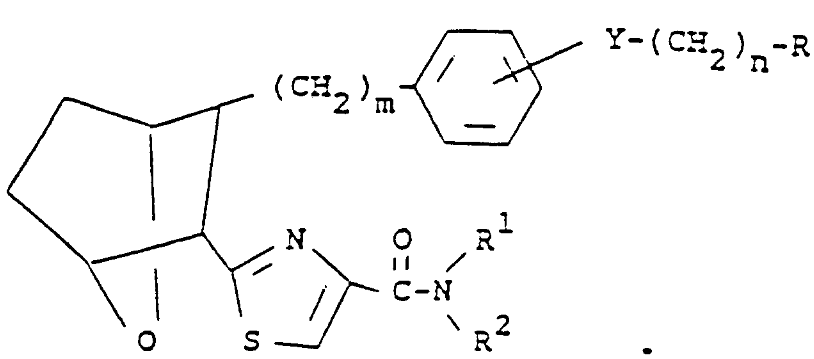

.

**5.** Procédé selon la revendication 1, dans lequel le produit a pour formule

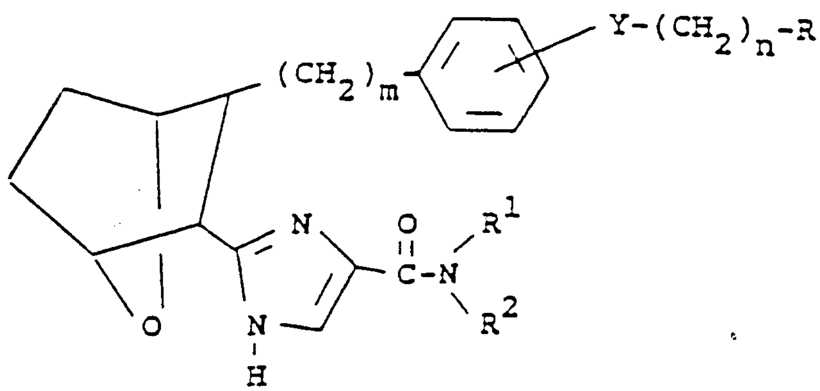

**6.** Procédé selon la revendication 1, dans lequel R est $CO_2H$, $CONHSO_2R^3$ ou 5-tétrazolyle.

**7.** Procédé selon la revendication 1, dans lequel le produit a pour formule

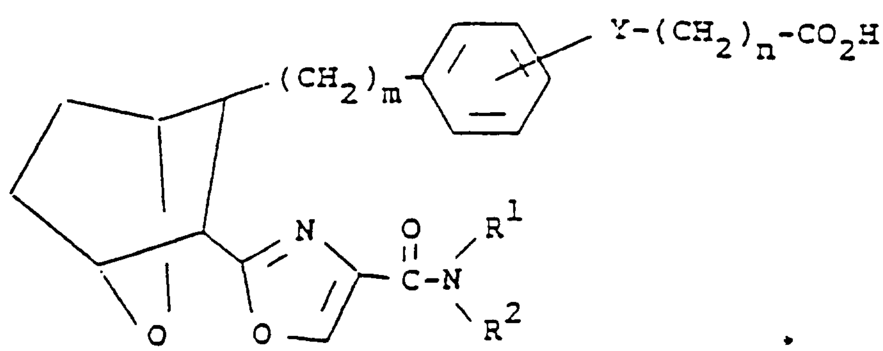

**8.** Procédé selon la revendication 1, dans lequel le produit est l'acide [1S-(1α,2α,3α,4α)]-2-[[3-[4-[[(4-cyclohexylbutyl)amino]carbonyl]-2-oxazolyl]-7-oxabicyclo[2.2.1]-hept-2-yl]méthyl]benzènepropanoïque, ou un ester ou un sel de celui-ci.

**9.** Procédé selon la revendication 1, dans lequel le produit est l'acide [1S-(1α,2α,3α,4α)]-2-[[3-[4-[[[4-chlorophényl)butyl]amino]carbonyl]-2-oxazolyl]-7-oxabicyclo[2.2.1]-hept-2-yl]méthyl]-benzènepropanoïque, ou un ester ou un sel de celui-ci.

**10.** Procédé selon la revendication 1, dans lequel le produit est l'acide [1S-(1α,2α,3α,4α)]-3-[[3-[4-[[(4-cyclohexylbutyl)amino]carbonyl]-2-oxazolyl]-7-oxabicyclo[2.2.1]-hept-2-yl]méthyl]benzèneacétique, ou un ester ou un sel de celui-ci.

**11.** Procédé selon la revendication 1, dans lequel le produit est l'acide [1S-(1α,2α,3α,4α)]-[2-[[3-[4-[[(4-cyclohexylbutyl)amino]carbonyl]-2-oxazolyl]-7-oxabicyclo[2.2.1]-hept-2-yl]méthyl]phénoxy]acétique, ou un ester ou un sel de celui-ci

**12.** Procédé selon la revendication 1, dans lequel le produit est l'acide [1S-(1α,2α,3α,4α)]-2-[[3-[4-[[(7,7-diméthyloctyl)amino]carbonyl]-2-oxazolyl]-7-oxabicyclo[2.2.1]-hept-2-yl]méthyl]benzènepropanoïque, ou un ester ou un sel de celui-ci.

**13.** Procédé selon la revendication 1, dans lequel, dans le produit, m = 1, n = 2, X est O, Y est une liaison simple en position 2, $R^2$ est H, R est COOH et $R^1$ est le groupe pentyle.

**14.** Procédé selon la revendication 1, dans lequel, dans le produit, m = 1, n = 2, X est O, Y est une liaison simple en position 2, $R^2$ est H, R est COOH et $R^1$ est le groupe p-chlorophénéthyle.

**15.** Procédé de préparation d'un composé de la première formule de la revendication 1, dans lequel m = 1, n = 2, X est O, Y est une liaison simple en position 2, $R^2$ est H, R est COOH et $R^1$ est le groupe 4-cyclohexylbutén-2-yle, les étapes du procédé étant telles que définies dans la revendication 1.

**16.** Procédé de préparation d'un composé de la première formule de la revendication 1, dans lequel m = 1, n = 2, X est O, Y est une liaison simple en position 2, $R^2$ est H, R est COOH et $R^1$ est le groupe 5-cyclohexylpentén-4-yle, les étapes du procédé étant telles que définies dans la revendication 1.

**17.** Procédé de préparation d'un composé de la première formule de la revendication 1, dans lequel m = 1, n = 2, X est O, Y est une liaison simple en position 2, $R^2$ est H, R est COOH et $R^1$ est le groupe 4-benzyloxyphényle, les étapes du procédé étant telles que définies dans la revendication 1.

**18.** Procédé de préparation d'un composé de la première formule de la revendication 1, dans lequel m = 1, n = 2, X est O, Y est une liaison simple en position 2, $R^2$ est H, R est COOH et $R^1$ est le groupe 3-iodo-propén-2-yle, les étapes du procédé étant telles que définies dans la revendication 1.